(19) **Europäisches Patentamt**
**European Patent Office**
**Office européen des brevets**

(11) **EP 3 303 563 B1**

(12) **EUROPEAN PATENT SPECIFICATION**

(45) Date of publication and mention
of the grant of the patent:
**27.05.2020 Bulletin 2020/22**

(21) Application number: **16794072.5**

(22) Date of filing: **05.06.2016**

(51) Int Cl.:
**C12N 5/00** (2006.01)    **C12N 5/0783** (2010.01)
**C12N 5/078** (2010.01)    **A61K 35/12** (2015.01)

(86) International application number:
**PCT/IL2016/050578**

(87) International publication number:
**WO 2016/193986 (08.12.2016 Gazette 2016/49)**

(54) **SELECTIVE SURFACES FOR, AND METHODS OF, SELECTING A POPULATION OF STEM AND PROGENITOR CELLS, AND USES THEREOF**

SELEKTIVE OBERFLÄCHEN UND VERFAHREN ZUR AUSWAHL EINER POPULATION VON STAMM- UND VORLÄUFERZELLEN UND VERWENDUNGEN DAVON

SURFACES SÉLECTIVES ET PROCÉDÉS POUR LA SÉLECTION D'UNE POPULATION DE CELLULES SOUCHES ET PROGÉNITRICES ET UTILISATIONS ASSOCIÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.06.2015 US 201562171403 P**
**05.06.2015 US 201562171412 P**

(43) Date of publication of application:
**11.04.2018 Bulletin 2018/15**

(73) Proprietor: **Cellect Biotherapeutics Ltd.**
**Kfar Saba 4442510 (IL)**

(72) Inventors:
• **YARKONI, Shai**
**4439529 Kfar Saba (IL)**
• **ALMOG, Tal**
**4584500 Kibbutz Nir Elyau (IL)**

(74) Representative: **Vossius & Partner**
**Patentanwälte Rechtsanwälte mbB**
**Siebertstrasse 3**
**81675 München (DE)**

(56) References cited:
**WO-A1-2013/132477    US-A1- 2014 072 603**

• **THIRAWAN NIPITHAKUL ET AL: "Preliminary study of extractable protein binding using maleic anhydride copolymer", CHINESE JOURNAL OF POLYMER SCIENCE, vol. 30, no. 2, 9 December 2011 (2011-12-09), pages 181-189, XP055335698, CN ISSN: 0256-7679, DOI: 10.1007/s10118-012-1112-8**
• **TOSHIYA YOKOZAWA ET AL: "Gelatin beads as platforms for targeting molecule and anti-Fas antibody", EXPERIMENTAL HEMATOLOGY, vol. 28, no. 10, 1 October 2000 (2000-10-01), pages 1129-1136, XP055336201, US ISSN: 0301-472X, DOI: 10.1016/S0301-472X(00)00528-2**
• **B.D.S.Allan, J. Bi, J.T. Jacob, C.E.Futter: "Lens epithelial cells cultured on plastic are protected from Fas mediated apoptosis by conditioned media from lens epithelial cells cultured on collagen IV | IOVS | ARVO Journals", , 1 April 2004 (2004-04-01), XP055336161, Retrieved from the Internet: URL:http://iovs.arvojournals.org/article.a spx?articleid=2405886&resultClick=1 [retrieved on 2017-01-17]**

SEQUENCE LISTING

<110> Cellect Biotherapeutics Ltd.

<120> DEVICES HAVING A SELECTIVE SURFACE FOR, AND METHODS OF, SELECTING A POPULATION OF STEM AND PROGENITOR CELLS, AND USES THEREOF

<130> 2389086

<150> US62/171,403
<151> 2015-06-05

<150> US62/171,412
<151> 2015-06-05

<160> 4

<170> Patent In version 3.5

<210> 1
<211> 280
<212> PRT
<213> human

<400> 1

```
Met Gln Gln Pro Phe Asn Tyr Pro Tyr Pro Gln Ile Tyr Trp Val Asp
1               5               10              15

Ser Ser Ala Ser Ser Pro Trp Ala Pro Pro Gly Thr Val Leu Pro Cys
            20              25              30

Pro Thr Ser Val Pro Arg Arg Pro Gly Gln Arg Arg Pro Pro Pro
            35              40              45

Pro Pro Pro Pro Pro Leu Pro Pro Pro Pro Pro Pro Pro Pro Leu Pro
    50              55              60

Pro Leu Pro Leu Pro Pro Leu Lys Lys Arg Gly Asn His Ser Thr Gly
65              70              75              80

Leu Cys Leu Leu Val Met Phe Phe Met Val Leu Val Ala Leu Val Gly
            85              90              95

Leu Gly Leu Gly Met Phe Gln Leu Phe His Leu Gln Lys Glu Leu Ala
            100             105             110

Glu Leu Arg Glu Ser Thr Ser Gln Met His Thr Ala Ser Ser Leu Glu
        115             120             125

Lys Gln Ile Gly His Pro Ser Pro Pro Glu Lys Lys Glu Leu Arg
        130             135             140

Lys Val Ala His Leu Thr Gly Lys Ser Asn Ser Arg Ser Met Pro Leu
```

```
                 145                      150                      155                      160

        Glu Trp Glu Asp Thr Tyr Gly Ile Val Leu Leu Ser Gly Val Lys Tyr
                        165              170              175

        Lys Lys Gly Gly Leu Val Ile Asn Glu Thr Gly Leu Tyr Phe Val Tyr
                        180              185              190

        Ser Lys Val Tyr Phe Arg Gly Gln Ser Cys Asn Asn Leu Pro Leu Ser
                        195              200              205

        His Lys Val Tyr Met Arg Asn Ser Lys Tyr Pro Gln Asp Leu Val Met
                        210              215              220

        Met Glu Gly Lys Met Met Ser Tyr Cys Thr Thr Gly Gln Met Trp Ala
        225                  230              235                  240

        Arg Ser Ser Tyr Leu Gly Ala Val Phe Asn Leu Thr Ser Ala Asp His
                        245              250              255

        Leu Tyr Val Asn Val Ser Glu Leu Ser Leu Val Asn Phe Glu Glu Ser
                        260              265              270

        Gln Thr Phe Phe Gly Leu Tyr Lys
                        275              280
```

<210> 2
<211> 200
<212> PRT
<213> human

<400> 2

```
Met Ser Thr Glu Ser Met Ile Arg Asp Val Glu Leu Ala Glu Glu Ala
1               5                   10                  15

Leu Pro Lys Lys Thr Gly Gly Pro Gln Gly Ser Arg Arg Cys Leu Phe
                20                  25                  30

Leu Ser Leu Phe Ser Phe Leu Ile Val Ala Gly Ala Thr Thr Leu Phe
                35                  40                  45

Cys Leu Leu His Phe Gly Val Ile Gly Pro Gln Arg Glu Glu Phe Pro
        50                  55                  60

Arg Asp Leu Ser Leu Ile Ser Pro Leu Ala Gln Ala Val Arg Ser Ser
65                  70                  75                  80

Ser Arg Thr Pro Ser Asp Lys Pro Val Ala His Val Val Ala Asn Pro
                85                  90                  95

Gln Ala Glu Gly Gln Leu Gln Trp Leu Asn Arg Lys Gly Gln Gly Cys
                100                 105                 110

Pro Ser Thr His Val Leu Leu Thr His Thr Ile Ser Arg Ile Ala Val
        115                 120                 125

Ser Tyr Gln Thr Lys Val Asn Leu Leu Ser Ala Ile Lys Ser Pro Cys
    130                 135                 140

Gln Arg Glu Thr Pro Glu Gly Ala Glu Ala Lys Pro Trp Tyr Glu Pro
145                 150                 155                 160

Ile Tyr Leu Gly Gly Val Phe Gln Leu Glu Lys Gly Asp Arg Leu Ser
                165                 170                 175

Ala Glu Ile Asn Arg Pro Asp Tyr Leu Asp Phe Ala Glu Ser Gly Gln
                180                 185                 190

Val Tyr Phe Gly Ile Ile Ala Leu
    195                 200
```

<210> 3
<211> 181
<212> PRT
<213> human

<400> 3

```
Met Ala Met Met Glu Val Gln Gly Gly Pro Ser Leu Gly Gln Thr Cys
1               5                   10                  15

Val Leu Ile Val Ile Phe Thr Val Leu Leu Gln Ser Leu Cys Val Ala
            20                  25                  30

Val Thr Tyr Val Tyr Phe Thr Asn Glu Leu Lys Gln Met Gln Asp Lys
        35                  40                  45

Tyr Ser Lys Ser Gly Ile Ala Cys Phe Leu Lys Glu Asp Asp Ser Tyr
    50                  55                  60

Trp Asp Pro Asn Asp Glu Glu Ser Met Asn Ser Pro Cys Trp Gln Val
65                  70                  75                  80

Lys Trp Gln Leu Arg Gln Leu Val Arg Lys Met Ile Leu Arg Thr Ser
                85                  90                  95

Glu Glu Thr Ile Ser Thr Val Gln Glu Lys Gln Gln Asn Ile Ser Pro
            100                 105                 110

Leu Val Arg Glu Arg Gly Pro Gln Arg Val Ala Ala His Ile Thr Gly
            115                 120                 125

Thr Arg Gly Arg Ser Asn Thr Leu Ser Ser Pro Asn Ser Lys Asn Glu
    130                 135                 140

Lys Ala Leu Gly Arg Lys Ile Asn Ser Trp Glu Ser Ser Arg Ser Gly
145                 150                 155                 160

His Ser Phe Leu Ser Asn Leu His Leu Arg Asn Gly Glu Leu Val Ile
            165                 170                 175

His Glu Lys Gly Phe
            180
```

<210> 4
<211> 182
<212> PRT
<213> human

<400> 4

```
Met Ala Ala Arg Arg Ser Gln Arg Arg Arg Gly Arg Arg Gly Glu Pro
1               5               10              15

Gly Thr Ala Leu Leu Val Pro Leu Ala Leu Gly Leu Gly Leu Ala Leu
            20              25              30

Ala Cys Leu Gly Leu Leu Leu Ala Val Val Ser Leu Gly Ser Arg Ala
        35              40              45

Ser Leu Ser Ala Gln Glu Pro Ala Gln Glu Glu Leu Val Ala Glu Glu
    50              55              60

Asp Gln Asp Pro Ser Glu Leu Asn Pro Gln Thr Glu Glu Ser Gln Asp
65              70              75              80

Pro Ala Pro Phe Leu Asn Arg Leu Val Arg Pro Arg Arg Ser Ala Pro
            85              90              95

Lys Gly Arg Lys Thr Arg Ala Arg Arg Ala Ile Ala Ala His Tyr Glu
        100             105             110

Val His Pro Arg Pro Gly Gln Asp Gly Ala Gln Ala Gly Val Asp Gly
        115             120             125

Thr Val Ser Gly Trp Glu Glu Ala Arg Ile Asn Ser Ser Ser Pro Leu

    130             135             140

Arg Tyr Asn Arg Gln Ile Gly Glu Phe Ile Val Thr Arg Ala Gly Leu
145             150             155             160

Tyr Tyr Leu Tyr Cys Gln Val His Phe Asp Glu Gly Lys Ala Val Tyr
            165             170             175

Leu Lys Leu Asp Leu Leu
            180
```

**Description**

**FIELD OF THE INVENTION**

The present invention relates to the field of medical devices and more specifically, to selective surfaces configured to select for stem and progenitor cells that arc resistant to apoptosis signaling.

**BACKGROUND OF THE INVENTION**

Stem cells are cells that can both divide and differentiate into diverse specialized cells type and to self-renew. In adult organisms stem cells act to replenish tissues, and hence their potential efficacy in replacement therapy.
Graft versus host disease (GvHD) includes an acute phase reaction, usually within the first 100 days post-transplantation, and a chronic reaction with more indolent progression but equally detrimental consequences. Both reactions are triggered by initial inflammation mediated by mature donor T cells within days from transplantation. WO/2013/132477 discloses a container comprising a biologically active apoptosis-inducing ligand attached to a surface thereof.
US 2014/0072603 discloses biomaterials that interact with and regulate immune functions, as well as implantable materials and devices. It also discloses an implantable medical device comprising a biomaterial coated with one or more CD200 molecules while a method of treating inflammation by administering a composition comprising one or more biomaterials that inhibit immune reactivity is also disclosed.

**SUMMARY OF THE INVENTION**

The present invention relates to the embodiments as characterized in the claims. Thus, it relates to the following items:

1. A selective surface, wherein said selective surface comprising:

   a biocompatible polymer comprising maleic anhydride molecules; and
   an apoptosis inducing ligand bound to the maleic anhydride molecules; wherein said biocompatible polymer is polyethylene or a fluorocarbon polymer;
   and wherein the apoptosis-inducing ligand is FasL.

2. The selective surface of item 1,wherein said selective surface is capable of selecting apoptosis-signaling resistant cells.

3. The selective surface of any one of the preceding items wherein said selective surface is in the form of an inner surface of a container, a film, a disc or beads.

4. The selective surface of any one of the preceding items wherein said fluorocarbon polymer is Ethylene tetrafluoroethylene (ETFE).

5. The selective surface of item 3 wherein said beads are selected from the group consisting of magnetic beads, paramagnetic beads, and dextran beads.

6. The selective surface of item 3 wherein said beads are agarose beads, polystyrene beads, sepharose beads or silica beads bound to N-hydroxysuccinimide (NHS), NHS-activated agarose beads or beads comprising aldehyde-activated agarose, azlactone-activated support, carbonyl diimidzaole agarose/Trisacryl, carboxyl groups, cyanogen bromide, polyethylene glycol (PEG), and any combination thereof.

7. The selective surface of any one of the preceding items, wherein the FasL is biologically active.

8. The selective surface of any one of the preceding items wherein from about 0.01 to about 3ng/cm$^2$ of FasL is bound to the selective surface.

9. A device comprising the selective surface of any one of the preceding items.

10. The device of item 9 wherein said device is a container.

11. The device of any one of items 9 to 10 wherein the selective surface is the selective surface of a plurality of beads contained within the container.

12. The selective surface of any one of items 1 to 8 or the device of any one of items 9 to 11 for use in selecting an apoptosis-signaling resistant cell.

13. Use of the selective surface of any one of items 1 to 8 or the device of any one of items 9 to 11 in a method of selecting an apoptosis-signaling resistant cell.

14. A method of preparing a device having a selective surface, said method comprising:

(i) Obtaining a selective surface comprising a biocompatible polymer coated with or comprising maleic anhydride molecules (maleic anhydride-copolymer); wherein said biocompatible polymer is polyethylene or a fluorocarbon polymer;
(ii) incubating said selective surface with an immobilization buffer or coating buffer comprising dissolved FasL;
(iii) removing the unbound FasL from the selective surface; thereby obtaining a device having a selective surface comprising a biocompatible polymer coated with or comprising maleic anhydride molecules (maleic anhydride-copolymer), and wherein said surface further having FasL immobilized thereon;
wherein said biocompatible polymer is polyethylene or a fluorocarbon polymer.

15. A cell selection kit comprising:

a. the selective surface of any one of items 1 to 9 or the device of any one of items 9 to 11; and
b. Instructions for use in the selection of stem and progenitor cells which are resistant to receptor-mediated apoptosis.

Moreover, there are disclosed method(s) that include preparing a specific selective surface comprising an apoptosis inducing ligand immobilized thereon, whereby the apoptosis inducing ligand maintains its biological activity, e.g. maintains the ability to induce apoptotic cell death and wherein the selective surface is composed of a biocompatible polymer comprising maleic anhydride molecules. Thus, there are disclosed a selective surface, wherein said selective surface comprising a biocompatible polymer comprising maleic anhydride molecules and an apoptosis inducing ligand bound to the maleic anhydride molecules.
It is also disclosed that said selective surface may be capable of selecting apoptosis-signaling resistant cells.
Said apoptosis-signaling resistant cells may be selected from the group consisting of stem-cells, progenitor cells and immune cells.
Said apoptosis-signaling resistant cells may be selected from the group consisting of umbilical cord blood stem and/or progenitor cells, mobilized peripheral blood stem and/or progenitor cells, bone marrow stem and/or progenitor cells, hematopoietic stem cells, cancer stem and/or progenitor cells, and neural stem and/or progenitor cell.
Said apoptosis-signaling resistant cells may be leukocytes (CD45+ cells) or a subset of leukocytes.
Said apoptosis-signaling resistant cells may be T cells or a subset of T-cells.
Said immune cells may be immune cells insensitive to activation-induced cell death (AICD).
Said immune cells insensitive to activation-induced cell death (AICD) may be leukocytes.
Said immune cells insensitive to activation-induced cell death (AICD) may be T cells.
Said apoptosis-signaling resistant cells may be hematopoietic stem cells.
Said apoptosis-signaling resistant cells may be identified on the basis of a surface phenotype.
Said surface phenotype may be CD34, and said cells may be CD34+ cells.
Said selective surface may be in the form of a film, a disc or beads.
The biocompatible polymer may be selected from the group consisting of polypropylene, polystyrene, silicone, polyvinyl chloride, polyethylene, polyurethane. ethylene vinyl acetate (EVA), fluoro carbon polymers, polyethylene glycol, polyethylene terephthalate (PET), Poly(2-hydroxyethyl methacrylate), Poly(N-2-hydroxypropyl methacrylamide), dimethacrylate, PEG-dMA, Poly(ethylene glycol) diacrylate (PEG-dA), pHEMA, pHPMA, and any combination thereof.
Said biocompatible polymer may be polyethylene or a fluorocarbon polymer.
Said fluorocarbon polymer may be Ethylene tetrafluoroethylene (ETFE), fluorinated ethylene propylene (FEP), or perfluoroalkoxy polymer (PFA).
The biocompatible material may be selected from the group consisting of aldehyde-activated agarose, azlactone-activated support, carbonyl diimidzaole agarose/Trisacryl. carbonyl diimidazole, carboxyl groups, azlactone. cyanogen bromide, hydroxyl groups, epoxy groups, amine groups, or any combination thereof.
Said beads may be selected from the group consisting of magnetic beads, paramagnetic beads, and dextran beads.

Said dextran beads may have an iron oxide or hydroxide core.

Said beads may be agarose beads, polystyrene beads, sepharose beads or silica beads bound to N-hydroxysuccinimide (NHS), or beads comprising aldehyde-activated agarose, azlactone-activated support, carbonyl diimidzaole agarose/Trisacryl, carboxyl groups, cyanogen bromide, polyethylene glycol (PEG), and any combination thereof.

Said beads may be NHS-activated agarose beads.

Said beads may be uniformly composed.

Said beads may be non-uniformly composed.

Said beads may be a mixture of uniformly and non-uniformly composed beads.

The apoptosis-inducing ligand may be TNFα, FasL, Trail, Tweak, or any combination thereof.

The TNFα, FasL, Trail, or Tweak may be biologically active.

From about 0.01 to about $3ng/cm^2$ of FasL or TNFα, may be bound to the selective surface.

Said FasL may be a FasL fusion protein,

Said FasL fusion protein may be a tagged FasL.

Said tagged FasL may be fused with a tag at the N-terminus, at the C-terminus, within the FasL protein, or any combination thereof.

Said tag may be selected from the group consisting of His-tag, FLAG-tag, MBP-tag and streptavidin-tag.

The activity and/or binding affinity of said FasL fusion protein may be substantially the same as or higher than that of native FasL, namely FasL in the non-fused form.

Said FasL may be a modified FasL protein, and the activity and/or binding affinity of said modified FasL protein is substantially the same as that of native FasL, namely FasL in the non-modified form.

Said modified FasL protein may be a truncated FasL protein, and the activity and/or binding affinity of said truncated FasL protein is substantially the same as that of native FasL, namely FasL in the non-truncated form.

Said TNFα may be a TNFα fusion protein.

Said TNFα fusion protein may be a tagged TNFa.

Said tagged TNFα may be fused with a tag at the N-terminus, at the C-terminus, within the TNFα protein, or any combination thereof.

Said tag may be selected from the group consisting of His-tag, FLAG-tag, MBP-tag and streptavidin-tag.

Said tagged TNFα may be streptavidin-TNFa.

The activity and/or binding affinity of said TNFα fusion protein may substantially be the same as that of native TNFα, namely TNFα in the non-fused form.

Said TNFα may be a modified TNFα protein, and the activity and/or binding affinity of said modified TNFα protein is substantially the same as that of native TNFα, namely TNFα in the non-modified form.

Said modified TNFα protein may be a truncated TNFα protein.

In another aspect, a device is disclosed that comprises the selective surface.

Said device may be a container.

Said container may be selected from the group consisting of a bag, a plate, a multi-well plate, a column, a tube, a bottle, a drum, a vial and a flask.

The inner surface of the container may be coated with the selective surface.

The selective surface of the container may be the selective surface of a plurality of beads contained within the container.

In another aspect, the selective surface or the device are for use in selecting an apoptosis-signaling resistant cell.

In another aspect, there is disclosed the use of the selective surface or the device in a method of selecting an apoptosis-signaling resistant cell.

In another aspect, there is disclosed a method of preparing a device having a selective surface, said method comprising:

(i) Obtaining a selective surface comprising a biocompatible polymer coated with or comprising maleic anhydride molecules (maleic anhydride-copolymer);

(ii) incubating said selective surface with an immobilization buffer or coating buffer comprising at least one dissolved apoptosis inducing ligand;

(iii) removing the unbound at least one dissolved apoptosis inducing ligand from the selective surface;

thereby obtaining a device having a selective surface comprising a biocompatible polymer coatcd with or comprising maleic anhydride molecules (maleic anhydride-copolymcr), and wherein said surface further having an apoptosis inducing ligand immobilized thereon.

The method may further comprise:

(iv) adding a protein blocking buffer to the selective surface after step (iii);

(v) optionally incubating the selective surface with the protein blocking buffer; and

(vi) washing the selective surface.

The method may further comprise activating a group included in the surface prior to step (ii).

Said group may be a carboxyl group.

Said apoptosis inducing ligand may be fasL or TNFα.

Also disclosed is a cell selection kit comprising:

a. the selective surface or the device; and

b. Instructions for use in the selection of stem and progenitor cells which are resistant to receptor-mediated apoptosis.

The cell selection kit may further comprise a solution for maintaining the integrity and/or activity of the apoptosis-inducihg ligand within the device.

The solution for maintaining the integrity and/or activity of the apoptosis-inducing ligand may be a buffer and/or a medium.

In another aspect, there is disclosed a method of selecting apoptosis-signaling resistant **cells** or enriching a heterogeneous population of cells with apoptosis-signaling resistant cells, said method comprising:

a. Obtaining the selective surface or the device;

b. contacting a sample comprising heterogeneous population of cells comprising apoptosis-signaling resistant cells and apoptosis-signaling sensitive cells with the surface or the device;

c. incubating said heterogeneous population of cells with the surface or in the device thereby exposing the cells to the apoptosis inducing ligand immobilized on the selective surface; and

d. retrieving the viable cells that have not undergone apoptosis; thereby selecting apoptosis-signaling resistant cells from the heterogeneous population or enriching the heterogeneous population of cells with apoptosis-signaling resistant cells.

Said incubating step on the selective surface or in the device may be from about 2 hours to about 72 hours.

Said apoptosis inducing ligand is FasL and said incubating step on the selective surface or in the device may be from about 21 hours to about 24 hours.

Said apoptosis inducing ligand is TNF-α and said incubating step on the selective surface or in the device may be from about 24 hours to about 48 hours.

Said incubating step on the selective surface or in the device may comprise sequential incubations of the cells on a selective surface comprising FasL and on a selective surface comprising TNF-α, or vice versa.

Said incubating step on the selective surface or in the device may comprise incubation of the cells on a selective surface comprising FasL and TNF-α.

Said apoptosis-signaling resistant cells may be stem cells and/or progenitor cells.

As disclosed herein, said retrieved cells may be used for autologous, allogeneic or haploidentical transplantation in a subject in need thereof.

There is also disclosed a method for improving the clinical outcome of hematopoietic stem and progenitor cells (HSPC) transplantation, said method comprising:

a. Obtaining the selective surface or the device;
b. contacting a sample comprising HSPC with the surface or the device;
c. incubating said sample with the surface or in the device thereby exposing the cells to the apoptosis inducing ligand immobilized on the selective surface;
d. retrieving the viable HSPC that have not undergone apoptosis thereby obtaining an enriched population of HSPC; and
e. transplanting the retrieved enriched population of HSPC in a subject in need thereof

thereby improving the clinical outcome of HSPC transplantation.

In another aspect, there is also disclosed a method for eliminating at least one malignant cell in a composition comprising a stem and/or progenitor cell transplant, said method comprising:

a. Obtaining the selective surface or the device;

b. contacting a composition comprising a stem and/or progenitor cell transplant optionally comprising malignant cells with the surface or the device;

c. incubating said composition with the surface or in the device thereby exposing the cells to the apoptosis inducing ligand immobilized on the selective surface;

d. retrieving the viable stem and/or progenitor cells of the cell transplant that have not undergone apoptosis thereby obtaining a depleted population of stem and/or progenitor cells from which apoptosis-sensitive malignant cells were at least partially eliminated; and

e. transplanting the retrieved depleted population of stem and/or progenitor cells in a subject in need thereof.

Said apoptosis inducing ligand is FasL or TNF-α and said incubating step may be for about 24 hours.

Said depleted population of stem and/or progenitor cells may be used for autologous transplantation in a subject in need thereof.

As disclosed herein, said subject may be a human.

There is also disclosed a method for reducing a transplant patient's likelihood of having graft vs. host disease (GvHD) while retaining graft vs. tumor (GvT) activity, said method comprising:

a. Obtaining the selective surface or the device;

b. contacting a cell population comprising HSPC and immune cells with the surface or the device;

c. incubating said cell population with the surface or in the device thereby exposing the cells to the apoptosis inducing ligand immobilized on the selective surface;

d. retrieving the viable cells that have not undergone apoptosis thereby obtaining an enriched population of HSPC from which apoptosis-sensitive immune cells were at least partially eliminated; and

e. transplanting the retrieved enriched population of HSPC into the transplant patient in need thereof.

Said apoptosis inducing ligand may be FasL or TNF-α and said incubating step is for about 2 to about 16 hours.

There is also disclosed a selective surface, wherein said selective surface comprising:

at least one fluorocarbon polymer; and

an apoptosis inducing ligand bound directly or indirectly to the fluorocarbon polymer.

Said fluorocarbon polymer may further comprise an agent which mediates the binding of the apoptosis inducing ligand, wherein said agent is selected from the group consisting of an organic chemical, a protein, a protein fragment, a linker, a polynucleotide, or any combination thereof and wherein the apoptosis inducing ligand is bound indirectly to the fluorocarbon via said agent.

Said fluorocarbon polymer may comprise maleic anhydride molecules and said apoptosis-inducing ligand is bound to the maleic anhydride molecules.

Said selective surface may be capable of selecting apoptosis-signaling resistant cells.

Said apoptosis-signaling resistant cells may be selected from the group consisting of stem-cells, progenitor cells and immune cells.

Said apoptosis-signaling resistant cells may be selected from the group consisting of umbilical cord blood stem and/or progenitor cells, mobilized peripheral blood stem and/or progenitor cells, bone marrow stem and/or progenitor cells, cancer stem and/or progenitor cells, and neural stem and/or progenitor cell.

Said selective surface may be in the form of a film, a disc or beads.

Said fluorocarbon polymer may be Ethylene tetrafluoroethylene (ETFE), Fluorinated ethylene propylene (FEP), or perfluoroalkoxy polymer (PFA).

Said beads may be selected from the group consisting of magnetic beads, paramagnetic beads, and dextran beads.

Said dextran beads may have an iron oxide or hydroxide core.

Said beads may be agarose ; beads, polystyrene beads, sepharose beads or silica beads bound to N-hydroxysuccinimide (NHS), or beads comprising aldehyde-activated agarose, azlactone-activated support, carbonyl diimidazole, agarose/Trisacryl, carbonyl diimidazole, carboxyl groups, azlactone, cyanogen bromide hydroxyl groups, epoxy groups, amine groups, polyethylene glycol (PEG), and any combination thereof.

The apoptosis-inducing ligand may be TNFα, FasL, Trail, Tweak, or any combination thereof.

## DESCRIPTION OF FIGURES

The present invention will be further explained with reference to the attached drawings, wherein like structures are referred to by like numerals throughout the several views. The drawings shown are not necessarily to scale, with emphasis instead generally being placed upon illustrating the principles of the present invention. Further, some features may be exaggerated to show details of particular components.

The figures constitute a part of this specification and include illustrative embodiments of the present invention and illustrate various objects and features thereof. Further, the figures are not necessarily to scale, some features may be exaggerated to show details of particular components. In addition, any measurements, specifications and the like shown in the figures are intended to be illustrative, and not restrictive. Therefore, specific structural and functional details disclosed herein arc not to be interpreted as limiting, but merely as a representative basis for teaching one skilled in the art to variously employ the present invention.

Among those benefits and improvements that have been disclosed, other objects and advantages of this invention will become apparent from the following description taken in conjunction with the accompanying figures. Detailed embodiments of the present invention are disclosed herein: however, it is to be understood that the disclosed embodiments are merely illustrative of the invention that may be embodied in various forms. In addition, each of the examples given in connection with the various embodiments of the invention is intended to be illustrative, and not restrictive.

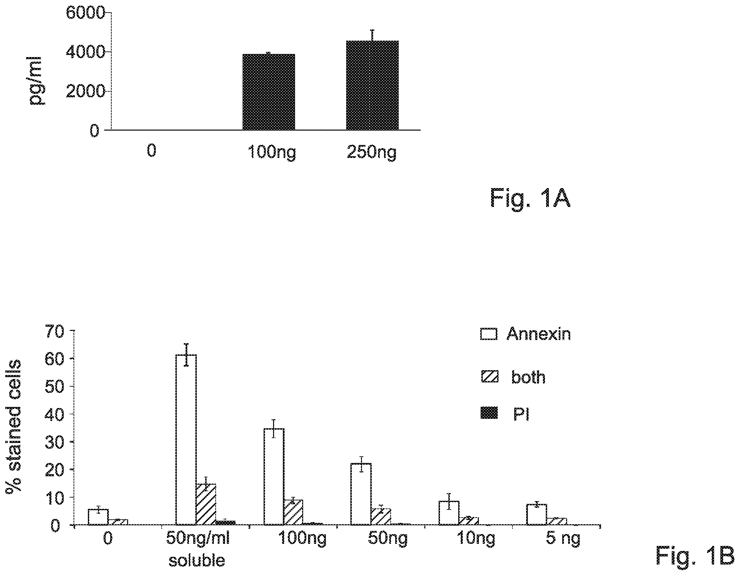

Figure 1: Figures 1A - 1B are graphs showing the instant compositions and methods of the present invention. Figure 1A is a graph showing the binding of superFasL to MA-coated surfaces. 100ng or 250 ng represent the amount of superFasL added to each well for binding; 0 - no superFasL added. Figure 1B shows a graph indicating dose-dependence of FasL regarding apoptotic activity.

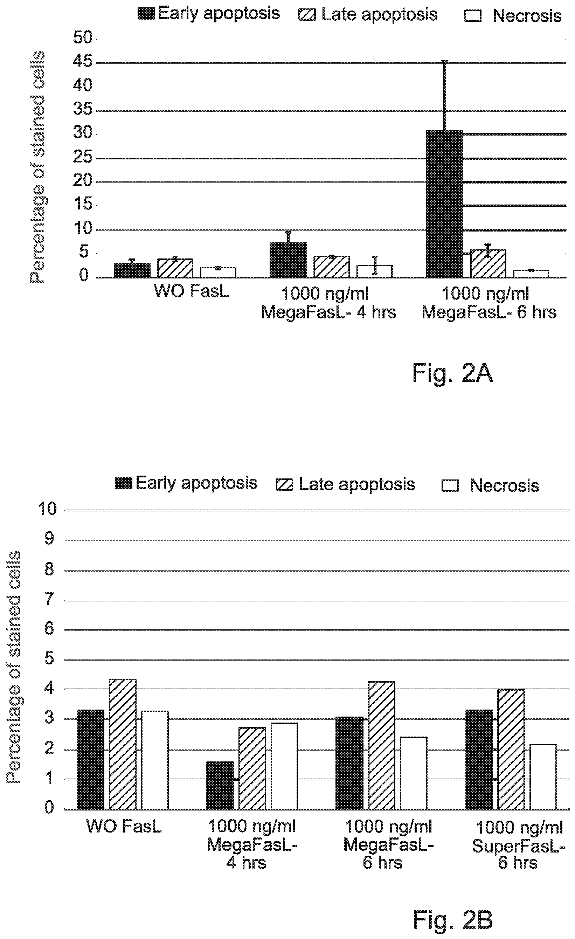

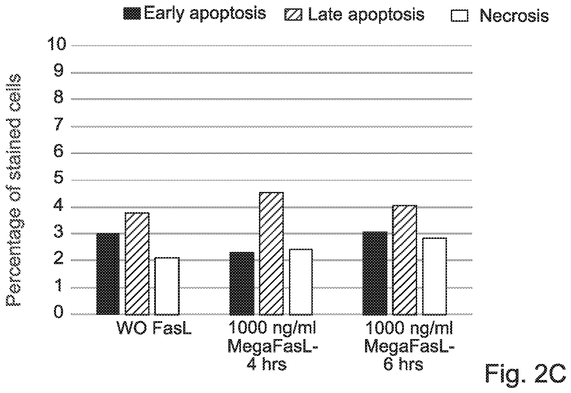

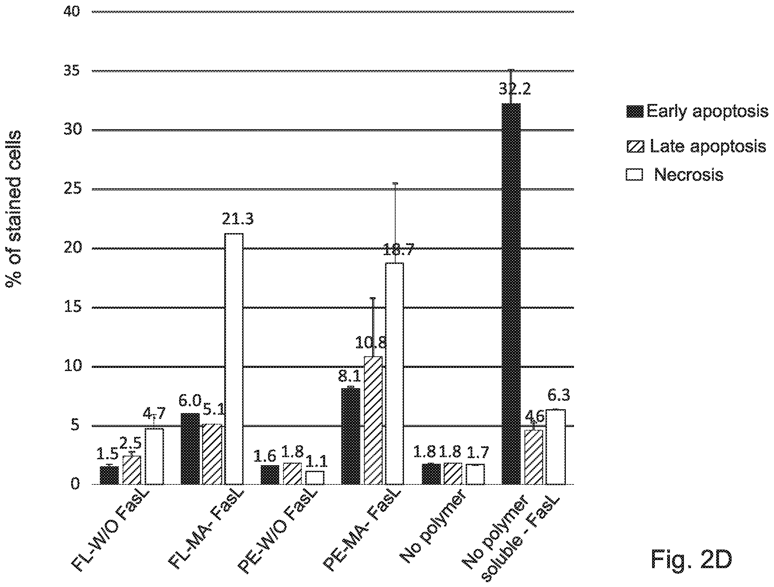

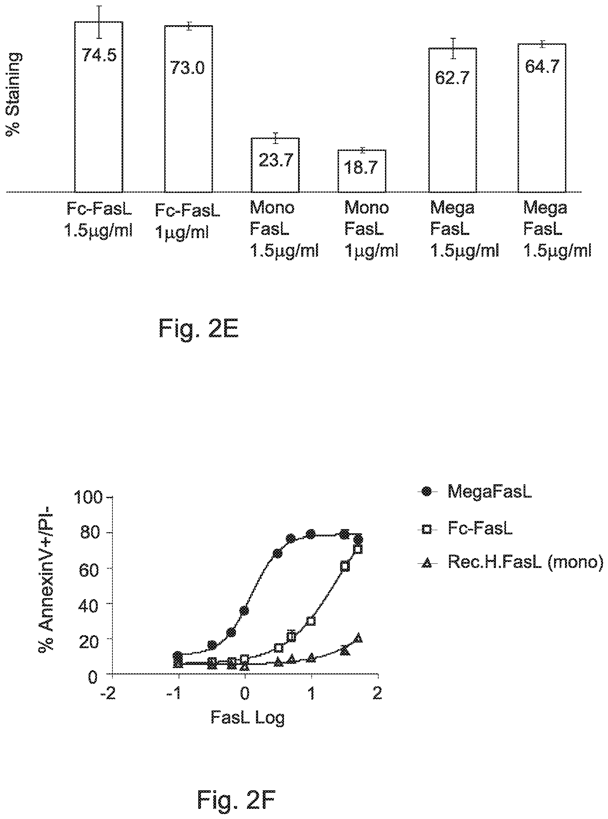

Figure 2: Figures 2A - 2F are graphs showing the instant compositions and methods of the present invention. Figure 2A shows results of an experiment using maleic anhydride (MA) coated plates that were incubated with MegaFasL (1000ng/mL). Jurkat cells were seeded and incubated for 4 or 6 hours on the MA-MegaFasL coated plates. Figure 2A shows an increase of 2.4+0.7 fold in the levels of early apoptotic cells following a 4 hour incubation and a $10.1\pm4.9$ fold increase in the levels of early apoptotic cells following 6 hours of incubation. Figure 2B shows results of an experiment using Nunc amino-immobilizer coated plates that were incubated with MegaFasL (1000ng/mL). Figure 2C shows results of an experiment using epoxy PolyAn coated plates that were incubated with MegaFasL (1000ng/mL). Figures 2A-C illustrate that MA was the only polymer that upon binding of FasL facilitates FasL induced apoptosis in human Jurkat cells. Figure 2D shows results of an experiment using fluoropolymer-MA and Polyethylene-MA bound FasL, in which the fluoropolymer-MA and Polyethylene-MA bound FasL induced apoptosis in human Jurkat cells. The density of MA per square nm is about 1.7, and can be used within a range of 0.01 - 3.5 MA/nm$^2$, where the increase to up to 3.5 MA/nm$^2$ can be performed with or without a microcarrier. Figure 2E is a graph indicating the level of apoptosis of Jurkat cells (as measured by % of cells stained with Annexin V) seeded on plates containing various amounts and various types of immobilized FasL. Figure 2F is a graph indicating the level of apoptosis of Jurkat cells (as measured by % of cells stained with Annexin V) incubated in the presence of soluble FasL molecules (shown as the EC50 of the various FasL).

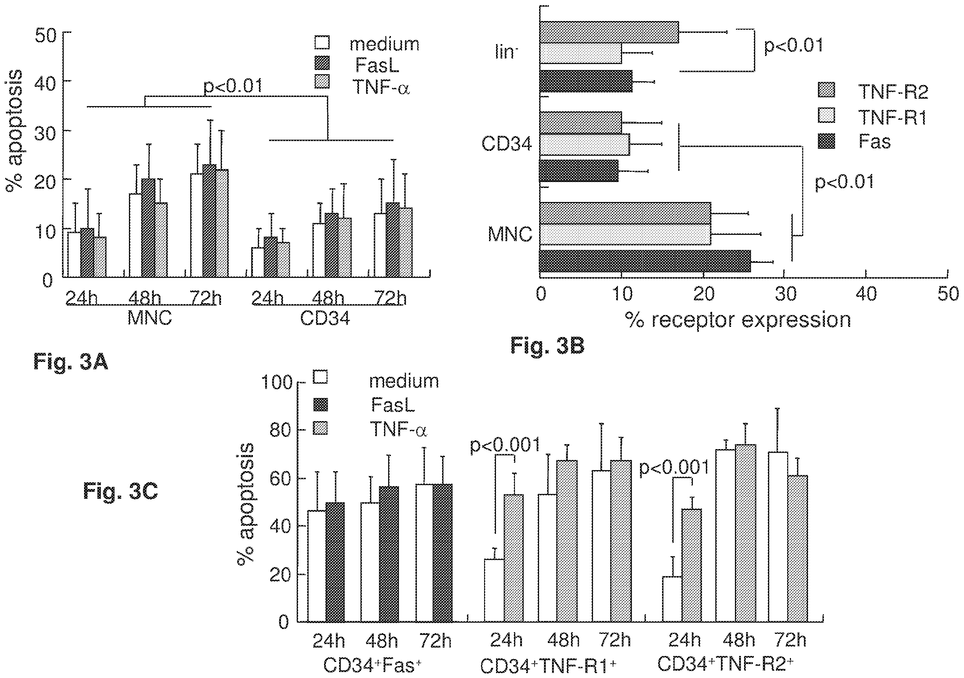

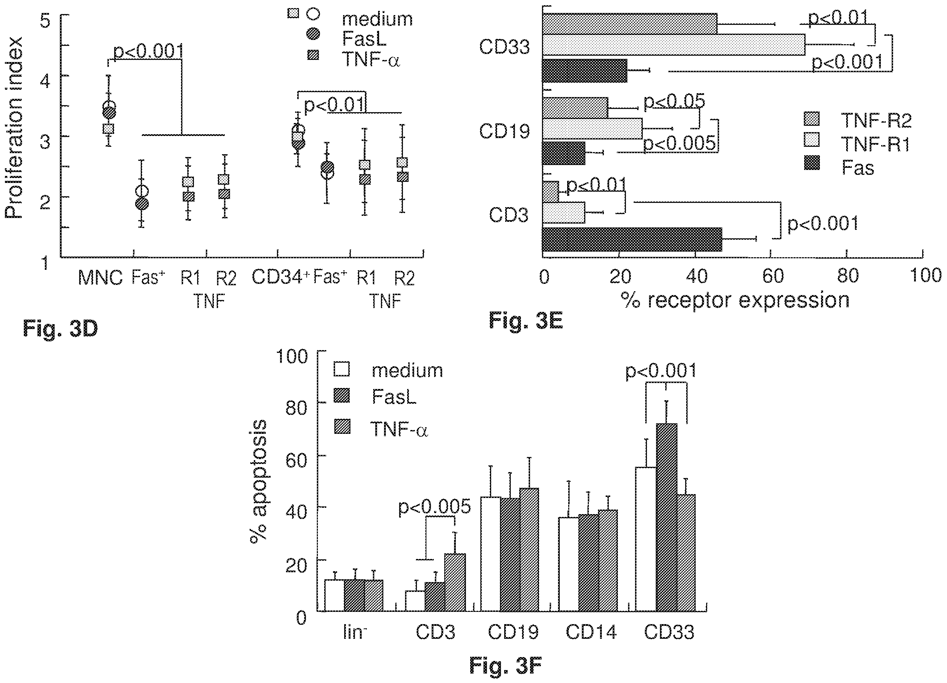

Figure 3: Figures 3A-3F are a set of graphs that relate to an embodiment of the present invention, showing the sensitivity of UCB cells to receptor- mediated apoptosis in vitro. (A) Fresh samples of UCB cells were incubated in medium without chemokine supplements for variable periods of time (n=35) and were exposed to 50 ng/ml FasL oligomers (n=21) and 20 ng/ml TNF-alpha (n=18). Apoptosis was determined by Annexin-V staining in mononuclear cells (MNC) and gated CD34+ progenitors. (B) Expression of the Fas and TNF receptors in fresh UCB mononuclear cells, gated CD34+ and isolated lineage-negative (lin-) progenitors. (C) Apoptosis was measured and is shown in the graph as a function of the incubation time with the various ligands in gated CD34+ progenitors expressing Fas and the TNF receptors under the influence of the cognate ligands (n=15-21). (D) Proliferation rates in MNC and gated CD34+ progenitors expressing Fas and the TNF receptors incubated with the cognate ligands (n=6). Proliferation was measured from CFSE dilution using the ModFit software. (E) Expression of Fas and TNF receptors in T cells (CD3+), B lymphocytes (CD19+) and myeloid cells (CD33+) in fresh UCB samples (n=8-13). (F) Apoptosis as determined from Annexin-V staining in lineage-positive UCB subsets, including monocytes/macrophages (CD14+), exposed for 48 hours to FasL and TNF- (n=6-14).

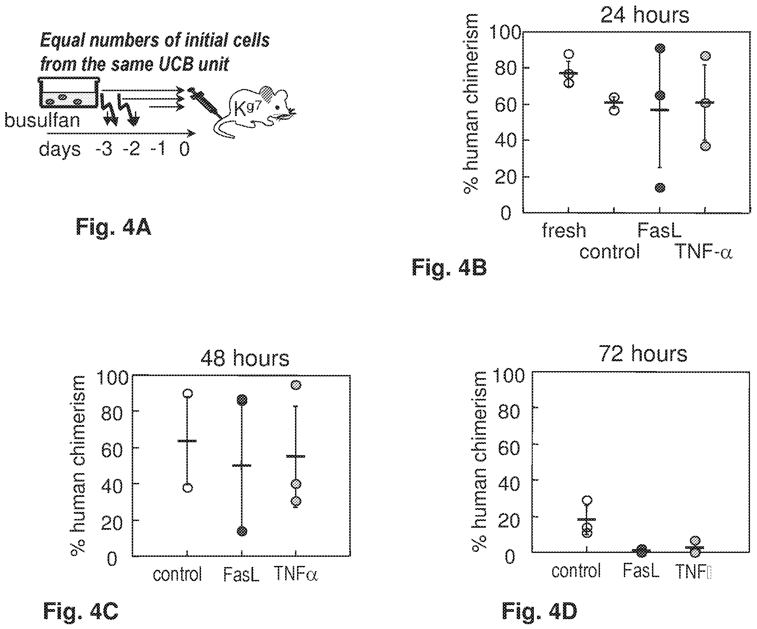

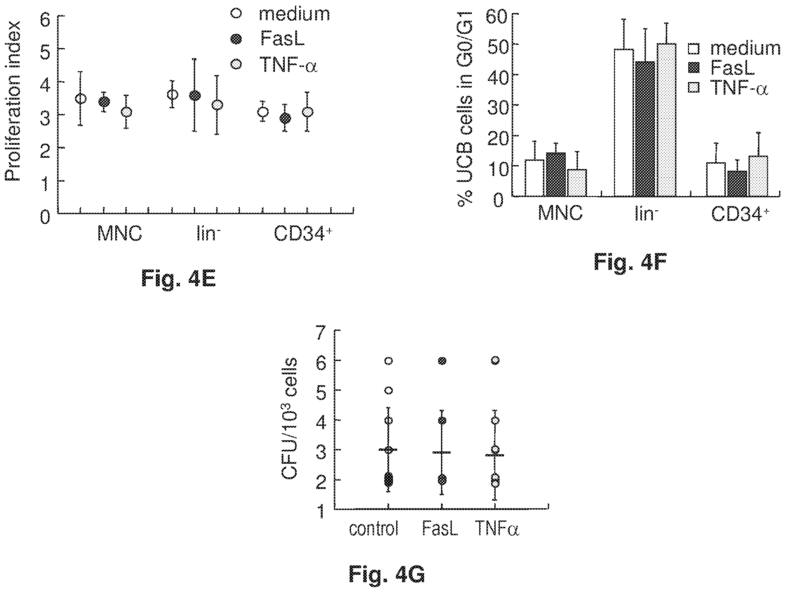

Figure 4 Figure 4A is a scheme and figures 4B-4G are graphs that relate to an embodiment of the present invention, showing that UCB progenitors are resistant to receptor-mediated apoptosis. (A) NOD.SCID mice were conditioned with two doses of 25 $\mu$g/g busulfan and were grafted after 2 days with equal numbers of UCB cells incubated under different conditions. Human chimerism was measured in the bone marrow after 12 weeks using selective human and murine anti-CD45 antibodies. (B) Engraftment of fresh UCB cells and following 24 hours of incubation in medium, with 50 ng/ml FasL and 20 ng/ml TNF-$\alpha$ from the same UCB unit. Data are representative of 7 different UCB samples. (C) Engraftment following 48 hours of incubation in medium, with FasL and TNF$\alpha$ from the same UCB unit (representative of 6 UCB samples). (D) Incubation for 72 hours in medium, with FasL and TNFα from the same UCB unit diminishes engraftment (representative of 5 UCB samples). (E) Proliferation rates of mononuclear cells (MNC), gated CD34[+] and isolated lineage- negative (lin[-]) progenitors after 48 hours of incubation in medium, with FasL and TNF-α (n=4-7). (F) Fractions of mitotically-quiescent UCB subsets positioned in the G0/G1 cell cycle phase as determined from nuclear incorporation of propidium iodide (n=5-8). (G) Long-term culture initiating cell (LTC-IC) frequency determined after plating of $10^3$ UCB cells over mesenchymal stromal cell layers for 5 weeks and subsequently transferred to semisolid methylcellulose cultures. FasL (50 ng/ml) and TNF-α (20 ng/ml) were present throughout the entire culture period and were refreshed at weekly intervals by exchange of half of the medium (n=8-13). Data represent comparative culture conditions of one UCB unit.

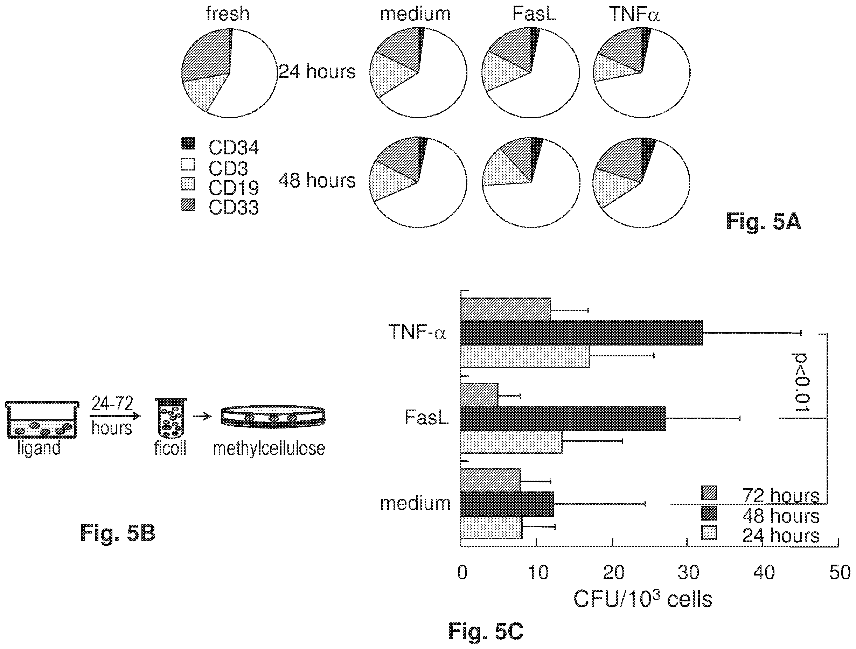

Figure 5: Figure 5B is a scheme and figures 5A and 5C are graphs that relate to an embodiment of the present invention, showing that exposure of UCB cells to death ligands increases the frequency of progenitors *in vitro* and myeloid differentiation *in vivo*. (A) Expression of lineage markers in fresh UCB cells: progenitors (CD34), T cells (CD3), B lymphocytes (CD19) and myeloid cells (CD33). The composition of viable cells changes after incubation for 24 and 48 hours with exposure to 50 ng/ml FasL and 20 ng/ml TNF-α, increasing the fraction of progenitors (n=17-31). (B) Following incubation for variable periods of time the dead cells were eliminated by centrifugation over ficoll gradient and equal numbers of viable cells were plated in semisolid methylcellulose cultures stimulated with stem cell factor (SCF), interleukin-3 (IL-3) and granulocyte-macrophage colony stimulating factor (GM-CSF). The frequency of colony forming cells (CFU, expressed per $10^3$ viable cells) was determined after 14 days. (C) Relative CFU frequencies in fresh UCB samples (control) and after incubation in medium, with FasL and TNF-α (n= 12-27).

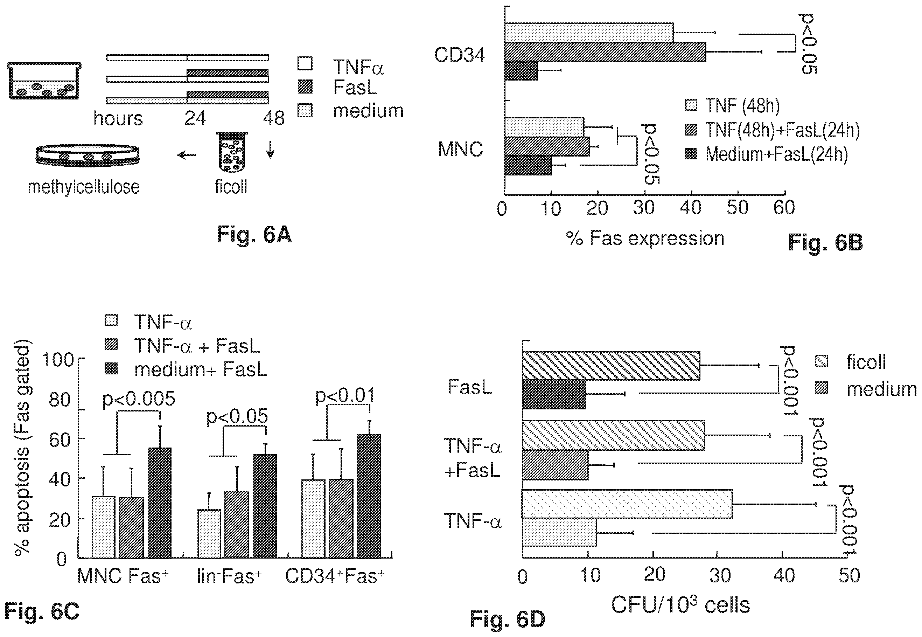

Figure 6: Figure 6A is a scheme and figures 6B-6D are graphs that relate to an embodiment of the present invention, showing the combined effects of death ligands on UCB cells. (A) Expression of Fas was stimulated by incubation with 20 ng/ml TNF-α for 24 hours and subsequently cells were incubated for additional 24 hours with and without 50 ng/ml FasL. (B) Fas expression in mononuclear cells (MNC) and gated CD34[+] progenitors under the various incubation conditions (n=6-9). (C) Apoptosis of Fas-positive MNC, gated CD34[+] and isolated lineage-negative (lin[-]) progenitors during 48 hours of incubation with TNF-α, with and without supplementation of FasL (n=5-12). (D) CFU frequencies in bulk cell suspensions after various incubations increases after elimination of dead cells by centrifugation over ficoll (n-7-15).

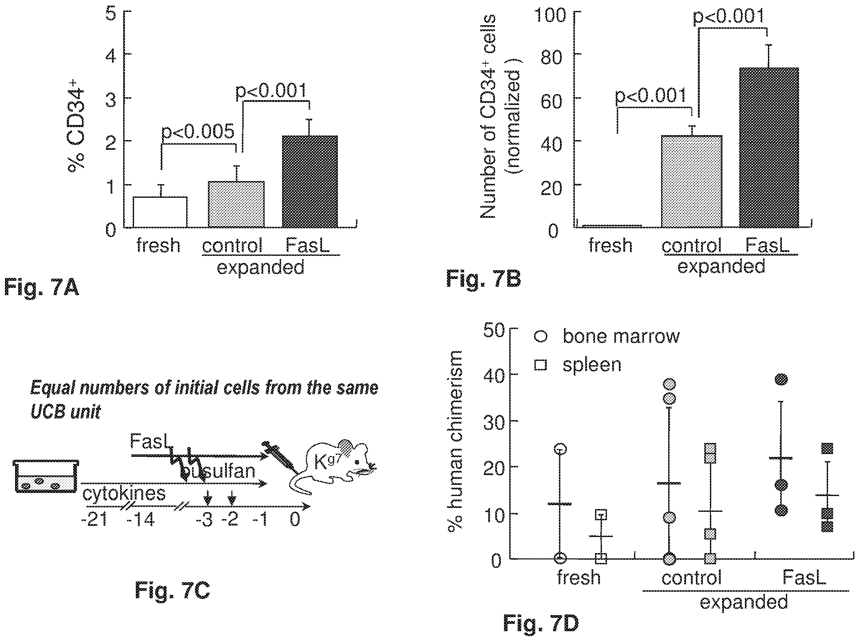

Figure 7: Figure 7C is a scheme and figures 7A, 7B and 7D are graphs that relate to an embodiment of the present invention, showing that death ligands increase progenitor frequency during *ex vivo* expansion of UCB cells. (A) CD34[+] progenitors were immunomagnetically isolated and expanded under a clinically approved protocol for 3 weeks in liquid culture. The fraction of CD34[+] progenitors increased during expansion as compared to the fresh UCB sample and further increased upon supplementation of 50 ng/ml FasL during the third and final week of culture (n=4). (B) Absolute numbers of CD34[+] cells in cultures after 3 weeks of expansion (n=4) normalized for $10^3$ total cells. (C) Equal numbers of fresh and expanded UCB cells (from same sample) were grafted into NOD.SCID mice (H2K[87]) conditioned with two doses of 25 µg/g busulfan. (D) Human hematopoietic chimerism was determined in the bone marrow and spleen after 12 weeks. Data are representative of 6 independent UCB units comparing fresh cells, expanded progenitors with and without FasL during the final third week of culture.

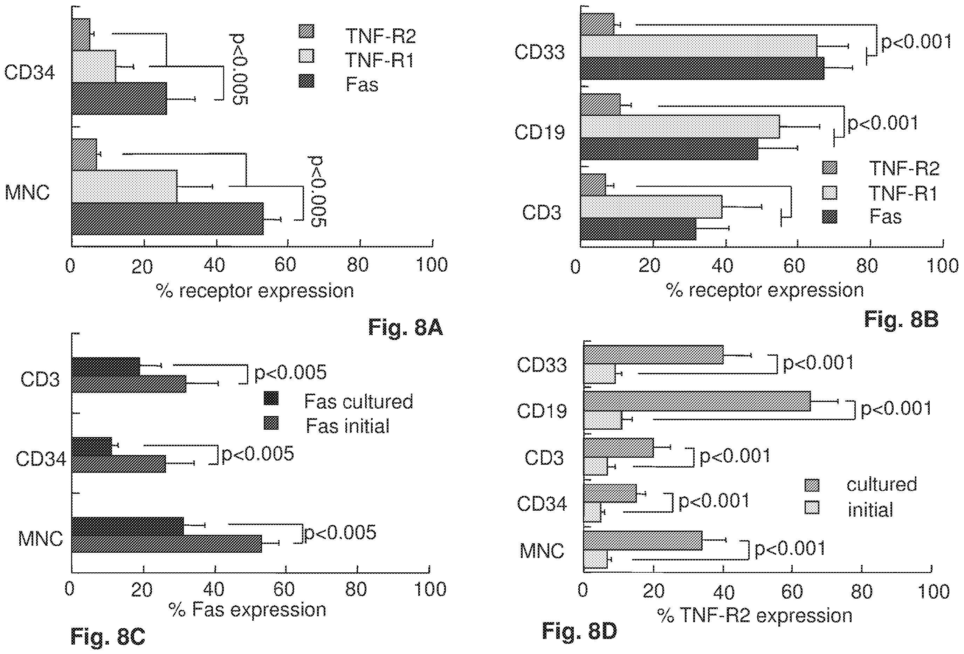

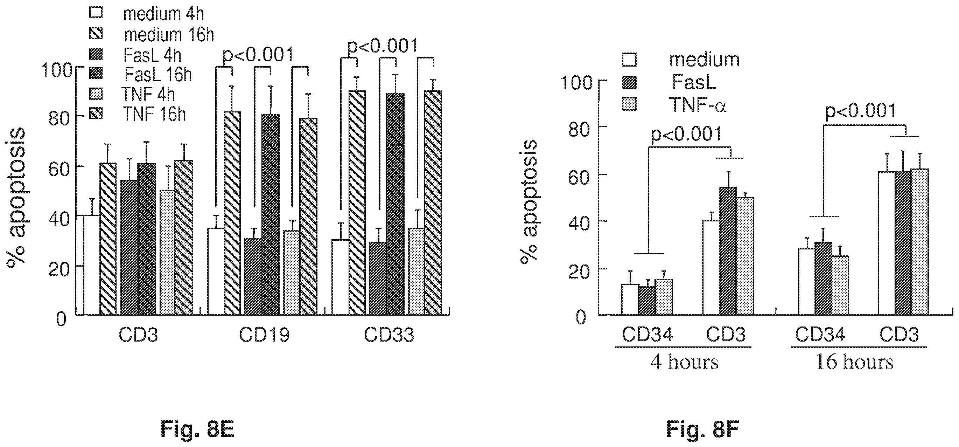

Figure 8: Figures 8A-8F are graphs that relate to an embodiment of the present invention, showing the expression of death receptors and sensitivity to apoptosis of mPB cells during brief culture. mPB samples cryopreserved for 2-7 years were thawed, excess DMSO was removed and cells were incubated for 4 and 16 hours with and without 50 ng/ml FasL and 29 ng/ml TNF-α. (A) Expression of Fas and TNF receptors in mononuclear (MNC) mPB cells and gated CD34[+] progenitors (n=7-11). (B) Comparative analysis of Fas and TNF receptor expression in lineage-positive T cells (CD3), B lymphocytes (CD19) and myeloid cells (CD33) (n=7-11). (C) Fas expression decreases substantially during 16 hours of culture in all subsets (n=5-9). (D) Upregulation of TNF-R2 during culture most prominent in B lymphocytes (CD19) and myeloid cells (CD33) (n=7-11). (E) Thawed mPB samples were incubated for 4 and 16 hours with and without FasL and TNF-α (n=5-11). Apoptosis was determined from Annexin-V incorporation. (F) Comparative rates of apoptosis of gated CD34[+] progenitors and CD3[+] T cells following incubation of thawed mPB for 4 and 16 hours with and without the death ligands.

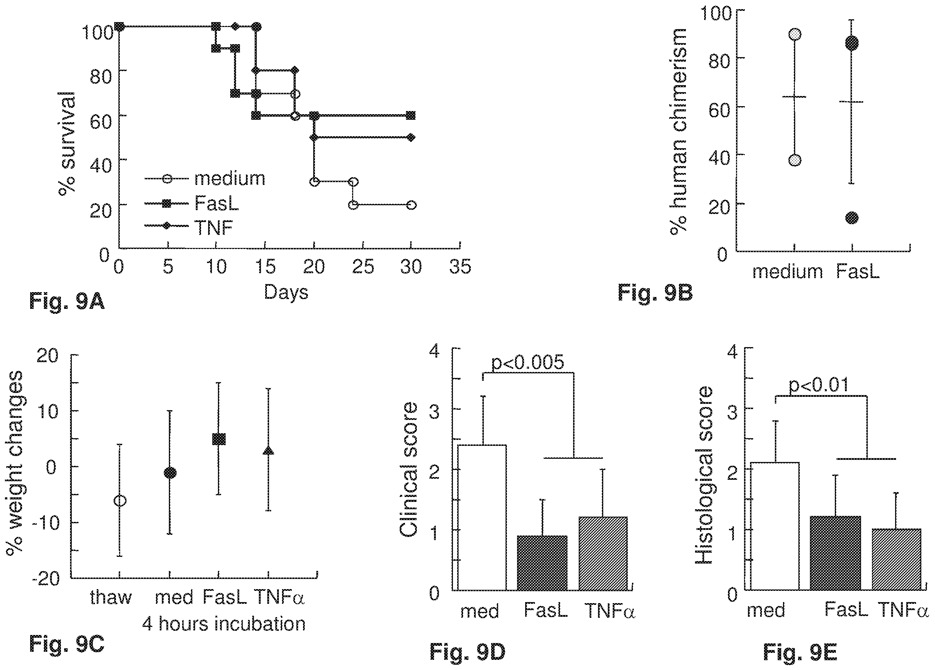

Figure 9: Figures 9A-9E are graphs that relate to an embodiment of the present invention, showing that death ligands prevent GvHD in transplants of cryopreserved mobilized peripheral blood (mPB). (A) Survival of NOD.SCID mice conditioned with two doses of 25 µg/g and grafted after 2 days with $1.5 \times 10^7$ mPB cells incubated in medium with 50 ng/ml FasL and 20 ng/ml TNFα for 4 hours (n=10 in each group). (B) Human xenochimerism was measured in the bone marrow at 12 weeks post-transplantation of cells from the same mPB sample with (n=10) and without (n=7) preincubation with FasL. (C) Body weight at three weeks post- transplantion in recipients of thawed cells (n=18) and after incubation for 4 hours in medium (n=12), with and without FasL (n=9) and TNFα (n=10). At the experimental point of 12 weeks mice grafted with cells incubated in medium and with FasL were assessed for: (D) clinical score according to normal (0) and abnormal (1) parameters: 1. skin disease and hair loss, 2. weakness, 3. footpad hyperkeratosis and 4. diarrhea, and (E) liver histology scored according to: 0-no infiltration, 1-scarce infiltrates, 2-patchy infiltration, 3-diffuse infiltration, 4-deterioration of tissue infrastructure.

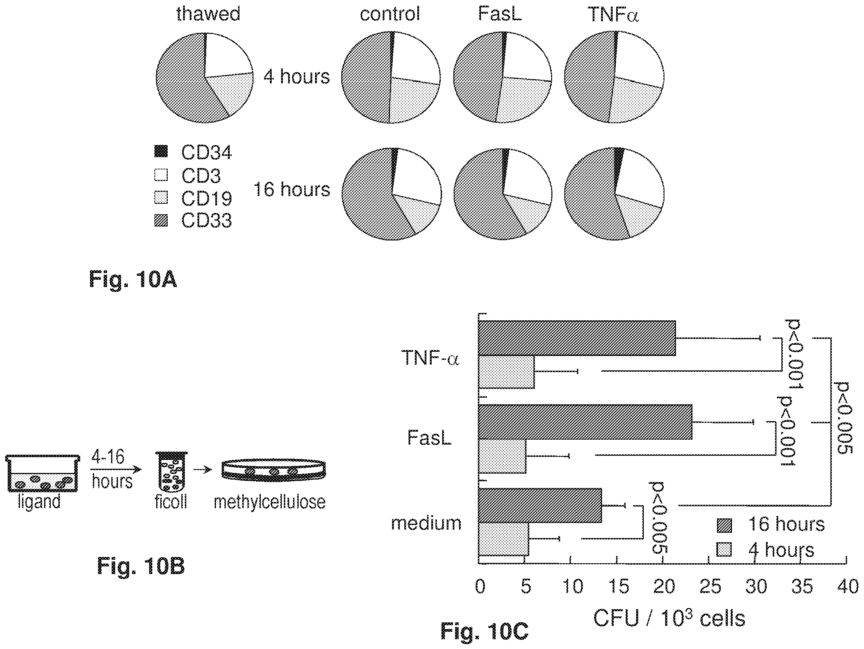

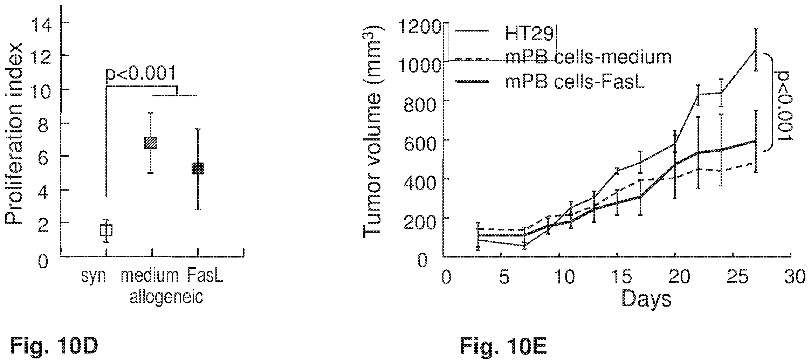

Figure 10: Figures 10B is a scheme and figures 10A, 10C, 10D and 10E are graphs that relate to an embodiment of the present invention, showing that exposure to death ligands does not impair antigenic stimulation and graft versus tumor reactivity. (A) Cryopreserved mPB were thawed and incubated for 4 or 16 hours with 50 ng/g FasL and 20 ng/g TNF-α. Relative distribution of CD34[+] progenitors, T cells (CD3[+]), B lymphocytes (CD19[+]) and myeloid cells (CD33[+]) within the viable cell fraction shows variable sensitivities of these subsets to apoptosis. (B) mPB samples were incubated for 4 or 16 hours with the death ligands, dead cells were eliminated by centrifugation over ficoll and equal numbers of viable cells were plated in semisolid methylcellulose cultures. (C) Clonogenic activity expressed as colony forming cell (CFU) frequency of mPB cells following incubation in medium and with 50 ng/ml FasL or 20 ng/ml TNF-α (n=7-ll in each group). (D) mPB incubated for 4 hours with 50 ng/g FasL were co-incubated with irradiated allogeneic mPB stimulators in mixed lymphocytes reaction (MLR) assays. Proliferation of the responders was determined from CFSE dilution and was quantified using the ModFit software. (E) NOD.SCID mice were inoculated subcutaneously with human colon carcinoma HT29 and infused intravenously with $3x10^7$ mPB cells preincubated in medium and with FasL for 4 hours. Tumor growth rates, as measured with a caliper according to ($mm^3$=length x $width^2$ x 0.4), were decreased by infusion of mPB cells irrespective of exposure to FasL.

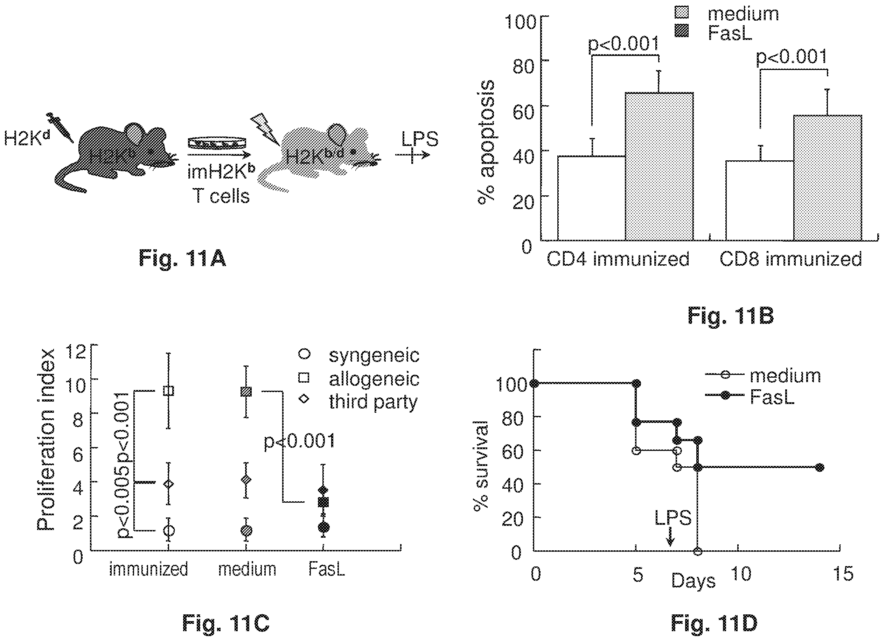

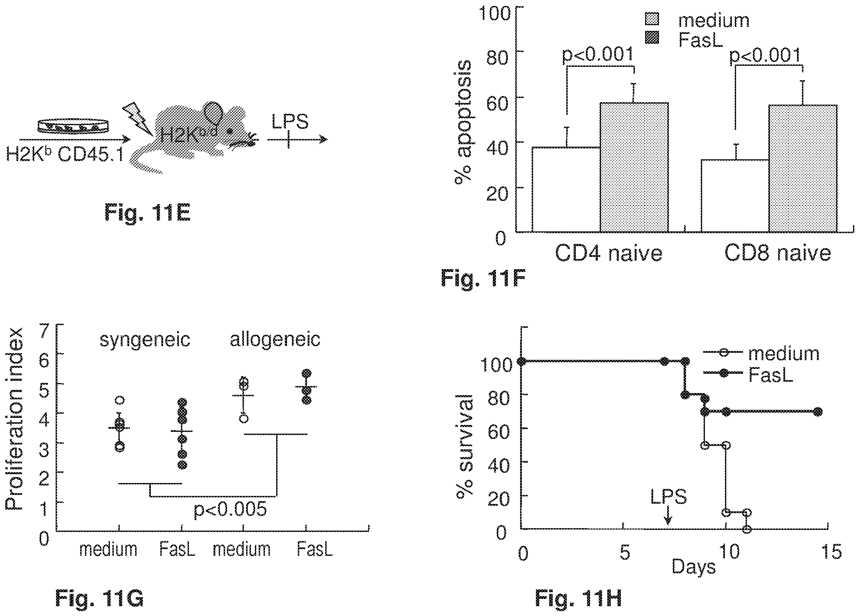

Figure 11: Figures 11A and 11E are schemes and figures 11B-11D and 11F-11H are graphs that relate to an embodiment of the present invention, showing that pretransplant depletion of apoptosis-sensitive cells prevents GvHD. (A-D) FasL mediated elimination of presensitized GvHD effectors. (A) $H2K^b$ mice were immunized twice with $10^7$ $H2K^d$ splenocytes at 3-day intervals. Three days after second immunization splenocytes (im$H2K^b$) were cultured in medium and with 50 ng/ml FasL for 24 hours. (B) Splenocytes harvested from $H2K^d$-immunized $H2K^b$ mice were incubated for 24 hours in medium (n=6) and with 50 ng/ml FasL (n=5). Apoptosis and death were measured from Annexin-V and 7-AAD uptake respectively, in gated CD4[+] and CD8[+] T cell subsets. (C) Splenocytes of immunized mice display increased responses to the irradiated (3000 rad) allogeneic stimulators ($H2K^d$) as compared to third party stimulators ($H2K^k$). Alloresponses are significantly suppressed by incubation with FasL for 24 hours, while sustaining responsiveness to third party stimuli. Proliferation index was determined from CFSE dilution (n=5 individual incubations). (D) Viable splenocytes ($1.5x10^6$) from the immunized donors were adoptively transferred into sublethally irradiated (650 rad) F1 recipients ($H2K^b$→$H2K^{b/d}$) following *ex vivo* incubation in medium and with FasL. Differences in survival were polarized by administration of 10 μg lipopolysacharide (LPS) on day +7 (n=8 in each group). (E-H) FasL-mediated depletion of unstimulated donor splenocytes. (E) Sublethally-irradiated (650 rad) F1 recipients ($H2K^{b/d}$) were infused with semiallogeneic splenocytes ($H2K^b$) preincubated for 24 hours in medium and with FasL. (F) Unstimulated splenocytes were incubated for 24 hours in medium (n-7) and with 50 ng/ml FasL (n-9) for measurement of apoptosis in gated CD4[+] and CD8[+] T cell subsets. (G) Unstimulated splenocytes ($H2K^b$) incubated for 24 hours with and without FasL respond to irradiated allogeneic stimulators ($H2K^d$). (H) Viable splenocytes ($1.5x10^6$) from unstimulated donors were adoptively transferred into sublethally irradiated (650 rad) F1 recipients ($H2K^b$→$H2K^{b/d}$) following *ex vivo* incubation in medium (n=10) and with FasL (n=10). Mice were challenged with 10 μg LPS (intravenous) on day +7.

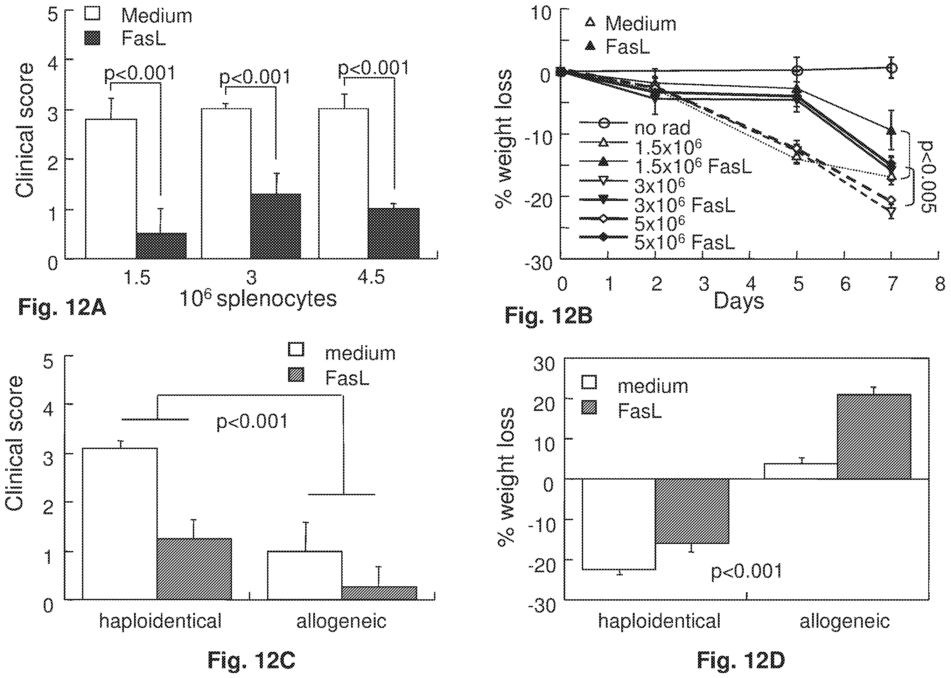

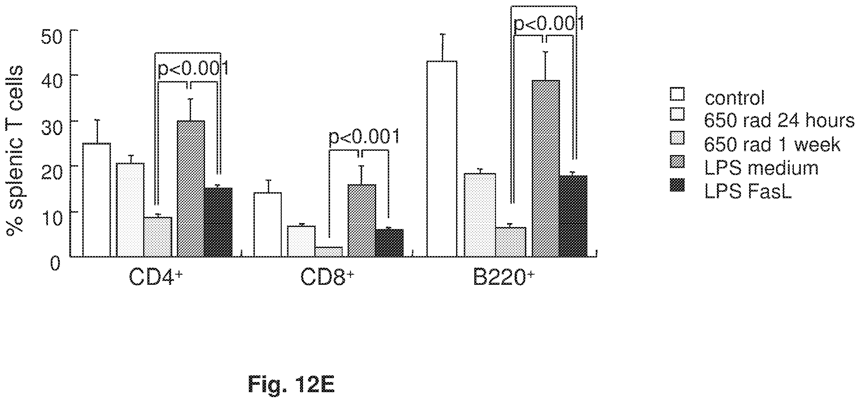

Figure 12: Figures 12A-12E are graphs that relate to an embodiment of the present invention, showing that Fas-mediated depletion of unstimulated splenocytes decreases GvHD activity of haploidentical and allogeneic immune cells. Sublethally-irradiated (650 rad) F1 recipients ($H2K^{b/d}$) were infused with various numbers ($1.5-4.5x10^6$) of semiallogeneic splenocytes preincubated for 24 hours in medium and with FasL. (A) Recipients of splenocytes incubated with and without FasL were scored clinically according to normal (0) and abnormal (1) parameters: 1. skin disease and hair loss, 2. weakness, 3. footpad hyperkeratosis and 4. diarrhea. (B) Weight loss in recipients of various numbers of donor splenocytes incubated with and without FasL (n=5-10 in each group). (C-D) Haploidentical transplants of $3x10^6$ splenocytes were compared to allogeneic transplants ($H2K^b$→$H2K^d$) following incubation with and without FasL for 24 hours (n=6-10 in each group). The clinical score (C) and weight loss (D) were measured after one week. (E) Splenic contents of T and B lymphocytes decreases gradually during the first week after irradiation (n=5 in each group). Survivors of the lipopolysaccharide (LPS) challenge display marked stimulation of these subsets (2 days after LPS infusion), which is blunted by pretreatment of unstimulated splenocytes with FasL (n=7).

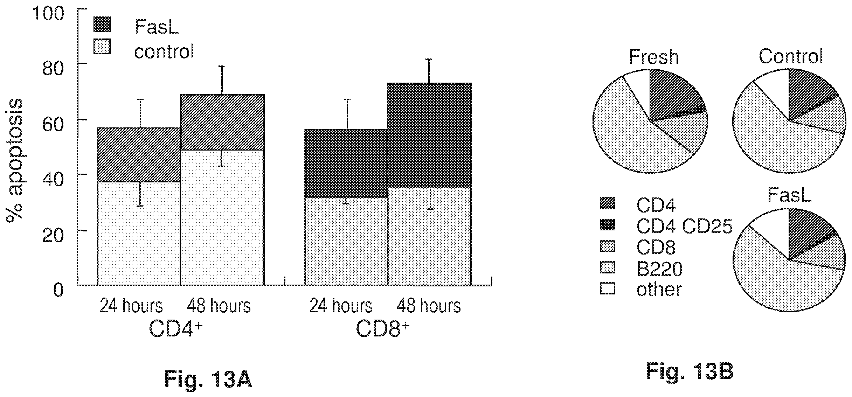

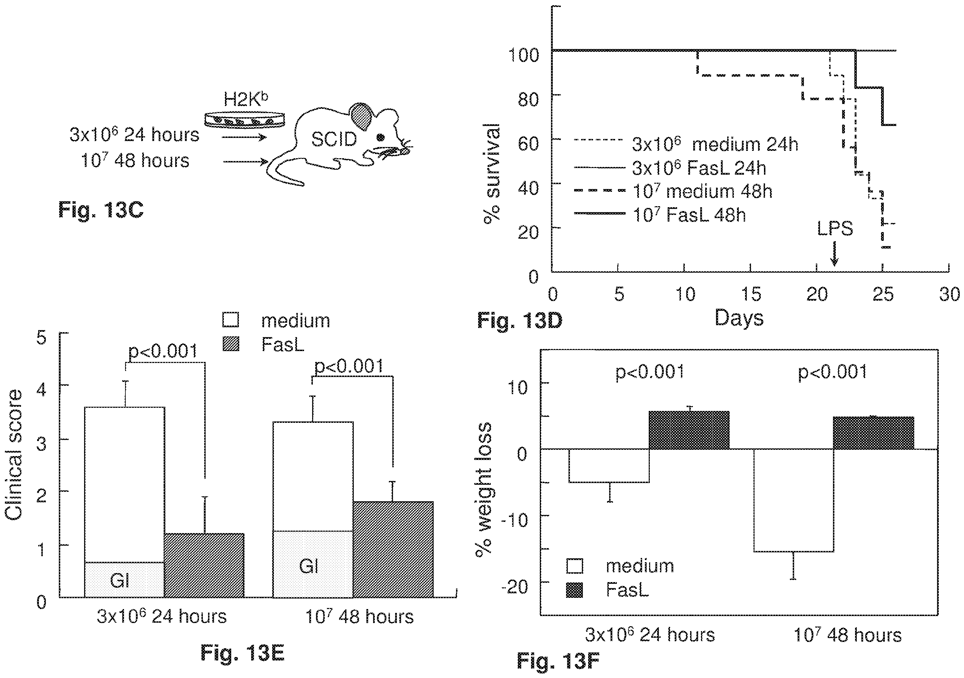

Figure 13: Figure 13C is a scheme and figures 13A, 13B and 13D-F are graphs that relate to an embodiment of the present invention, showing that Fas cross-linking ameliorates GvHD in NOD.SCID mice. (A) Splenocytes incubated in medium and with 50 ng/ml FasL display variable levels of spontaneous (light bars) and Fas-mediated apoptosis (dark bars). Apoptosis was measured by Annexin-V uptake on gated CD4$^+$ and CD8$^+$ T cells subsets after 24 and 48 hours of incubation (n=7-9). (B) Fractional distribution of viable CD4$^+$ and CD8$^+$ T cells, CD4$^+$CD25$^+$ regulatory T cells and B lymphocytes (B220+) before and after 48 hours of incubation in medium and with FasL. (C) Splenocytes from C57BL/6 donors incubated in medium and with 50 ng/ml FasL were infused into allogeneic NOD.SCID mice (H2K$^b$→H2K$^{g7}$): 3x10$^6$ and 10$^7$ naïve splenocytes preincubated for 24 and 48 hours, respectively. (D) Survival of NOD.SCID mice infused with splenocytes preincubated in control medium (n=9) and with 50 ng/ml FasL (n=10). Lethal GvHD was precipitated by intravenous injection of 10 $\mu$g LPS. Mice were evaluated after 3 weeks for clinical score with incidence of gastrointestinal involvement (GI) (E), and weight loss (F).

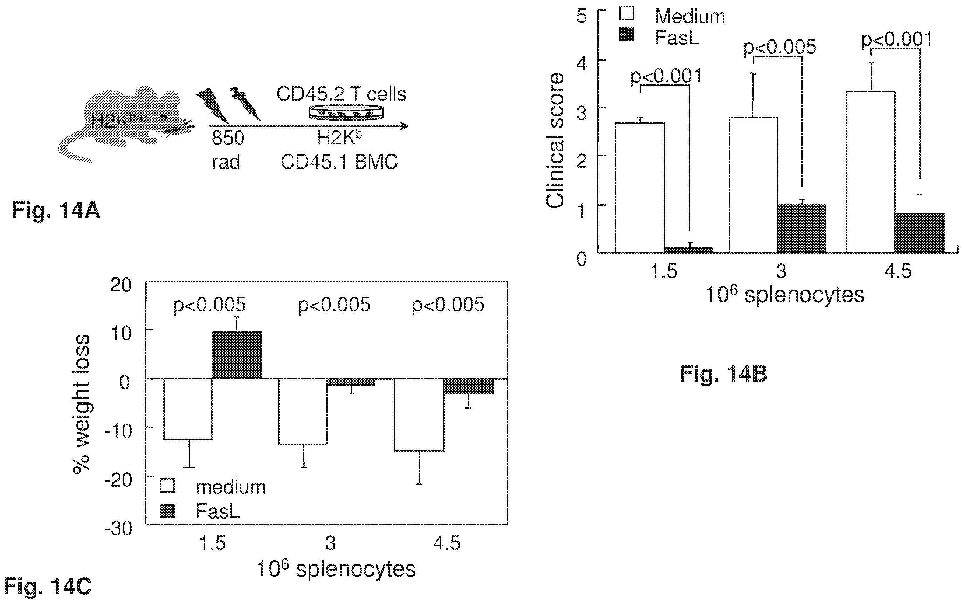

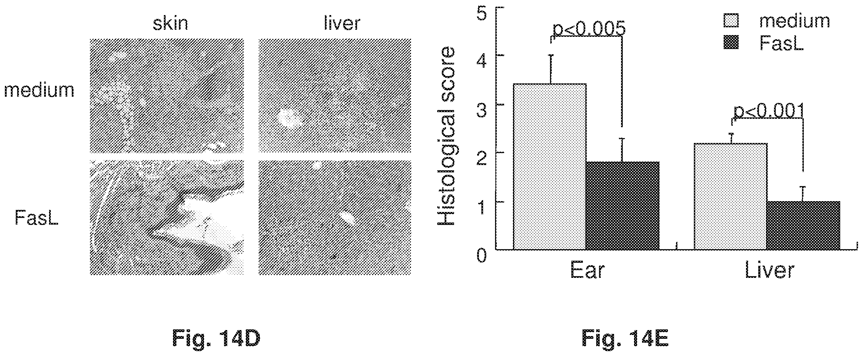

Figure 14: Figure 14A is a scheme, figures 14B, 14C and 14E are graphs and figure 14D is a picture that relate to an embodiment of the present invention, showing elimination of Fas-sensitive naïve immune cells in haploidentical bone marrow transplants. (A) F1 recipients (H2K$^{b/d}$) irradiated at 850 rad were grafted with 5x10$^6$ BMC (H2K$^b$, CD45.1) and various numbers of donor splenocytes (H2K$^b$, CD45.2, GFP). Recipients of splenocytes incubated in medium and with FasL for 48 hours (n=8 in each group) were assessed for clinical score (B) with incidence of gastrointestinal involvement (GI) and weight loss (C) at 3 weeks post-transplantation. (D) At 3 weeks post-transplantation the mice were sacrificed for histological evaluation of skin and liver in recipients of 3x10$^6$ splenocytes incubated in medium and with FasL (n=5). (E) is a graph showing the histological score: 0-no infiltration, 1-scarce infiltrates, 2-patchy infiltration, 3-diffuse infiltration, 4- deterioration of tissue infrastructure.

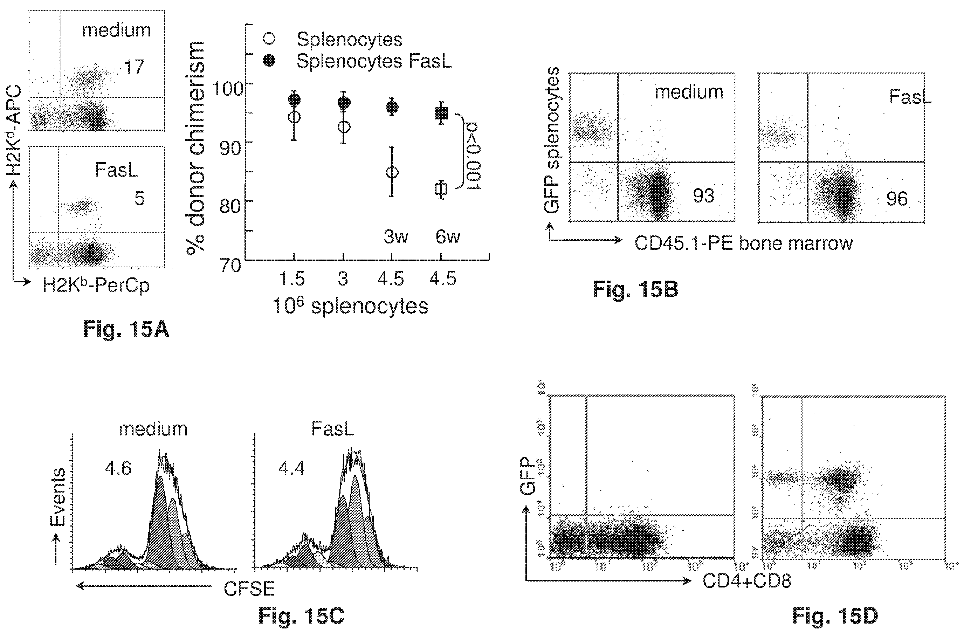

Figure 15: Figures 15A-D relate to embodiments of the present invention: Figure 15A is a graph and FACS scan, figures 15B and 15D are FACS scans and figure 15C is a graph showing immune reconstitution after selective depletion of Fas- sensitive naïve immune cells in haploidentical bone marrow transplants. (A) F1 recipients (H2K$^{b/d}$) irradiated at 850 rad were grafted with 5x10$^6$ BMC (H2K$^b$, CD45.1) and various numbers of donor splenocytes (H2K$^b$, CD45.2, GFP). Donor (H2K$^b$) and host (H2K$^{b/d}$) chimerism was determine in peripheral blood at 3 weeks post-transplantation (n=5). The mice were sacrificed at 3 weeks post-transplantation to determine: (B) The major contribution to spleen reconstitution was from the bone marrow (CD45.1, GFP$^-$) with few of the infused splenocytes being present at this time point (CD45.2, GFP$^+$). Data are representative of 5 independent measurements. (C) Responses to third party (H2K$^k$) irradiated stimulators (at 1:3 responders:stimulators ratio) in MLR assays. Proliferation was determined from CFSE dilution in recipients of unstimulated splenocytes incubated in medium and with FasL (n=4). (D) GFP$^+$ donor splenocytes in mesenteric lymph nodes at 24 hours after transplantation into sublethally irradiated (650 rad) wild type recipients. Data are representative of 4 independent measurements.

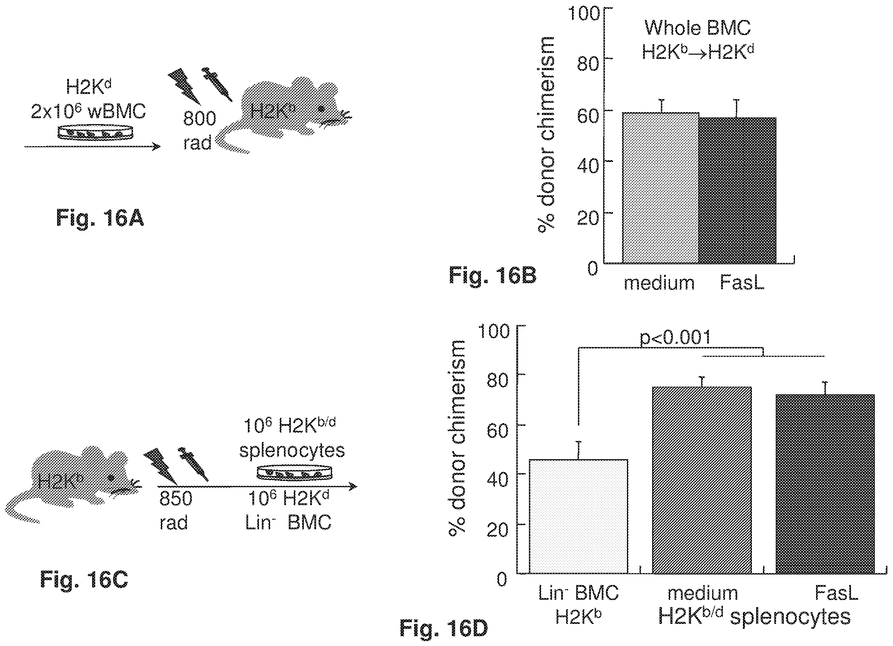

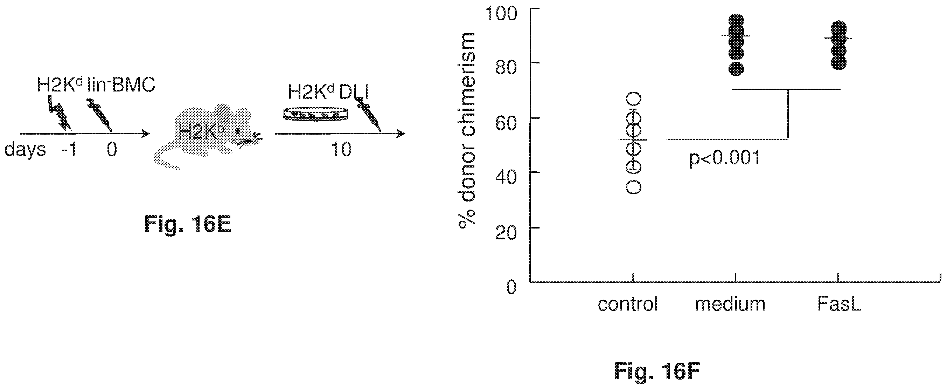

Figure 16: Figures 16A, 16C and 16E are schemes and figures 16B, 16D and 16F are graphs that relate to embodiments of the present invention, showing that FasL-mediated depletion of unstimulated lymphocytes support hematopoietic cell engraftment. (A) Whole bone marrow cells (wBMC) were incubated for 24 hours with and without 50 ng/ml FasL and 2x10$^6$ cells were grafted into sublethally irradiated (800 rad) allogeneic hosts (H2K$^d$→H2K$^b$). (B) Peripheral blood chimerism at 3 weeks was not affected by preincubation with FasL (n=6). (C) Transplantation of 10$^6$ lineage-negative BMC (n=7) into irradiated (800 rad) allogeneic hosts (H2K$^d$→H2K$^b$) was supplemented with 10$^6$ splenocytes from F1 donors (H2K$^{b/d}$) devoid of GvHD activity after preincubation in medium (n=5) and with FasL (n=6) for 24 hours. (D) Addition of splenocytes improved hematopoietic cell engraftment irrespective of exposure to FasL. (E) Mixed chimerism was induced by transplantation of 5x10$^5$ lin BMC into allogeneic recipients (H2K$^d$→H2K$^b$) irradiated at 750 rad (control, n=6). After 10 days the mice were infused with allogeneic (H2K$^d$) 10$^6$ lymphocytes (DLI). (F) The levels of donor chimerism increased 3 weeks after DLI preincubated for 24 hours in medium and with FasL (n=6), as compared to mice that did not receive lymphocyte infusion (control).

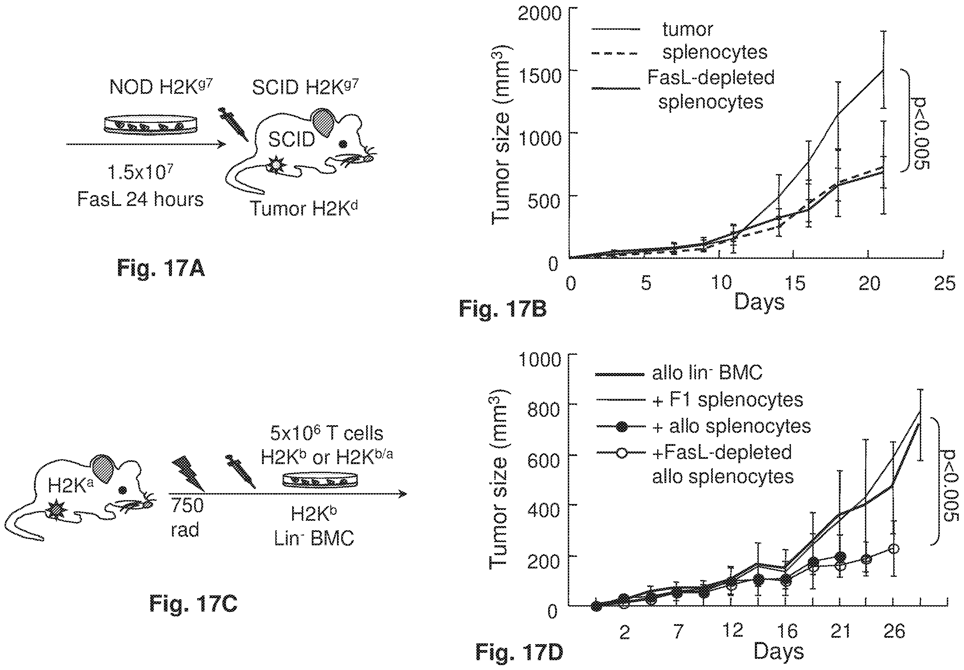

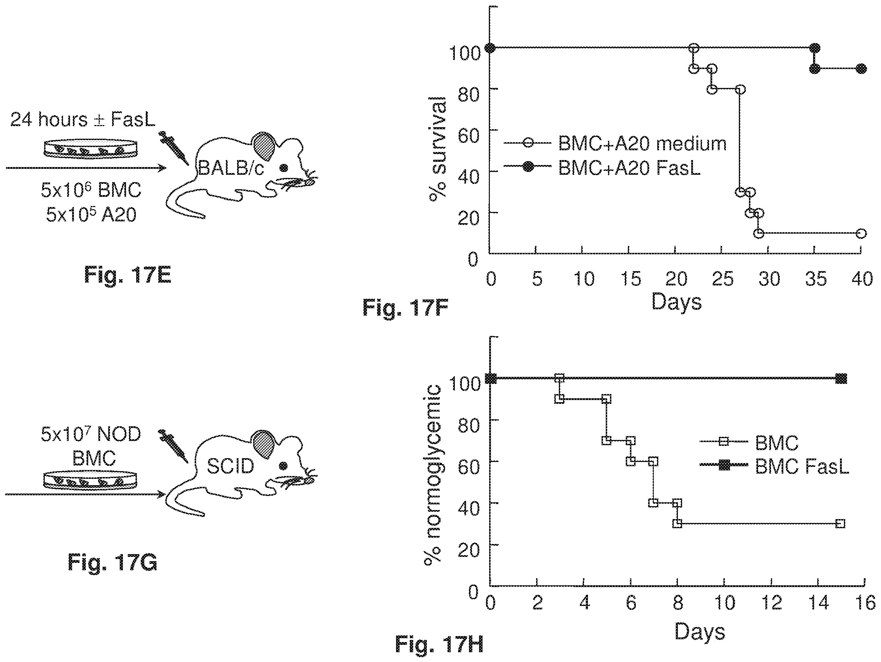

Figure 17: Figures 17A, 17C, 17E and 17G are schemes and figures 17B, 17D, 17F and 17H are graphs that relate to embodiments of the present invention, showing that transplantation of FasL-depleted unstimulated lymphocytes retains graft versus host activity. (A) NOD.SCID mice (H2K$^{g7}$) bearing subcutaneous allogeneic (H2K$^d$) CT26 colon carcinoma tumors were infused (1 day after tumor implantation) with 1.5x10$^7$ splenocytes from 5 weeks old host-matched NOD donors (H2K$^{g7}$). (B) Infusion of lymphocytes suppressed tumor growth in NOD.SCID mice (n=10) irrespective of preincubation for 24 hours in medium and with FasL (n-8). (C) H2K$^a$ mice bearing MHC-matched subcutaneous neuroblastoma (Neuro-2a, H2K$^a$) tumors were sublethally irradiated (750 rad) and grafted with 2x10$^6$ lineage-negative bone marrow cells from allogeneic donors (H2K$^b$). (D) Infusion of lymphocytes from F1 donors (H2K$^{b/d}$), devoid of GVH activity, had no significant impact on tumor growth (n=17). Infusion of allogeneic splenocytes (H2K$^b$) incubated in medium for 24 hours reduced tumor growth rates (n=10), but 80% of the mice died within 3 weeks because of severe GvHD. Infusion of splenocytes preincubated with FasL equally reduced tumor growth but all mice were alive (n=11). (E) Sublethally irradiated (750 rad) BALB/c mice were infused with a mixture of $5 \times 10^6$ syngeneic (H2K$^d$) bone marrow cells and $2 \times 10^5$ A20 Lymphoblastoma cells (H2K$^d$). The cell mixtures were preincubated for 24 hours in medium and with FasL. (F) Recipients of bone marrow and A20 cells incubated in medium developed disseminated tumors with lethal outcome (n=10), whereas recipients of cells preincubated with FasL survived (n=10). (G) Immunocompromized NOD.SCID mice (H2K$^{g7}$) irradiated at 650 rad were adoptively transferred with $5 \times 10^7$ bone marrow cells from syngeneic NOD donors (H2K$^{g7}$) after 48 hours of incubation in medium and with 50 ng/ml FasL protein. Hyperglycemia was considered following two measurements of tasting blood glucose levels exceeding 200 mg/dl. (H) Development of hyperglycemia after adoptive transfer of whole bone marrow cells preincubated in medium and with FasL protein (n=10).

## DETAILED DESCRIPTION OF THE INVENTION

Before explaining at least one embodiment of the invention in detail, it is to be understood that the invention is not limited in its application to the details set forth in the following description or exemplified by the Examples. Also, it is to be understood that the phraseology and terminology employed herein is for the purpose of description and should not be regarded as limiting.

Throughout the specification and claims, the following terms take the meanings explicitly associated herein, unless the context clearly dictates otherwise. The phrases "in one embodiment" and "in some embodiments" as used herein do not necessarily refer to the same embodiment(s), though it may. Furthermore, the phrases "in another embodiment" and "in some other embodiments" as used herein do not necessarily refer to a different embodiment, although it may.

In addition, as used herein, the term "or" is an inclusive "or" operator, and is equivalent to the term "and/or," unless the context clearly dictates otherwise. The term "based on" is not exclusive and allows for being based on additional factors not described, unless the context clearly dictates otherwise. In addition, throughout the specification, the meaning of "a," "an," and "the" include plural references. The meaning of "in" includes "in" and "on."

In a first of its aspects, there is disclosed a selective surface, wherein said selective surface comprises a biocompatible polymer comprising maleic anhydride molecules and an apoptosis inducing ligand bound to the maleic anhydride molecules.

As used herein the term "selective surface" refers to a surface configured to selectively bind and/or affect a cell of interest. Non-limiting examples of surfaces include an inner surface of a container (e.g. a bag, a plate, a multi-well plate, a column, a tube, a bottle, a vial, a drum or a flask), or a removable surface such as a film or a bead (or a plurality of beads).

As used herein, "biocompatible" is a term describing a compatibility of a material, an object, a surface, a device or a polymer with biological entities e.g. cells, biological fluids, such as blood or plasma, and tissues. A biocompatible material may be used for providing surfaces which will contact blood, plasma, cells or tissues in, for example, a container (e.g. a bead, a removable surface, a bag, a plate, a multi-well plate, a column, a tube, a bottle, a vial, a drum or a flask).

As used herein "a biocompatible polymer" is a polymer suitable for contacting a biological entity (e.g. cells). A biocompatible polymer may comprise a hydrophobic polymer coated with at least one layer that promotes the survival of apoptosis resistant cells.

Non limiting examples of a biocompatible polymer include polypropylene, polystyrene, silicone, polyvinyl chloride, polyethylene, polyurethane, ethylene vinyl acetate (EVA), fluorocarbon polymers such as perfluoroalkoxy alkanes (PFA), polytetrafluoroethylene (PTFE), fluorinated ethylene propylene (FEP), EFEP, Polyvinylidene fluoride (PVDF), fluoro modified polypropylene films, Polyether ether ketone (PEEK), High-density polyethylene (HDPE), Low-density polyethylene (LDPE), polysulfone (PSU), Ethylene tetrafluoroethylene (ETFE), polyethylene glycol, polyethylene terephthalate (PET), Poly(2-hydroxyethyl methacrylate), Poly(N-2-hydroxypropyl methacrylamide), dimethacrylate, PEG-dMA, Poly(ethylene glycol) diacrylate (PEG-dA), pHEMA, pHPMA, or any combination thereof.

In the invention the biocompatible polymer further comprises maleic anhydride (MA) molecules. A biocompatible polymer which further comprises maleic anhydride (MA) molecules is also termed herein *a maleic anhydride-copolymer.* Maleic anhydride is an organic compound with the formula $C_2H_2(CO)_2O$. It is the acid anhydride of maleic acid. In one embodiment the biocompatible polymer is coated with MA. In another embodiment MA is incorporated into the biocompatible polymer using any method known in the art, for example as described in Nipithakul T. et al Chinese J. of Polymer Science Vol. 30, No. 2 (2012) or Chausse et al Clin. Chem 36/3, 525-528 (1990).

The biocompatible polymer which comprises maleic anhydride (MA) molecules may further comprise at least one additional compound capable of mediating binding of an apoptosis inducing ligand.

Said additional compound may be selected from the group consisting of an organic chemical, a protein, a protein fragment, a linker, a polynucleotide, or any combination thereof.

The selective surface consists of a biocompatible polymer having maleic anhydride molecules incorporated therein and an apoptosis inducing ligand bound to the maleic anhydride molecules.

As used herein the term *"apoptosis inducing ligand"* refers to a compound with pro-apoptotic activity. As disclosed herein, the apoptosis inducing ligand may be selected from a group consisting of TNFα, FasL, Trail, Tweak, and any combination thereof, The apoptosis inducing ligand may be TNFα or FasL. In a further non-limiting example, the apoptosis inducing ligand maintains its biological activity (e.g. its apoptosis inducing activity) when bound or immobilized onto a surface, such that its apoptotic activity can measure about at least 50% activity compared with a soluble apoptosis inducing ligand. Apoptotic activity can be measured for example by seeding cells that are susceptible to apoptosis (e.g. Jurkat cells as shown in the Examples) onto the selective surface of the invention and measuring the amount of apoptosis, for example by Annexin V staining.

An apoptosis inducing ligand may be FasL and/or TNFα comprising at least an active portion capable of binding the respective receptors and inducing apoptosis in its immobilized form bound to a surface.

The apoptosis inducing ligand may be derived from a mammalian source. An apoptosis inducing ligand may be a rodent (i.e., murine, including but not limited to mouse, rat, etc.) apoptosis inducing ligand. The apoptosis inducing ligand may be a human apoptosis inducing ligand.

A human FasL sequence, a human TNFα sequence, a human Trail sequence and a human Tweak sequence are provided below as SEQ ID NO: 1, SEQ ID NO: 2, SEQ ID NO: 3, and SEQ ID NO: 4, respectively.

| SEQ ID No. | Identification | Sequence |
|---|---|---|
| 1 | Human FasL | MQQPFNYPYPQIYWVDSSASSPWAPPGTVLPCP TSVPRRPGQRRPPPPPPPPPLPPPPPPPPPLPPLPLPP LKKRGNHSTGLCLLVMFFMVLVALVGLGLGMF QLFHLQKELAELRESTSQMHTASSLEKQIGHPSP PPEKKELRKVAHLTGKSNSRSMPLEWEDTYGIV LLSGVKYKKGGLVINETGLYFVYSKVYFRGQSC NNLPLSHKVYMRNSKYPQDLVMMEGKMMSYC TTGQMWARSSYLGAVFNLTSADHLYVNVSELS LVNFEESQTFFGLYK |
| 2 | Human TNF-alpha (α) | MSTESMIRDVELAEEALPKKTGGPQGSRRCLFLS LFSFLIVAGATTLFCLLHFGVIGPQREEFPRDLSLI SPLAQAVRSSSRTPSDKPVAHVVANPQAEGQLQ WLNRKGQGCPSTHVLLTHTISRIAVSYQTKVNL LSAIKSPCQRETPEGAEAKPWYEPIYLGGVFQLE KGDRLSA EINRPDYLDFAESGQVYFGIIAL |
| 3 | Human Trail | MAMMEVQGGPSLGQTCVLIVIFTVLLQSLCVAV TYVYFTNEL KQMQDKYSKSGIACFLKEDDSYWDPNDE ESMNSPCWQVKWQLRQLVRKMILRTSEETISTV QEKQQNIS PLVRERGPQRVAAHITGTRGRSNTLSSPN SKNEKALGRKINSWESSRSGHSFLSNLHLRNGEL VIHEKGF |
| 4 | Human Tweak | MAARRSQRRRGRRGEPGTALLVPLALGLGLALA CLGLLLAV VSLGSRASLSAQEPAQEELVAEEDQDPSE LNPQTEESQDPAPFLNRLVRPRRSAPKGRKTRAR RAIAAHY EVHPRPGQDGAQAGVDGTVSGWEEARINS SSPLRYNRQIGEFIVTRAGLYYLYCQVHFDEGKA VYLKLDLL |

An apoptosis inducing ligand may be a derivative or an analogue of the full protein (i.e., a complete amino acid sequence of a protein). An apoptosis inducing ligand may be a variant, derivative, modified version or truncated version of the full protein.

FasL may be a human FasL such as set forth in SEQ ID NO: 1.

TNFα may be a human TNF α such as set forth in SEQ ID NO: 2. The proteins disclosed herein may be produced by recombinant or chemical synthetic methods.

As used herein, Fas Ligand ("FasL") can refer to a FasL fusion protein, where a FasL protein can be fused with a tag at the N-terminus, the C-terminus, within the FasL protein, or any combination thereof, to construct a FasL fusion protein, such that the activity/binding affinity of FasL fusion protein is substantially the same or higher compared with endogenous (native) FasL. Namely the fusion protein is at least as active as the native molecule. Tags can include His-tags, FLAG-tags, MBP-tags, streptavidin-tags or any other tags typically used when performing protein analysis. Exemplary non-limiting embodiments of tagged FasL are: SuperFasLigand™ (Enzo Life Sciences), Mega FasLigand™ (AdipoGen), APO010 and streptavidin-FasL. Additionally, FasL can refer to a modified FasL protein, such as a truncated FasL protein, where the truncated FasL protein has substantially the same activity/binding affinity as endogenous FasL.

As used herein, TNF α (TNFα) can refer to a TNFα fusion protein, where a TNFα protein can be fused with a tag at the N-terminus, the C-terminus, within the TNFα protein, or any combination thereof, to construct a TNFα fusion protein such that the activity/binding affinity of TNFα fusion protein is substantially the same or higher compared with endogenous TNFα. Namely the fusion protein is at least as active as the native molecule. Tags can include His-tags, FLAG-tags, MBP-tags, His-tags, streptavidin-tags or any other tags typically used when performing protein analysis. An exemplary non-limiting embodiment of tagged TNFα is streptavidin-TNFα. Additionally, TNFα can refer to a modified TNFα protein, such as a truncated TNFα protein, where the truncated TNFα protein has substantially the same activity/binding affinity as endogenous TNFα.

TNF-related apoptosis-inducing ligand (Trail), also known as Apo-2 ligand and TNFSF10, is a type II transmembrane protein with a carboxy-terminal extracellular domain that exhibits homology to other TNF superfamily members. Among TNF superfamily members, Trail is the most homologous to Fas Ligand, sharing 28% amino acid sequence identity in their extracellular domains. Recombinant preparations of Trail arc commercially available from various sources, e.g. R&D Systems.

Tweak is a type II transmembrane protein belonging to the TNF superfamily. A soluble form of Tweak is generated from the membrane-associated molecule by proteolytic cleavage after Arg 93. Tweak is expressed widely in many tissues and cells. Recombinant preparations of Tweak are commercially available from various sources, e.g. R&D Systems or AdipoGen.

An apoptosis inducing ligand may be a small organic molecule.

As used herein the term *"bound to"* or *"immobilized to",* when referring to the interaction of the apoptosis inducing agent with the biocompatible biopolymer comprising the maleic anhydride molecules, encompasses any means of adherence or attachment of the apoptosis inducing agent to the biocompatible biopolymer comprising the maleic anhydride molecules. The binding or immobilization may be by chemical interaction with the maleic anhydride molecules, e.g. the MA molecules interact with primary amines (-NH2) of the apoptosis inducing agent thus forming amide bonds.

The selective surface may be capable of selecting apoptosis-signaling resistant cells.

The term *"selecting"* or *"selection"* as used herein refers to a procedure in which only selected cells out of a heterogeneous cell population survive. The selection may be performed by incubating a heterogeneous population of cells with the selective surface. The procedure may further comprise filtering the population of cells after the incubation to retrieve at least one cell having selected-for characteristics. The surviving cell (i.e. selected-for cell) may be an apoptosis-signaling resistant cell. An apoptosis inducing agent, e.g. FasL and/or TNFα may be used as apoptotic inducing agent for selecting an apoptosis resistant cell.

The terms *"apoptosis-signaling resistant cells". "apoptosis resistant"* or *"receptor-mediated apoptosis resistant"* are used interchangeably herein and relate to cells that do not undergo apoptosis in the presence of an apoptosis inducing agent. In some embodiments, the term refers to cells that do not undergo apoptosis in the presence of an apoptosis inducing agent, under conditions in which other (apoptosis-sensitive) cells do undergo apoptosis. Non-limiting examples of such conditions, e.g. concentrations of the apoptosis inducing agent and incubation times are provided in the Examples section below. The term apoptosis-signaling resistant cells may relate to stem and progenitor apoptosis-resistant cells. Such cells include, for example, umbilical cord blood stem/progenitor cell, mobilized peripheral blood stem/progenitor cell, bone marrow stem/progenitor cell, cancer stem/progenitor cell, hematopoietic stem cells, or neural stem/progenitor cell.

The apoptosis signaling resistant cells may be leukocytes (also known as White blood cells (WBCs)). Leukocytes include neutrophils, eosinophils, basophils, monocytes and lymphocytes. Lymphocytes include B cells, T cells and NK cells.

Leukocytes may be identified on the basis of a surface phenotype, e.g. CD45, and are therefore identified as CD45+ cells.

CD45 (leukocyte common antigen) is a receptor-linked protein tyrosine phosphatase that is expressed on all leucocytes, and which plays a crucial role in the function of these cells.

The apoptosis signaling resistant cells may be identified on the basis of a surface phenotype, e.g. CD34+ cells.

The CD34 protein is a member of a family of single-pass transmembrane sialomucin proteins that show expression on early hematopoietic and vascular-associated tissue. Cells expressing CD34 (CD34$^+$ cell) are found in various tissues, for example in the umbilical cord and bone marrow as hematopoietic cells, a subset of mesenchymal stem cells, or endothelial progenitor cells.

The selected stem and progenitor apoptosis-resistant cell can be an immune cell insensitive to activation-induced cell death (AICD).

The immune cell insensitive to activation-induced cell death may be a T cell. The immune cell insensitive to AICD may be a non-activated T cell.

The selective surface may be in the form of an inner surface of a container, a plate, a film, a disc, a bead, or a plurality of beads.

When a selective surface is a surface of a bead of a plurality of beads, each bead of the plurality of beads can be uniformly composed, where a uniform composition refers to a surface material of each bead having the substantially the same chemical and/or physical composition as a core material of the bead.

When a selective surface is a surface of a bead of a plurality of beads, each bead of a plurality of beads can be non-unifornily composed, where a non-uniform composition refers to a surface material of each bead having a substantially different chemical and/or physical composition as a core material of the bead.

The selective surface of the instant composition may include MA-bound beads, plates, or containers configured to bind a protein, e.g. an apoptosis inducing ligand, e.g., FasL, and where about ~1 ng -~4000 ng of FasL can be bound to the MA-coated beads, plates, containers, or any combination thereof. In a non-limiting example, standard tissue culture 96-well plates can be used as a selective surface bound to maleic anhydride; accordingly, the following proportions can be used to calculate the amount/concentration of, e.g., FasL and/or TNFα for binding to other selective surfaces of containers and/or surfaces of beads. MA-bound beads may be configured to bind a protein, e.g. an apoptosis inducing ligand, e.g., FasL, where about ~4000 ng of FasL can be bound to the MA-coated beads. A protein, e.g., FasL, can be bound to a surface at a concentration calculated by using an equivalent measurement of another protein, e.g. biotin, using for example, but not limited to, an equivalent amount of FasL in mass (grams) units to 125 picomoles of biotin.

The resulting MA-coated FasL bound wells can measure about 50% activity compared to 50ng/ml soluble FasL (i.e., 10 ng per well in a plate).

The resulting MA-coated FasL bound wells can measure between 30 - 70% activity compared to 50ng/ml soluble FasL.

The resulting MA-coated FasL bound wells can measure between 40 - 70% activity compared to 50ng/ml soluble FasL. The resulting MA-coated FasL bound wells can measure between 50 - 70% activity compared to 50ng/ml soluble FasL. The resulting MA-coated FasL bound wells can measure between 60 - 70% activity compared to 50ng/ml soluble FasL. The resulting MA-coated FasL bound wells can measure between 30 - 60% activity compared to 50ng/ml soluble FasL.

The resulting MA-coated FasL bound wells can measure between 30 - 50% activity compared to 50ng/ml soluble FasL.

The resulting MA-coated FasL bound wells can measure between 30 - 40% activity compared to 50ng/ml soluble FasL.

The selective surface may be an amine immobilizer plate (e.g., Nunc amine immobilizer plate) composed of maleic anhydride bound to a protein, e.g., FasL (and/or TNFα), where FasL protein measures >30kDa, and where about ~4000 ng of FasL can be bound to the amine immobilizer plate. While this exemplary embodiment illustrates FasL bound to the selective surface composed of maleic anhydride, using bound TNFα in comparable concentrations is also disclosed. Both FasL and TNFα may be bound to immobilizer plates such that the total concentrations of FasL and TNFα equal the concentrations and activity noted herein.

Between 1 ng and 4000 ng of FasL can be bound to the amine immobilizer plates. Between 50 ng and 4000 ng of FasL can be bound to the amine immobilizer plates. Between 500 ng and 4000 ng of FasL can be bound to the amine immobilizer plates.

Between 1000 ng and 4000 ng of FasL can be bound to the amine immobilizer plates. Between 2000 ng and 4000 ng of FasL can be bound to the amine immobilizer plates. Between 3000 ng and 4000ng of FasL can be bound to the amine immobilizer plates. Between 1ng and 3000ng of FasL may be bound to the amine immobilizer plates. Between 1ng and 2000ng of FasL may be bound to the amine immobilizer plates. Between 1ng and 1000ng of FasL may be bound to the amine immobilizer plates. Between 1ng and 500ng of FasL may be bound to the amine immobilizer plates. Between 1ng and 50ng of FasL may be bound to the amine immobilizer plates.

The selective surface of the instant composition may include maleic anhydride (MA)-bound beads, plates, or containers configured to bind at least one protein, e.g., FasL, TNFα, Trail, Tweak, or any combination thereof, where about 1ng - 100ng of FasL, TNFα, Trail, Tweak, or any combination thereof, can be bound to the MA-coated beads, plates, containers, or any combination thereof. As disclosed herein, the at least one protein, i.e., FasL, TNFα, Trail, Tweak, or any combination thereof, can be bound to the MA-coated beads, plates, containers, or any combination thereof at a concentration of 1-10ng. The at least one protein, i.e., FasL, TNFα, Trail, Tweak, or any combination thereof, can be bound to the MA-coatcd beads, plates, containers, or any combination thereof at a concentration of 1-25ng.

The at least one protein, i.e., FasL, TNFα, Trail, Tweak, or any combination thereof, can be bound to the MA-coated beads, plates, containers, or any combination thereof at a concentration of 1-50ng. The at least one protein. i.e., FasL, TNFα, Trail. Tweak, or any combination thereof, can be bound to the MA-coated beads, plates, containers, or any combination thereof at a concentration of 1-75ng. The at least one protein, i.e.. FasL, TNFα, Trail, Tweak, or any combination thereof, can be bound to the MA-coated beads, plates, containers, or any combination thereof at a concentration of 10-100ng. The at least one protein, i.e., FasL, TNFα, Trail, Tweak, or any combination thereof, can be bound to the MA-coated beads, plates, containers, or any combination thereof at a concentration of 25-100ng.

The at least one protein, i.e., FasL, TNFα, Trail, Tweak, or any combination thereof, can be bound to the MA-coated beads, plates, containers, or any combination thereof at a concentration of 50-100ng. The at least one protein, i.e., FasL, TNFα, Trail, Tweak, or any combination thereof, can be bound to the MA-coated beads, plates, containers, or any combination thereof at a concentration of 75-100ng.

The density of linkers (e.g. the density of maleic anhydride molecules) may be 1.7-3.4 residues/square nm. The density of linkers may be 2.0-3.4 residues/square nm. The density of linkers may be 2.5-3.4 residues/square nm. The density of linkers may be 3.0-3.4 residues/square nm. The density of linkers may be 1.7-3.0 residues/square nm. The density of linkers may be 1.7-2.5 residues/square nm. The density of linkers may be 1.7-2.0 residues/square nm. The density of linkers may be 2.0-3.0 residues/square nm. Micro-carriers may be used. Micro-carriers may not be used.

As disclosed herein, the composition may include a selective surface, where the selective surface can be selected from the following group: agarose/magnetic/polystyrene/sepharose/silica beads bound to N-hydroxysuccinimide (NHS) (e.g., NHS-activated agarose), aldehyde-activated agarose, azlactone-activated support, carbonyl diimidzaole agarose/Trisacryl, carboxyl groups, cyanogen bromide, polyethylene glycol (PEG), maleic anhydride, or any combination thereof. The composition may include a plurality of selective surfaces that can he selected from the following group: agarose/magnetic beads bound to N-hydroxysuccinimide (NHS) (e.g., NHS-activated agarose), Nunc amine immobilizer (Nune®), aldehyde-activated agarose, azlactone-activated support, carbonyl diimidzaole agarose/Trisacryl, carbonyl diimidazole, carboxyl groups, azlactone, or any combination thereof.

More specific examples of the composition include selective surfaces, where the selective surfaces are the surfaces of a plurality of beads selected from the group consisting of agarose/magnetic beads bound to NHS (e.g., but not limited to, NHS-activated agarose, Thermo Scientific Catalog No. 26196, 26197, 26198, 26199); aldehyde-activated agarose (e.g., but not limited to, amino link resin, Thermo Scientific Catalog No. 20381, 20382), azlactone-activated support (e.g., but not limited to, Ultra link bio-support, Thermo Scientific Catalog No. 53110, 53111), carbonyl diimidazole agarose/Trisacryl, or any combination thereof.

Examples of the composition include a selective surface, where the selective surface has a regular bead shape(s) (e.g., spherical shapes or spherical-like shape). Examples of the selective surface of the composition of the present invention include geometrical shapes, including bead (and any polygonal shape and ellipsoid shape), fiber, and film shapes.

The composition may include a protein ligand bound to a surface, where the protein ligand is FasL, and where the surface can be a plurality of beads, where the plurality of beads comprise a biocompatible material. Each of the plurality of beads may measure between 20 μ - 350 μ (as used herein μ=micron or micrometer). Each of the plurality of beads may measure between 20μ-300μ. Each of the plurality of beads may measure between 20μ-250μ. Each of the plurality of beads may measure between 20μ-200μ. Each of the plurality of beads may measure between 20μ -150μ. Each of the plurality of beads may measure between 20μ-100μ. Each of the plurality of beads may measure between 20μ-300μ. Each of the plurality of beads may measure between 50μ-350μ. Each of the plurality of beads may measure between 100μ-350μ. Each of the plurality of beads may measure between 150μ-350μ. Each of the plurality of beads may measure between 200μ-350μ. Each of the plurality of beads may measure between 250 μ - 350 μ. Each of the plurality of beads may measure between 300 μ - 350 μ. Each of the plurality of beads may measure between 20 μ - 50 μ. Each of the plurality of beads may measure between 20 μ - 90 μ. Each of the plurality of beads may measure between 50 μ - 150 μ. Each of the plurality of beads may measure between 250 μ - 350 μ.

Each of the plurality of beads may measure between 1 - 5 $\mu^3$. Each of the plurality of beads may measure between 0.1 - 10 $\mu^3$. Each of the plurality of beads may measure between 1 - 10 $\mu^3$. Each of the plurality of beads may measure between 2.5 - 10 $\mu^3$. Each of the plurality of beads may measure between 5 - 10 $\mu^3$. Each of the plurality of beads may measure between 7.5 - 10 $\mu^3$. Each of the plurality of beads may measure between 0.1 - 7.5 $\mu^3$. Each of the plurality of beads may measure between 0.1 - 5 $\mu^3$. Each of the plurality of beads may measure between 0.1 - 2.5 $\mu^3$. Each of the plurality of beads may measure between 0.1 - 1 $\mu^3$. Each of the plurality of beads may measure between 1 - 3 $\mu^3$. Each of the plurality of beads may measure between 3 - 5 $\mu^3$.

The composition may include surfaces of a plurality of beads, where the surfaces of the plurality of beads are configured to increase binding of cellular ligands, resulting in increased apoptosis compared with a flat surface bound to comparable peptides/proteins.

The composition may include surfaces of a plurality of beads, where the surfaces of the plurality of beads can be housed in a column. The composition may include the surfaces of a plurality of beads, where the beads are in slurry form. The composition may include surfaces of a plurality of beads, where the beads are in a packed form.

The method can include the packing a plurality of beads in a column, where the packing is achieved by a typical flow packing technique.

The selective surface may be kept dry. The selective surface may be placed in a solution for maintaining the integrity and activity of the apoptosis-inducing ligand, e.g. buffer or media.

Apoptosis-signaling resistant cell may be resistant to FasL.

There is disclosed a device having a selective surface wherein the device can be used in the selection of stem and progenitor cells. The device may be a container made of or coated with a biocompatible material further comprising a selective surface, namely a surface comprising a biocompatible polymer comprising maleic anhydride and a biologically active apoptosis-inducing ligand immobilized thereto. The biologically active apoptosis-inducing ligand may be immobilized to (a) an inner selective surface of the container and/or (b) selective surfaces of a plurality of beads within the container. The container may be selected from the group consisting of a bag, a plate, a multi-well plate, a column, a tube, a bottle, a vial, a drum and a flask.

The device may comprise a biocompatible material, where the biocompatible material includes, but is not limited to: polypropylene, polystyrene, silicone, polyvinyl chloride, polyethylene, polyurethane, ethylene vinyl acetate (EVA), fluoro carbons polymers such as perfluoroalkoxy alkanes (PFA), polytetrafluoroethylene (PTFE), fluorinated ethylene propylene (FEP), EFEP, Polyvinylidene fluoride (PVDF), fluoro modified polypropylene films, Polyether ether ketone (PEEK), High-density polyethylene (HDPE), Low-density polyethylene (LDPE), polysulfone (PSU), Ethylene tetrafluoroethylene (ETFE), polyethylene glycol, polyethylene terephthalate (PET), Poly(2-hydroxyethyl methacrylate), Poly(N-2-hydroxypropyl methacrylamide), dimethacrylate, PEG-dMA, Poly(ethylene glycol) diacrylate (PEG-dA), pHEMA, pHPMA, magnetic/paramagnetic beads, dextran beads having an iron oxide/hydroxide core (iron-dextran complex, about 50-100 nm in diameter) or any combination thereof. The beads may include: agarose/magnetic/polystyrene/sepharose/silica beads bound to N-hydroxysuccinimide (NHS) (e.g.. NHS-activated agarose), aldehyde-activated agarose, azlactone-activated support, carbonyl diimidzaole agarose/Trisacryl. carboxyl groups, cyanogen bromide, polyethylene glycol (PEG). The immobilized apoptosis-inducing ligand is Fas ligand (FasL). It is disclosed that the immobilized apoptosis-inducing ligand is TNFα (TNFα).

When a selective surface is a surface of a bead or a plurality of beads, each bead of the plurality of beads can be uniformly composed, where a uniform composition refers to a surface material of each bead having substantially the same chemical and/or physical composition as a core material of the bead. When a selective surface is a surface of a bead of a plurality of beads, each bead of a plurality of beads can be non-uniformly composed, where a non-uniform composition refers to a surface material of each bead having a substantially different chemical and/or physical composition as a core material of the bead.

The device can utilize and/or relate to a container, wherein the container having an inner selective surface and/or selective surfaces of a plurality of beads, where the selective surface(s) is/are composed of, e.g., a biopolymer with maleic anhydride bound to an apoptosis inducing ligand. The device can provide a selective surface for binding stem cells. The device can use a container, with an apoptosis-inducing ligand immobilized to the surface of beads introduced therein. The device is utilized for selecting stem/progenitor cells using a single step. Single step includes the incubation of a heterogeneous cell population within the container. Incubation exposes the cell population to apoptotic ligands (e.g., FasL and/or TNFα) as provided herein. Incubation results in survival of apoptotic-resistant cells such as stem-cells and apoptotic death of apoptosis-sensitive cell types.

Incubating a population of cells with the selective surface composed of, e.g., maleic anhydride, bound to FasL and/or TNF α results in binding specific cells expressing proteins that interact/bind with FasL and/or TNF α.

A selected population of apoptosis-signaling resistant cells do not bind to the selective surface.

As disclosed herein, a selected population of apoptotic-resistant cells (such as stem and progenitor cells) may be used for transplanting in the course of treating a disease, such as, but not limited to: cancer, immune diseases, chemotherapy-resistant cancer, or congenital or acquired immunodeficiency. In another embodiment, one of skill in the art can readily determine the use of apoptosis resistant cells of the invention.

The container may be constructed of at least one biocompatible material. A biocompatible material may include but not limited to: polypropylene, polystyrene, silicone, polyvinyl chloride or a combination thereof. In another embodiment, a biocompatible material is selected according to parameters such as, but not limited to: durability or ability to facilitate immobilization of apoptosis- inducing ligands on its inner surface or transparency.

A biocompatible material may be inert with respect to eliciting any undesirable local or systemic effects in the recipient or beneficiary of that therapy, but generating the most appropriate beneficial cellular or tissue response in that specific situation, and optimizing the clinically relevant performance of that therapy.

Container can be used in the selection of a population of stem and progenitor cells, and is a bag that is configured to house a plurality of beads having a plurality of surfaces having selective surfaces composed of, e.g., but not limited to, maleic anhydride bound to FasL and/or TNFα. In another embodiment, the container is a column, where a column can be packed with beads according to typical packing parameters, including adding a slurry of a plurality of beads to the column and flow packing the column. In another embodiment, the container is a tube. The container may be a bottle. In another embodiment, the container is a vial. The container may be a flask. The container may be a drum. The container may be any other receptacle which suits the specific intended selective use of the device.

The device that can be used in a selective method is comprised of a single container into which a cell-population is introduced. The container may comprise two interlocking chambers separated by a filter apparatus adapted to separate whole cells from cell debris and proteins. A cell-population may be introduced into one chamber, incubated within the chamber and after incubation the filter may be used to further isolate the selected cells from cell debris and proteins in the solution.

As disclosed herein, the container that can be used in a selective method is in the form of a column in which the cell population is incubated, allowing attachment of whole cells or specifically of stem and/or progenitor cells. to the column. Following incubation, the remaining cell debris and proteins may be discarded and the selected cells are isolated from the column.

The method of preparing a selective surface or a plurality of selective surfaces includes: (i) dissolving an apoptosis inducing ligand in a solution, medium or buffer, e.g. an immobilization buffer, (ii) adding the dissolved apoptosis inducing ligand to a surface composed of, or comprising, a maleic anhydride-copolymer as defined above, (iii) incubating the surface with the dissolved apoptosis inducing ligand, (iv) optionally, removing the unbound dissolved apoptosis inducing ligand from the surface, (v) optionally, adding a protein blocking buffer to the surface, (vi) optionally, incubating the surface with the protein blocking buffer, (vii) optionally, washing the surface. The method may further include contacting and/or incubating a population of cells with the selective surface or a plurality of selective surfaces so as to result in (a) selective binding of cells expressing apoptosis inducing ligand binding proteins and (b) an unbound (i.e., free) cell population, where the cell population can include stem and progenitor cells.

The biologically active apoptosis-inducing ligand(s) (e.g., FasL, and/or TNFα) may be immobilized/bound to a selective surface composed of, e.g., maleic anhydride, where the selective surface can be the surfaces of a plurality of beads and/or a flat surface (e.g., a multi-well plate).

The biologically active apoptosis-inducing ligand(s) (e.g., FasL and/or TNFα) may be immobilized to beads within the container. The biologically active apoptosis-inducing ligands may be immobilized in a manner that allows free interaction of the ligands with cells contained within the container.

As disclosed herein, the selective method can use immobilization methods that involve modification or coating of the surface with appropriate substances to change the surface property or provide functional groups for the binding of protein. Immobilization of proteins on a bare surface with no modification necessitates using an affinity peptide that is specific to the particular surface. Immobilization may be achieved by utilizing a chelator compatible with the apoptosis inducing ligand of the invention. A chelator may be both compatible with the apoptosis inducing ligand of the invention and maintains its biological activity (e.g., but not limited to, at least 25% biological activity, at least 50% biological activity, at least 75% biological activity, at least 90% biological activity, at least 99% biological activity).

A chelator may be (1) compatible with the apoptosis inducing ligand of the invention and (2) promotes biological activity of the apoptosis inducing ligand of the invention.

Immobilization of an apoptosis-inducing ligand may be achieved by: physical adsorption, interaction between His-6 and $Ni^+$ ions, coiled coil association of a heterodimeric Leu zipper pair. Chemisorption of SH-groups. Schiffs base linkage between aldehyde and amino groups, acyl transfer reaction of TGase. affinity between streptavidin-biotin, affinity between FLAG and anti-FLAG antibody, affinity between glutathione and GST or binding of a protein fused with a PS-affinity peptide to hydrophilic polystyrene, or any combination thereof.

For example, a method for immobilizing/stabilizing a protein and/or peptide on tissue culture-treated plastic surfaces can include:

(a) coating a tissue culture-treated plastic surface with a coating solution, where the coating solution comprises a protein or polypeptide to be immobilized in a concentration of about 1 ng/mL to about 2000 ng/mL, in a coating buffer, wherein the coating buffer has a buffer species dissolved in distilled and/or deionized water in a concentration of about 2 mM to about 500 mM and in one embodiment the pH of the coating buffer is from about 2 to about 6, in another embodiment the pH of the coating buffer is from about 6 to about 8, and the pH may also be from about 8 to about 13;
(b) incubating the coaling solution on the tissue culture-treated plastic surface;
(c) washing the plastic surface to remove any extraneous materials not firmly attached to the plastic surface;
(d) drying the plastic surface having the coating solution thereon;
(e) obtaining a tissue culture-treated plastic surface having a protein efficiently and stably bound thereto, or
(f) any combination thereof.

For example, a method for immobilizing/stabilizing a protein and/or peptide on tissue culture-treated plastic surfaces can include:

(a) activating a group (e.g., but not limited to, carboxyl group) included in a solid substrate;
(b) surface-treating the solid substrate having the activated group by adhering the peptide hybrid for protein immobilization of the present invention onto the surface;
(c) binding a protein onto the surface-treated solid substrate of step 2), and a substrate for protein immobilization surface-treated with the peptide hybrid for protein immobilization of the invention prepared by the same;
(d) or any combination thereof.

For example, a method of immobilizing a protein onto a support comprising in an expression system can be utilized, expressing a fusion protein comprising a cleavable intein and reacting the fusion protein with a ligand capable of cleaving the intein to form a protein-ligand; and contacting the products of the expression system with a support that is functionalized with an affinity receptor, thereby immobilizing the protein-ligand onto the support. The method can increase the efficiency of intein-mediated covalent attachment of a ligand to the C-terminus of a protein comprising expressing a fusion protein comprising a cleavable intein, wherein the fusion protein comprises at least one small side-chain amino acid immediately upstream to the N-terminus of the intein. There is also disclosed a method of immobilizing a protein onto a support comprising in a cell-free expression system, expressing a protein and covalently attaching, e.g., but not limited to, a puromycin-ligand at the C-terminus of the protein; and contacting the products of the cell-free expression system with a support that is functionalized with an affinity receptor, thereby immobilizing the protein onto the support.

There is also provided herein a method for selecting apoptosis-resistant cells out of a heterogeneous cell population, wherein the heterogeneous population comprises apoptosis-signaling resistant cells and apoptosis-signaling sensitive cells. The method may consist of introducing the heterogeneous cell population into a device and incubation therein.

The method may comprise introducing a sample comprising the heterogeneous cell population into the device of the present invention and incubation therein. An apoptosis-signaling resistant cell may be a stem/progenitor cell.

The device may include a biologically active apoptosis-inducing ligand (e.g. FasL and/or TNF$\alpha$) immobilized to (a) an inner selective surface of a container and/or (b) selective surfaces of a plurality of beads within the container, resulting in selecting stem and progenitor apoptosis-signaling resistant cells from the cell population. The method may include enriching stem and progenitor apoptosis-signaling resistant cells from a first cell population. The selected stem and progenitor apoptosis-resistant cell may be a stem/progenitor cell, selected from a group comprising: umbilical cord blood stem/progenitor cell, mobilized peripheral blood stem/progenitor cell, bone marrow stem/progenitor cell, hematopoietic stem cells, cancer stem/progenitor cell, and neural stem/progenitor cell.

As disclosed herein, the selected stem and progenitor apoptosis-resistant cell can be an immune cell insensitive to activation-induced cell death (AICD). AICD, as used herein refers to cell of the immune system that does not undergo apoptosis upon activation. The immune cell insensitive to activation-induced cell death (AICD) may be a T cell. An immune cell insensitive to AICD may be a non-activated T cell. The selected apoptosis-resistant cell may be a stem/progenitor cell. The cell population used in the method may be derived from: bone marrow. a stem/progenitor cell mobilized peripheral blood, or umbilical cord blood (UCB). A stem/progenitor cell may be an umbilical cord blood stem/progenitor cell.A stem/progenitor cell may be a mobilized peripheral blood stem/progenitor cell. A stem/progenitor cell may be bone marrow stem/progenitor cell. stem/progenitor cell may be a hematopietic stem/progenitor cell. A stem/progenitor cell may be a cancer stem/progenitor cell. A stem/progenitor cell may be a neural stem/progenitor cell. A stem/progenitor cell may be a cord blood stem/progenitor cell, a mobilized peripheral blood stem/progeniror cell, a bone marrow stem/progenitor cell, a hematopoietic stem cell, a cancer stem/progenitor cell, a neural stem/progenitor cell or a combination thereof.

Harvesting of stem-cells for therapeutic purposes requires extraction of a tissue which contains stem-cells, either autologous or from a donor, and then isolation of the stem-cells from other cell populations that may have deleterious effects if co-transplanted along with stem-cells.

The phrase "apoptosis resistant" may be "receptor-mediated apoptosis resistant". The cell-selection method as disclosed herein may be a single-step negative selection method for cell types that are resistant to receptor-mediated apoptosis. The present method enables parallel isolation of both stem cells and immune-cells that support the clinical outcome of transplantation, via a single use of the device, as is exemplified below.

The cell populations which may be used in the method as disclosed herein may be stem/progenitor cells derived from, but are not limited to, bone marrow, umbilical cord blood (UCB) and progenitor cell mobilized peripheral blood (mPB). The cell population can be derived from adult tissues (e.g., but not limited to: fat, heart, brain) which contain apoptosis-resistant cells. The cell population may derive from embryonic tissue.

A heterogeneous cell population may be derived from an organ or a tissue. It is disclosed herein that a heterogeneous cell population may be derived from an embryo. A heterogeneous cell population may be derived from a tissue comprising embryonic cells, stem cells. immune cells, or any combination thereof. A heterogeneous cell population may be derived from bone marrow. A heterogeneous cell population may be derived by mechanical dislodgement from the bone marrow stroma by aspiration or by apheresis following mobilization into the peripheral blood (mPB) through activation and disruption of the molecular anchors. A heterogeneous cell population may be derived from peripheral-blood.

The stem cell may be selected from a heterogeneous population of cells. The phrase "heterogeneous population of cells" as used herein may refer to a mixture of cell types comprising a stem cell as defined above and at least one apoptosis-sensitive cell. The heterogeneous population of cells may be derived from any organism. The heterogeneous population of cells may be derived from a mammalian source. The heterogeneous population of cells may be derived from a human source.

The heterogeneous population of cells may comprise a mixture of a lineage positive cell and stem/progenitor cells. "a lineage positive cell" as used herein refers to a cell expressing mature cell lineage marker. A mature cell lineage marker may be a Cluster of Differentiation (CD) protein.

The method as disclosed herein may be used to perform lineage depletion.

The heterogeneous population of cells may be a tissue or a part thereof. The heterogeneous population of cells may be a cell aggregate. The heterogeneous population of cells may be a single cell suspension. The heterogeneous population of cells may be a primary culture. The heterogeneous population of cells may be a cellular sample. The heterogeneous population of cells may comprise a population which is accessible to the pro-apoptotic agents of the present invention.

Incubating within the device may last from 2 hours to 72 hours. Incubating within the device may last from 2 hours to 4 hours. Incubating within the device may last from 4 hours to 10 hours. Incubating within the device may last from 10 hours to 24 hours. Incubating within the device may last from 12 hours to 36 hours. Incubating within the device may last from 24 hours to 48 hours. Incubating within the device may last from 36 hours to 72 hours. Incubating within the device may last from 48 hours to 72 hours.

The incubation time within the device may be from 6 hours to 72 hours. The incubation time within the device may be from 12 hours to 72 hours. The incubation time within the device may be from from 18 hours to 72 hours. The incubation time within the device may be from 24 hours to 72 hours. The incubation time within the device may be from 30 hours to 72 hours. The incubation time within the device may be from 36 hours to 72 hours. The incubation time within the device may be from 42 hours to 72 hours. The incubation time within the device may be from 48 hours to 72 hours. The incubation time within the device may be from 54 hours to 72 hours. The incubation time within the device may be from 60 hours to 72 hours. The incubation time within the device may be from 66 hours to, 72 hours. The incubation time within the device may be from 2 hours to 66 hours. The incubation time within the device may be from 2 hours to 60 hours. The incubation time within the device may be from 2 hours to 54 hours. The incubation time within the device may be from 2 hours to 48 hours. The incubation time within the device may be from 2 hours to 42 hours. The incubation time within the device may be from 2 hours to 36 hours. The incubation time within the device may be from 2 hours to 30 hours. The incubation time within the device may be from 2 hours to 24 hours. The incubation time within the device may be from 2 hours to 18 hours. The incubation time within the device may be from 2 hours to 12 hours. The incubation time within the device may be from 2 hours to 6 hours.

Unexpectedly, the inventors found dial different incubation times with different combinations of apoptosis-inducing ligands may result in functionally distinctive clinical outcomes. Therefore, incubation time is in accordance of specific use, as is exemplified herein below.

In some embodiments, a kit is disclosed. The kit can be used in the selection of stem and progenitor cells that are resistant to receptor-mediated apoptosis.

The cell selection kit may comprise a device and a solution for maintaining the integrity and/or activity of an apoptosis-inducing ligand within the device.

The kit may further comprise an insert with instructions for performing cell selection according to the disclosed methods.

The solution for maintaining the integrity and activity of an apoptosis-inducing ligand may be a buffer or media. The constituents of the solution may vary depending on the apoptosis-inducing ligands used.

The solution may comprise a protease inhibitor. The protease inhibitor may be selected from, but not limited to: Phenylmethylsulfonyl fluoride, Benzamidine, Pepstatin A, Leupeptin, Aprotinin. Antipain, EDTA, EGTA, or any combination thereof. The solution may comprise a buffer system selected from, but not limited: to TRIS buffer, Phosphate-buffered saline, and Glycine-NaOH.

The solution may comprise elements which enable and/or promote cell survival, cell growth, cell proliferation, or any combination thereof. Elements which enable and/or promote cell survival, cell growth, cell proliferation, or any combination thereof include: growth media, serum or anti- bacterial agents. The solution enables to maintain viability of a cell within the device of the kit.

The solution may comprise factors which enable stem-cell and/or progenitor cell proliferation. Factors which enable stem-cell proliferation are selected from, but are not limited to, growth factors, hormones, enzymes or chemicals.

The kit can include an additional apoptosis-inducing ligand, e.g.. TNF $\alpha$ that is added to the device or to the solution, thus rendering the solution as selective towards the apoptosis resistant cells of the invention.

The kit can be used to provide flexibility in the identity or concentration of apoptosis-inducing ligands that are added to a population of cells. According to this, the kit comprises additional containers, each containing a different apoptosis inducing ligand. The kit's user selects a combination of apoptosis-inducing ligands according to a specified use, as is exemplified below, introduces the ligands into the device and allows incubation with the ligands.

The kit can be used to provide temporal flexibility in addition of the apoptosis-inducing ligands. As is exemplified herein below, different combinations, sequential administration or incubation times of apoptosis- inducing ligands results in a clinically distinct outcome. The kit's user can select the desired order of addition of the apoptosis-inducing ligands and the incubation period with each ligand within the device, thus achieving the clinical outcome of choice.

The present disclosure can be used in a method for improving the clinical outcome of hematopoietic stem and progenitor cells (HSPC) transplantation. A sample comprising a cell population can be provided; the cell population may comprise stem and progenitor cells. The population may be incubated in a device comprising the selective surface. Upon contact with the apoptosis-inducing ligand (e.g., FasL and/or TNF$\alpha$) which is immobilized on the selective surface apoptosis sensitive cells undergo apoptosis and the remaining cells (i.e., the cells that have not undergone apoptosis) are retrieved and used for transplantation in a mammal (e.g., but not limited to, a human). The contacting may occur within the device of the present invention, wherein the device can have at least one selective surface, or has a plurality of selective surfaces (e.g., but not limited to beads and/or multi-well plates), where the at least one selective surface or the plurality of selective surfaces is/are composed of or comprise a biocompatible polymer comprising maleic anhydride bound to an apoptosis-inducing ligand (e.g., FasL and/or TNF$\alpha$). The cell population may be derived from: bone marrow, a stem/progenitor cell mobilized peripheral blood, or umbilical cord blood (UCB). The stem/progenitor cell can be selected using the method, and is selected from a group comprising: umbilical cord blood stem/progenitor cell, mobilized peripheral blood stem/progenitor cell, bone marrow stem/progenitor cell, hematopoietic stem cell, cancer stem/progenitor cell, and neural stem/progenitor cell. The population of cells may be incubated with FasL for a period of 21 to 24 hours. The population of cells may be incubated with tumor necrosis factor $\alpha$ (TNF-$\alpha$) for a period of 24 to 48 hours. The population of cells may sequentially be incubated with (1) FasL and (2) TNF-$\alpha$. The population of cells may sequentially be incubated with (1) TNF-$\alpha$ and (2) FasL. The population of cells may be incubated with FasL and TNF-$\alpha$ for an overlapping duration. Retrieved cells from the method may be used for either autologous, allogeneic or haploidentical transplants. The retrieved cells may further comprise an immune-cell insensitive to activation-induced cell death (AICD). The immune cell insensitive to AICD may be a T cell.

Allogeneic cord blood may be introduced into the device related to the kit. FasL may be immobilized on a selective surface or a plurality of selective surfaces of the device. The blood may be incubated in the device for less than 24 hours. When the incubation has completed, TNF-$\alpha$ is introduced into the device and incubation is continued for 24-48 hours. Following the second incubation the cells are extracted from the device and are transplanted into the patient. An isolation step may be used between the end of incubation and transplantation, in order to isolate the living cells-from cell debris and proteins in the solution.

The cell population can be derived of an autologous transplant. The cell population can be derived of an allogeneic transplant.

The present disclosure can be used in a method for eliminating at least one malignant cell in a composition comprising a stem/progenitor-cell transplant. A composition comprising a stem/progenitor-cell transplant may be provided and then contacted/incubated with an apoptosis-inducing ligand. The contacting/incubating may occur within the device and/or on a surface or plurality of surfaces.

The composition may be contacted with the apoptosis-inducing ligand FasL for a period of, e.g.. but not limited to: about 24 hours. The composition may be contacted with the apoptosis-inducing ligand TNF-$\alpha$ for a period of, e.g., but not limited to: about 24 hours. The stem/progenitor-cell transplant may be used as an autologous transplant.

The present disclosure can relate to a method for reducing the numbers of malignant cells in a cell population. In this method, the cell population is contacted and incubated with at least one apoptosis-inducing ligand, e.g., FasL and/or TNF$\alpha$. The composition may comprise a progenitor-cell transplant.

The apoptosis-inducing ligand is FasL. The incubation may be for about 24 hours. The inventors have shown *in-vivo* that, unexpectedly, addition of FasL for about 24 hours to a transplant containing a malignant cell eliminates the malignant contaminant and improves survival rate (see, e.g., Figure 17E-F). This elimination technique has the potential to reduce the amount of malignant cells in grafts from apparently healthy donors that suffer of subclinical malignant disease.

As disclosed herein, a transplant may be extracted out of the bone marrow of a cancer-patient and introduced into the device. The transplant may be incubated with immobilized FasL and/or TNFalpha for about 24 hours. Upon completion of the incubation, it is disclosed that the selected cells may be transplanted back into the cancer patient. It is also disclosed that an isolation step may precede transplantation, as so to isolate the selected cells from cell debris and proteins.

There is also disclosed a method for selecting cells using a selective surface, which can then be used to treat a mammal, and where the selected cells reduce the likelihood of causing graft vs. host disease (GvHD) while retaining graft vs. tumor (GvT) activity. According to this method, a sample is provided. The sample may comprise a cell population. The cell population may comprise HSPC. The cell population may comprise an immune cell. The cell population may comprise HSPC, an immune cell or a combination thereof. The cell population may be contacted with an apoptosis-inducing ligand, e.g., FasL and/or TNFα. Surviving cells may be retrieved from the device having a selective surface composed of, e.g., but not limited, maleic anhydride bound to FasL and/or TNFα. In another embodiment, the retrieved cells are transplanted in a subject.

It is also disclosed that the method provides a selection of T cell subsets that do not induce GvHD using a selective surface. The inventors have found, unexpectedly, that short incubation with FasL, of 2-16 hours, without concurrent T cell sensitization, results in an effective removal of GvHD effectors (see, e.g., Figure 11). According to this, survival of T cell subsets which support hematopoietic progenitor engraftment is achieved, as treatment of transplanted cells with FasL did not impair engraftment (see, e.g., Figure 16).

The selective elimination of apoptosis-sensitive T cells from the donor inoculum does not impair generation of a potent graft vs. tumor (GvT) reaction.

The method as disclosed herein can be used on an allogeneic mobilized peripheral blood transplant using a selective surface. The allogeneic mobilized peripheral blood transplant may be introduced into a device. The transplant is next incubated with immobilized FasL and/or TNFalpha bound to a selective surface composed of, e.g., but not limited to, maleic anhydride for a short period of 2-16 hours. Following incubation. It is disclosed herein that the remaining cells may be retrieved and transplanted into the patient. It is also disclosed that an isolation step may precede the transplantation, as so to isolate the selected cells from cell debris and proteins.

As also disclosed herein, the method as disclosed may use a selective surface for selective depiction of unstimulated T cells prior to donor lymphocyte infusion (DLI).

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

EXAMPLES

Example 1

**Coating a surface with an apoptosis inducing ligand**

The following example illustrates the use of coating a surface to select for a population of stem and progenitor cells. While this example included the use of a plate, the results discussed herein can be extrapolated and used on other surfaces, such as beads or surfaces of containers.

**Materials and Methods:**

Immobilizing FasL on Maleic Anhydride (MA) coated surfaces

MA-bound tissue culture well plates (e.g., Pierce® Amine-binding, Maleic Anhydride Plates Nos. 15100, 15102, 15110, 15112. or 15108); Immobilization buffer (phosphate-buffered saline (PBS) pH 7.2; or 0.1M sodium phosphate, 0.15M sodium chloridc, pH 7.2 (Thermo scientific (Pierce) Cat. No. 28372), or an amine-free buffer, e.g., carbonate/bicarbonate buffer 0.2M, pH 9.4); Recombinant human Super FasLigand™ (Enzo Life Sciences); MegaFasL™ or APO010 (Adipogen); Protein blocking buffer (e.g., Thermo Scientific SuperBlock Blocking Buffer, Product No. 37515 or 37535); and wash buffer (PBS containing 0.05% Tween®·20 Detergent).

The polystyrene tissue culture well plates were washed three times with wash buffer (e.g. with 200µL). FasL was dissolved in the Immobilization buffer and various amounts (e.g. 0.5ng, 10ng, 50ng or 200ng) of FasL were added to each well (e.g., FasL concentration was 1µg/ml and 200µl of the FasL solution was used). The plates were incubated (while shaking) for 1 hour at room temperature or 37 degrees C, or overnight at 4 degrees C or at room temperature or 37 degrees C. The FasL solution was removed, and then Protein Blocking Buffer was added and incubated (e.g. 200µL of Protein Blocking Buffer was added per well and incubated for 1 hour at room temperature). Wells were washed (e.g. 3 times with Wash Buffer, and then washed with PBS) and the assay method was performed.

Seeding of Jurkat cells

Jurkat cells (a human T lymphocyte cell line) were centrifuged at 1300 rpm (300g) for 8' at 4°C and the supernatant was discarded. The cell pellet was re-suspended in 10ml RPMI complete medium. 20 $\mu$l of re-suspended cells were sampled and mixed with 190$\mu$l of pre-diluted Trypan Blue. The mixture was loaded (10 $\mu$l) into a hemocytometer (total loading volume = 20 $\mu$l). The number of viable cells and of dead cells was counted in at least 4 fields. The concentration of viable cells (viable cells / ml) were calculated using the following equation:

$$\left( \frac{\text{Number of viable cells counted}}{\text{Number of fields}} \right) \times \text{Dilution (20)} \times 10^4$$

Calculate cell viability:

$$\frac{\text{Number of viable cells} \times 100}{\text{Total number of cells (viable + dead)}}$$

The cells were seeded at a concentration of 2.5x10$^6$ cells/ml (0.5x10$^6$ cells/well) in 96 well plate (0.2ml per well), in RPMI complete medium, with or without immobilized FasL. As control, cells were incubated in regular well plates (without MA and without immobilized FasL) in the presence of 50 ng/ml FLAG-tagged soluble super FasL. The cells were incubated for 6 hours at 37°C in 5% $CO_2$ in a humidified incubator, and then collected. 2 ml PBS were added to wash the cells, followed by centrifugation at 4°C at 1300 rpm (300g) for 8 minutes. Annexin V staining was performed to identify dead cells (Annexin V is an apoptotic marker and PI is a marker for dead cells). The number of cells was determined using FACS analysis.

FACS analysis

At the end of the incubation time the cells (200$\mu$l) were removed from the wells and placed in FACS tubes filled with 1ml PBS (between 0.5-1 x 10$^6$ cells in each tube). The cells were centrifuged for 3 min at 1300 RPM, 4°C. The supernatant was discarded and 155 $\mu$l of AnnexinV solution (eBioscience) was added to each tube. The tubes were incubated in room temperature, in the dark for 10 min. At the end of incubation 1ml PBS was added to each sample and the tubes were centrifuged. 200 $\mu$l of PI/7AAD staining solution (eBioscience) were added into each well. Negative control samples were prepared without staining (i.e. an unstained sample, Annexin V only sample, and PI/7AAD only sample). The samples were read using a Flow Cytometer (using FACS Calibur, according to the BD CellQuest™ Pro Software Acquisition Tutorial).
Table 1 illustrates the conditions of the experiments.

Table 1:

| | Name of treatment (amount of superFasL per well) | Number of repetitions |
|---|---|---|
| 1 | 100 ng superFasL | 3 |
| 2 | 50 ng superFasL | 3 |
| 3 | 10 ng superFasL | 3 |
| 4 | 5 ng superFasL | 3 |
| 5 | 10 ng soluble superFasL (50 ng/ml) | 3 |
| 6 | No superFasL | 3 |

Results

FasL was immobilized onto MA-coated cell culture well plates as described above. The amount of immobilized FasL was measured by ELISA using a specialized kit (Human Fas Ligand/TNFSF6 DuoSet ELISA CAT # DY126). The measurements were performed according to manufacturer's instructions. As shown in Figure 1A ~3,5 ng/ml superFasL was bound to the plates when 100ng superFasL were added and -4.5 ng/ml superFasL was bound to the plates when 250ng superFasL were added. The values stated are extrapolated from the calibration curve as the maximum concentration of the curve is 1 ng/ml. In order to assess the apoptotic activity of superFasL bound to the MA-coated plates, Jurkat cells were seeded onto FasL immobilized MA-coated well plates. Various concentrations of superFasL were used for immobilization. Soluble superFasL served as a positive control (10 ng were added per well to reach a final concentration of 50 ng/ml). While the soluble superFasL induced >60% Annexin V staining of Jurkat cells (indicative of apoptosis), the maximal amount of bound superFasL to MA-coated plates (100 ng) induced a ~35% staining of Jurkat cells, which is ~57% of the effect of soluble superFasL. Furthermore, the effect was dose-dependent (Figure 1B).

**Example 2**

**Comparison of MA. Epoxy and NUNC Amino-Immobilizer coated plates**

Jurkat cells were seeded on maleic anhydride (MA) coated plates (Thermo scientific (Pierce), Cat. No. 15100) which were previously incubated with 1000ng/ml MegaFasL (Adipogen) overnight at 4°C. The cells were incubated for 4 hours or for 6 hours. As control, cells were seeded onto MA coated plates without FasL. As can be seen in Figure 2A there was a minor increase of 2.4+0.7 fold in the levels of early apoptotic cells following a 4 hour incubation and a 10.1 ±4.9 fold increase following 6 hours of incubation, reaching about 30% apoptotic cells.

Next, Jurkat cells were seeded on Nunc amino-immobilizer coated plates (Thermo Scientific Nunc, Cat. No. 436006) which were previously incubated with 1000ng/ml MegaFasL (Adipogen) or SuperFasL (Enzo Life Sciences). The cells were incubated for 4 hours or for 6 hours. As control, cells were seeded onto the same plates without immobilized FasL. As can be seen in Figure 2B there was no difference in the levels of apoptotic cells between the groups with or without FasL, the presence of FasL not exceeding 4.5% of apoptotic cells in any of the samples.

Next, Jurkat cells were seeded on 3D-Epoxy coated 96 well plates (PolyAn, Cat. No. 00 690 251) which were previously incubated with 1000ng/ml MegaFasL. The cells were incubated for 4 hours or for 6 hours. As control, cells were seeded onto the same plates without immobilized FasL. As can be seen in Figure 2C there was no difference in the levels of apoptotic cells between the groups with or without FasL, the presence of FasL not exceeding 4.5% of apoptotic cells in any of the samples.

Example 3

Apoptosis of Jurkat cells seeded onto maleic anhydride - biocompatible polymers

Attaching FasL to maleic anhydride-coated fluoropolymer or polyethylene films

Copolymers comprising Gamma-irradiation stable polymers ETFE (poly (ethene-co-tetrafluoroethene)) or HDPE (high density polyethylene) and maleic anhydride were prepared according to methods known in the art (see for example Nipithakul T. et al Chinese J. of Polymer Science Vol. 30, No. 2 (2012) or Chausse et al Clin. Chem 36/3, 525-528 (1990), the contents of which are incorporated herein by reference). The copolymer films were then cut into 24-well size discs and put into cell culture plates. The discs were kept in the dark until use. The discs were then washed twice with Wash buffer and coated with FasL overnight at 4 degrees C as described in Example 1 above.

Apoptosis of Jurkat cells incubated on FasL-coated maleic anhydride-fluoropolymer films

Next, Jurkat cells were seeded on a fluoropolymer (ETFE)-maleic anhydride (MA) substrate or a polyethylene-maleic anhydride (MA) substrate which was previously incubated with 1000ng/ml MegaFasL (FL-MA-FasL and PE-MA-FasL, respectively). The cells were incubated for 6 hours. As control, cells were seeded onto the fluoropolymer-maleic anhydride (MA) substrate or a polyethylene-maleic anhydride (MA) substrate without FasL. Additional control included seeding without polymer. A positive control included seeding without polymer in the presence of soluble FasL. As can be seen in Figure 2D there was an increase in the amount of apoptotic cells when seeded onto FL-MA-FasL or PE-MA-FasL as compared to the polymers in the absence of immobilized FasL.

FasL (Fc-FasL, monomer FasL and MegaFasL) was attached onto maleic anhydride (MA)-coated fluoropolymer as described above using 1000ng/mL or 1500 ng/ml solutions. Following coating with the ligands, human Jurkat cells were incubated with the bound ligands for 4 hours and stained with Annexin V to test early apoptosis (Fig. 2E). Despite differences in levels of efficiency all types of FasL molecules were effective in inducing apoptosis. Interestingly, as shown in Figure 2F, the EC50 of soluble monomer FasL is very high 362422ng/ml as compared to the MegaFasL (1.28ng/ml) and Fc-FasL (26.25ng/ml). Namely, the efficacy of apoptosis induction of the monomer FasL when administered as a soluble molecule is very low. The data thus demonstrate that binding of ligands to the polymer surface increases their efficacy and reduces significantly the difference in apoptosis induction efficacy of the three ligand molecules. Namely, the attachment of the FasL to the MA-coated fluoropolymer increases significantly the apoptosis induction efficiency of the ligands.

Determination of the efficacy of beads

In an experiment, polystyrene magnetic beads are used and functionalized with MA for binding FasL. In this experiment, the functionalized MA-bound beads induce apoptosis in human Jurkat cells, but not limited to, and can be used for the selection of stem and progenitor cells.

## Example 4

### *Ex vivo* exposure of umbilical cord blood cells to death ligands

Apoptotic activity of death receptor activation *in vitro*

The primary activity of the TNF superfamily is transduction of apoptotic signals. Fresh umbilical cord blood (UCB) obtained from term deliveries upon informed consent were exposed to FasL and TNF-$\alpha$ for various periods of time in liquid culture without supplementation of growth factors. Gated CD34$^+$ progenitors within the bulk UCB cultures displayed reduced rates of apoptosis, with increasing susceptibility as a function of time (see, e.g., Figure 3A), however apoptotic cell death was not enhanced by exposure to death ligands of the TNF family. The analysis revealed lower expression of the Fas and TNF receptors in gated CD34$^+$ and isolated lin$^-$ progenitors than in the bulk UCB population (see, e.g., Figure 3B). More focused evaluation of subsets of cells expressing the receptors showed excessive susceptibility to apoptosis of CD34$^+$ progenitors, however apoptosis was not induced by the cognate receptors with one exception (see, e.g., Figure 3C). Short-term exposure to TNF-$\alpha$. (<24 hours) was the only case of detectable receptor-mediated apoptosis of CD34$^+$ cells expressing both TNF-R1 and TNF-R2. These data suggested that expression of the death receptors was a characteristic of cells that were particularly susceptible to spontaneous apoptosis in liquid culture. The variations in apoptosis may have originated from differential rates of proliferation, however increased susceptibility to apoptosis of receptor-positive CD34$^+$ progenitors were associated with reduced proliferation rates that were also unaffected by the presence of the cognate ligands (see, e.g., Figure 3D).

To define which subsets of UCB cells were susceptible to apoptosis, the myeloid and lymphoid lineages were further assessed. Whereas CD3$^+$ T cells express high levels of Fas, the TNF receptors were dominant in CD33$^+$ myeloid cells (see, e.g., Figure 3E). TNF-R1 was in general expressed at higher levels than TNF-R2 in all the lineage-positive subsets. Exposure to the apoptotic challenge with death ligands for 48 hours revealed excessive rates of spontaneous apoptosis of all lineage-positive subsets as compared to lin$^-$ progenitors, which were largely unaffected by the presence of ligands (see, e.g., Figure 3F). Two notable exceptions were observed: a) Fas-mediated apoptosis in CD14$^-$CD33$^+$ myeloid cells, corresponding to UCB neutrophils, which express high levels of the TNF receptors; and b) TNF-induced apoptosis in CD3$^+$ T cells. Resistance of UCB-derived T cells to apoptosis in liquid culture was explained by the naïve nature of this antigen-inexperienced subset, which requires stimulation in order to sensitize to AICD-type negative regulation. Therefore, apoptosis of unstimulated UCB cells in culture was governed by death of CD19$^+$B lymphocytes and myeloid cells (CD14$^+$, CD33$^+$), with selective apoptotic functions of the TNF family receptors in the different subsets.

SCID repopulating cells and long-term culture initiating cells were resistant to receptor-mediated apoptosis

Several surrogate assays were employed to evaluate the function of progressively committed progenitors, including SCID repopulating cells (SRC), long-term initiating cells (LTC-IC) and short-term clonogenic assays. SRC activity was partially compatible with the human reconstituting cell and served as a surrogate functional assay for the most primitive progenitors within the sample. Equal initial numbers of cells incubated with and without FasL and TNF-$\alpha$ prior to transplantation were used, because the wide variability in levels of xenochimerism precludes precise evaluation of enhanced engraftment (see, e.g., Figure 4A). SRC function was preserved after exposure to FasL and TNF-$\alpha$ for 24-48 hours prior to transplantation (see, e.g., Figure 4B-D), indicating that the most primitive hematopoietic precursors were insensitive to spontaneous and receptor- mediated apoptosis in liquid culture. Extended *ex vivo* incubation was associated with loss of engraftment capacity of human hematopoietic progenitors, irrespective of the presence of FasL and TNF-$\alpha$ (see, e.g., Figure 4E). Prolonged *ex vivo* incubation of UCB cells in liquid medium supplemented with chemokines and growth factors was associated with dramatic changes in phenotype and decreases cell homing and engraftment in immunocompromized mice. However, unlike prior studies that attributed deficient SRC engraftment to receptor-mediated apoptosis by death ligands, the detrimental influences of extended culture were largely independent of activation of membranal death receptors.

Deficient human cell engraftment after extended periods of ex *vivo* culture was associated with proliferation and egress from the G0/G1 cell cycle phase. Both the proliferation rates (see, e.g., Figure 4E) and the cell cycle phase (see, e.g., Figure 4F) of UCB cells, CD34$^+$ and lin$^-$ progenitors were unaffected by the presence of FasL and TNF-$\alpha$ after 48 hours of incubation. Preservation the high fractions of mitotically-quiescent progenitors in the presence of the death ligands explains equal levels of SRC engraftment, which was restricted to cells positioned in G0/G1.

Surrogate *ex vivo* assays for hematopoietic progenitor activity include long-term cultures over mesenchymal stromal cells (LTC-IC), which represent a more primitive subset than colonies formed by committed progenitors in semisolid methylcellulose cultures. Exposure to FasL and TNF-$\alpha$ at concentrations that were toxic to somatic cells during the entire 5 weeks period of the long-term cultures did not impact LTC-IC activity (Figure 4G), consistent with overall resistance of the CD34$^+$ and lin$^-$ progenitors to Fas and TNF receptors-mediated apoptotic signaling. Thus, despite remarkable modulation in composition of viable subsets during exposure to the death ligands caused by differential inherent susceptibilities of various UCB subsets to apoptosis, SRC and LTC-IC activities of the most primitive progenitors were preserved, reflecting resistance to receptor-mediated apoptosis.

Enrichment of myeloid progenitors by elimination of dead cells

Inherent susceptibility to spontaneous and receptor-induced apoptosis of various subsets of UCB cells results in marked variations in the composition of viable cells after *ex vivo* incubation. For example, following 48 hours of incubation with toxic doses of FasL there was marked enrichment in viable lineage-negative cells, whereas incubation with TNF-$\alpha$ results in relative enrichment in viable myeloid cells (see, e.g., Figure 5A). It was reasoned that the enrichment in progenitors following exposure to death ligands would augment clonogenic activity in semisolid cultures. Variations in colony forming unit (CFU) frequency were evaluated following removal of dead cells by sedimentation over ficoll and plating of equal numbers of viable UCB cells in semisolid methylcellulose cultures (see, e.g., Figure 5B) stimulated with stem cell factor (SCF), interleukin-3 (IL-3) and granulocyte-macrophage colony stimulating factor (GM-CSF). Consistent with predominant apoptosis of mature UCB cells, CFU-GM frequency increased from 1:143 in control cultures to 1:37 and 1:31 after 48 hours of exposure to FasL and TNF-$\alpha$, respectively (see, e.g., Figure 5C). While maximal enrichment was observed after 48 hours of incubation, extended *ex vivo* culture beyond this period had detrimental consequences on the function of committed myeloid progenitors. This assay uncovered selective receptor-mediated elimination of non-progenitor cells in UCB samples, which despite similar overall rates of apoptosis resulted in 4-5 fold increase in CFU-GM frequency.

Cross-talk between TNF family receptors did not sensitize to apoptosis

To determine whether TNF-induced Fas expression sensitized UCB cells to apoptosis, cells were first exposed to TNF-$\alpha$ and then exposed to FasL (see, e.g., Figure 6A), resulting in marked upregulation of the Fas receptor (see, e.g., Figure 6B). Whereas Fas$^+$ cells were induced into apoptosis by exposure to FasL during the second day of culture, UCB cells, gated CD34$^+$ and isolated lin$^-$ progenitors were relatively protected by the presence of TNF-$\alpha$ (see, e.g., Figure 6C), indicating that upregulation of Fas expression was dissociated from the sensitivity to apoptosis. Functional assays of progenitor enrichment by ligand-mediated elimination of apoptosis-sensitive cells showed that joint exposure to FasL and TNF-$\alpha$ increase CFU-GM frequency (see, e.g., Figure 6D) and consistently, joint exposure of UCB cells to both ligands did not impair SRC activity *in vivo* (not shown). These data documented resistance of hematopoietic progenitors to apoptotic signal transduction by two independent pathways, their joint activation, and crosstalk between TNF family receptors.

Depletion of differentiating cells during *ex vivo* expansion of UCB cells

Umbilical cord blood was a sufficient source of hematopoietic progenitors for reconstitution of the immuno-hematopoietic system after aggressive radiochemotherapy, however had two major disadvantages: small number of cells and slow engraftment. To overcome these limitations, a number of approaches to *ex vivo* expansion of UCB cells prior to transplantation have been developed. The cells were numerically expanded and progenitors became activated, which improved the quality of hematopoietic reconstitution. It was reasoned that exposure of the cell cultures to TNF-family ligands awarded a relative advantage to more primitive progenitors by limitation of the clonal expansion of differentiated cells, which did not impact significantly the efficiency of engraftment. Using a standard expansion protocol that increased the fraction (see, e.g., Figure 7A) and absolute numbers of CD34$^+$ progenitors (see, e.g., Figure 7B), supplementation FasL during the third and final week of *ex vivo* culture resulted in further significant expansion of the CD34$^+$ subset. To determine the frequency and function of SRC, the most primitive assay for human hematopoietic progenitors, equal numbers of UCB cells from the same UCB unit were grafted into Busulfan-conditioned immunocompromized NOD.SCID mice (see, e.g., Figure 7C). Exposure to FasL during the third week of culture resulted in equal and/or higher levels of human hematopoietic xenochimerism (see, e.g., Figure 7D), emphasizing that the function of progenitors was improved. The wide variability of engraftment in individual mice precluded more accurate determination of superior functionality of the increased numbers of expanded CD34+ progenitors. Thus, selective depletion of apoptosis-sensitive cells was an effective way to increase the fractions and umbers of *ex vivo* expanded UCB progenitors for transplantation.

## Example 5

### *Ex vivo* exposure of mobilized peripheral blood cells to death ligands

Apoptotic activity of death receptor activation *in vitro*

A prevalent source of hematopoietic progenitors for transplantation was peripheral blood following mobilization with granulocyte colony stimulating factor (G-CSF) or antagonists of c-kit and CXCR4. The mobilized mononuclear cells were subsequently collected from peripheral blood by apheresis, containing a substantial number of CD34+ progenitors. Cells harvested from the peripheral blood were generally activated, therefore the periods of exposure to death ligands for selective depletion were significantly shorter.

An additional difference from data presented for UCB cells was the use of cryopreserved mPB samples, the thawing of which was associated with apoptotic death of 15-25% of the cells. Fas was expressed in ~25% of CD34+ progenitors (see, e.g., Figure 8A) and considerable fractions of B lymphocytes and myeloid cells (50-65%, see, e.g., Figure 8B). The TNF receptors were expressed primarily in lineage-positive mPB cells with predominant expression of TNF-R1. Following brief culture Fas expression decreased (see, e.g., Figure 8C), whereas all subsets displayed marked upregulation of TNF-R2 (see, e.g., Figure 8D). These data disclosed dynamic variations in death receptor expression in thawed mPB cells, which was affected on the one hand by death of Fas+ cells and on the other hand by upregulation of TNF-R2.

Whereas 25-45% of lineage-positive cells were apoptotic within 4 hours of *ex vivo* incubation, 60-90% of these cells were induced into apoptosis within 16 hours of incubation (see, e.g., Figure 8E). The presence of FasL and TNF-α did not attenuate significantly the high rates of apoptosis of lineage-positive cells observed in medium. The most prominent variation from UCB cells was the activated CD3+ T cell subset in mobilized peripheral blood (mPB), which was induced into apoptosis by the ligands within a short period of time. These relative sensitivities to receptor-mediated apoptosis result in significant changes in composition of viable cells, with remarkable reduction in T cells following 4 hours of incubation and of B lymphocytes and myeloid cells following 16 hours of incubation. At both time points CD3+ T cells were selectively depleted, enriching the viable fraction with CD34+ progenitors (see, e.g., Figure 8F). Therefore, brief incubation of mobilized peripheral blood with death ligands depleted subsets of mature cells and enriched progenitors in cryopreserved mPB samples.

Depletion of mPB-derived T cells sensitive to Fas cross-linking ameliorated graft versus host disease

The most significant cause of morbidity in mPB cell transplants was GvHD, a consequence of the intrinsic state of activation of T cells in peripheral blood To assess the impact of FasL on xenogeneic GvHD, NOD-SCID mice were grafted with $1.5 \times 10^7$ viable mPB from the same unit with and without exposure to the ligand. Preincubation with FasL for 4 hours resulted in survival of all mice, whereas incubation in medium caused death of one third of the recipients (see, e.g., Figure 9A). Survivors displayed similar levels of human hematopoietic chimerism (see, e.g., Figure 9B), demonstrating again that SRC within mPB were resistant to Fas-mediated apoptosis. Severe GvHD was the cause of mortality in mice inoculated with mPB incubated in medium, as determined by the severe weight loss (see, e.g., Figure 9C), clinical score (see, e.g., Figure 9D) and liver histology (see, e.g., Figure 9E). Survival and all these features of xenogeneic GvHD were alleviated by brief exposure of mPB cells to FasL. In addition, it was evident from the functional assay that despite apparently similar rates of overall apoptosis, the presence of the ligands affected distinct subsets of lineage-positive mPB cells.

Exposure to death ligands increased myeloid progenitor frequency

The composition of viable subsets within mPB cultures following brief incubation reflected differential sensitivities of mPB-derived immune cells to apoptosis mediated by the Fas and TNF receptors. The relative distribution of cells was consistent with the high sensitivity of CD3+ T cells to apoptosis during 4 hours of incubation and subsequent death of CD19+ B lymphocytes and CD33+ myeloid cells during longer incubation periods (see, e.g., Figure 10A). These variations suggested that mPB samples the frequency of progenitors was increased by brief incubation with the death ligands and elimination of the dead cells (see, e.g., Figure 10B). Exposure to FasL and TNF-α resulted in a 4-fold enrichment in myeloid progenitors, doubling the enrichment observed following incubation in medium (see, e.g., Figure 10C). Similar to UCB cells, exposure to death ligands resulted in increased CFU frequency, however in variance from UCB cells the period of mPB exposure to the death ligands was much shorter. These data complemented the efficiency and safety of the proposed functional elimination of GvHD effectors, showing that the apoptosis-insensitive progenitors were markedly enriched.

Pretransplant exposure to FasL did not impair immune and graft versus tumor reactivity

The crucial question in experiments using selective T cell depletion to prevent and reduce GvHD severity is whether the graft retains the capacity to elicit potent graft versus tumor reactions. Despite these changes in composition, mPB incubated in medium and with FasL preserve reactivity against irradiated allogeneic human mPB stimulators (see, e.g., Figure 10D). Consistently, inoculation of mPB with and without exposure to FasL has similar effects on tumor growth suppression in immunocompromized mice bearing subcutaneous HT29 human colon carcinoma tumors (see, e.g., Figure 10E). Therefore, unstimulated cryopreserved mPB cells exposed to FasL suppress lethal GvHD, foster engraftment and retain GvT reactivity.

**Example 6**

**Pretransplant depletion of T cells prevents lethal GvHD**

Prevention of lethal GvHD by *ex vivo* selective depletion of host-sensitized T cells

Both physical depletion of T cells from donor inoculum and FasL-mediated elimination of host-reactive T cells reliably prevented GVHD. Haploidentical murine transplants (parent to child) represented the extreme risk of GvHD, characterized by high levels of mortality. In first stage experiments were recapitulated using depletion of T cells by apoptotic signals following sensitization to host antigens. Antigen-specific sensitization *in vitro* for 2-3 days caused T cell receptor (TCR)-mediated stimulation of responsive T cells that concomitantly upregulated Fas and its cognate ligand, resulting in execution of the apoptotic cascade in parallel to downregulation of protective antiapoptotic mechanisms. To compare this procedure to tested approach using brief exposure to apoptotic ligands *ex vivo* without prolonged incubation, the donors were pre- immunized against host antigens *in vivo* (see, e.g., Figure 11A). As expected, exposure of the sensitized splenocytes to FasL induced significant apoptosis in both $CD4^+$ and $CD8^+$ T cells (see, e.g., Figure 11B). The efficacy of sensitization was evident from stronger proliferative responses of lymphocytes harvested from B6 mice ($H2K^b$) immunized with BALB/c lymphocytes ($H2K^d$) against the stimulating $H2K^d$ alloantigens, as compared to third party ($H2K^k$) antigens (see, e.g., Figure 11C). These responses were sustained following brief (24 hours) incubation of the responders in control medium, however addition of FasL reduced the responsiveness of presensitized splenocytes to $H2K^d$ stimulators. The responses to third party $H2K^k$ antigens were preserved.

To evaluate the effects of FasL exposure on the ability of sensitized lymphocytes to cause GvHD, viable cells from the $H2K^d$-immunized $H2K^b$ parental donors were adoptively transferred into sublethally irradiated haploidentical F1 recipients ($H2K^b{\rightarrow}H2K^{b/d}$). Infusion of presensitized splenocytes incubated in control medium caused severe GvHD, which became uniformly lethal following lipopolysacharide (LPS) challenge leading to death of all mice (see, e.g., Figure 11D). The LPS challenge caused potent immune activation and release of inflammatory cytokines, which exacerbated to maximum ongoing GvHD reactions and precipitated death. By contrast, *ex vivo* incubation of splenocytes from presensitized donors with FasL prior to infusion led to survival of 50% of mice even after administration of LPS.

Prevention of lethal GvHD by *ex vivo* selective depletion of unstimulated T cells

We reasoned that pretransplant exposure of donor lymphocytes to host antigens might augment T cell stimulation, and that these alloresponses might persist due to the limited sensitivity of some effector/memory lymphocyte subsets generated in culture to Fas-mediated apoptosis or due to the inability of Fas cross-linking to eliminate completely all the alloreactive T cells. In addition, clinical adaptation of this presensitization approach to human donor:host pairs might be quite challenging. In search for a simpler and more reliable model of FasL selection of alloreactive T cells, the incubation technique was modified to use naive donor cells that had not been previously exposed to recipient alloantigens (see, e.g., Figure 11E). In the new approach, naive splenocytes were depleted *ex vivo* by exposure to FasL-containing medium in the absence of previous or concurrent exposure to host antigens and in the absence of proliferative stimuli. FasL increased apoptosis of naïve $CD4^+$ and $CD8^+$ T cells (see, e.g., Figure 11F), however alloresponses were preserved in viable cells subsequently stimulated *in vitro* (see, e.g., Figure 11G). Therefore, exposure of unstimulated splenocytes to FasL preserved the capacity of apoptosis-insensitive cells to respond to allogeneic antigens.

To assess the effect of *ex vivo* elimination of unstimulated lymphocytes, viable splenocytes were infused into sublethally irradiated haploidentical F1 recipients ($H2K^b$- GFP${\rightarrow}H2K^{b/d}$). Unsensitized lymphocytes incubated in medium were less potent mediators of lethal GvHD, therefore LPS was used to induce cytokine storm and polarize the activity of donor lymphocytes. Survival of 70% of recipients of FasL-pretreated unstimulated splenocytes following the LPS challenge was superior to survival of recipients of *ex vivo* depleted host-stimulated splenocytes, whereas lethal GvHD was precipitated in all recipients of splenocytes incubated in control medium (see, e.g., Figure 11H). Thus, treatment of donor lymphocytes with FasL prevented lethal GvHD (including LPS challenge) in a high-risk parent to F1 hybrid acute GVHD model in the absence of previous exposure to host antigens.

Quantitative and qualitative aspects of GvHD prevention

Adoptive transfer of viable splenocytes after incubation in control medium caused significant GvHD in sublethally irradiated haploidentical F1 recipients, which was blunted by donor cell preincubation with FasL (see, e.g., Figure 12A). The dose-dependent decrease in body weight was evidence of progressive GVHD severity in recipients of haploidentical splenocytes incubated in medium (see, e.g., Figure 12B). By contrast, weight loss was significantly reduced in F1 hybrid mice infused with naïve parental splenocytes that had been pre-incubated with FasL, consistent with amelioration of GvHD severity. The clinical GvHD score (see, e.g., Figure 12C) and weight loss (see, e.g., Figure 12D) in haploidentical transplants (H2K$^b$→H2K$^{b/d}$) exceeded those observed in allogeneic mouse combinations (H2K$^b$→H2K$^d$), and consistently the protective effect of preincubation with FasL was more effective in GvHD prevention in allogeneic transplants. Notably, adoptive transfer of 1.5-5x10$^6$ T cells in mice was equivalent to doses of 60-200x10$^6$ T-cells/kg in humans, whereas threshold safety for prevention of GvHD by phenotypic T cell depletion was 0.02x10$^6$ T-cells/kg in haploidentical transplants.

To evaluate the differences in immune profiles of survivors in the different experimental groups, the composition of the spleens was assessed before and 2 days after the LPS challenge (see, e.g., Figure 12E). All T and B cell subsets decreased during the first week following sublethal irradiation, irrespective of adoptive transfer of splenocytes. Recipients of lymphocytes incubated in control medium responded to LPS by a surge in splenic CD4$^+$ and CD8$^+$ T cells and B lymphocytes (p<0.001), which were significantly attenuated in recipients of FasL-pretreated splenocytes (p<0.001). Repetition of these experiments using purified T cells as opposed to unfractionated splenocytes showed similar results (data not shown).

Simulation of GvHD in NOD.SCID mice to determine the time of exposure to death ligands *ex vivo*

Prevention of GvHD by selective depletion of unstimulated donor T cells questions the involvement of two possible mechanisms. First, partial immuno and myelodepletion induced by sublethal irradiation suggests that residual host immune elements might operate successfully against FasL-deleted donor T cells, resulting in suppression of GvHD. Second, conditioning with total body irradiation induces tissue injury that plays an important role in the afferent arm of GvHD. To assess both these mechanisms, the disease was induced in lymphocyte-deficient NOD.SCID mice without pretransplant conditioning. Exposure of unstimulated splenocytes to FasL for 24 and 48 hours elicited significant apoptosis (see, e.g.. Figure 13A), with corresponding decrease in fractions of CD4$^+$ and CD8$^+$ viable T cells (see, e.g., Figure 13B).

Following a series of calibration experiments, NOD.SCID mice were infused with 3x10$^6$ or 10$^7$ naïve allogeneic splenocytes (H2K$^b$→H2K$^{g7}$) after 24 or 48 hours of *ex vivo* incubation in FasL-containing medium, respectively (see, e.g., Figure 13C). Infusion of splenocytes incubated in medium caused ~20% mortality at the higher dose, and severe mortality was recorded after administration of the LPS challenge (see, e.g., Figure 13D). The protective effects of FasL incubation on donor lymphocyte-mediated lethal GvHD were again highlighted by superior survival of mice following LPS injection (see, e.g., Figure 13D). This protective effect was accompanied by remarkable improvement in clinical score (see, e.g., Figure 13E) and weight loss (see, e.g., Figure 13F). Prevention of GvHD in NOD.SCID mice in the absence of competent residual host immunity and without predisposing host factors such as conditioning-induced tissue injury emphasizes that GvHD is primarily a consequence of modulation of donor T cell inoculum by exposure of unstimulated lymphocytes to FasL.

**Example 7**

**The impact *of ex vivo* selective depletion of unstimulated lymphocytes on hematopoietic cell engraftment**

Depletion of Fas-sensitive naive splenocytes alleviates GVHD in bone marrow transplants

To extend the findings of the GvH-like model to the transplant setting, the effect of Fas-mediated depletion of host-naive cells was assessed in mice undergoing haploidentical BMT together with donor lymphocyte infusion (see, e.g., Figure 14A). Irradiated F1 recipients that received 5x10$^6$ haploidentical BMC together with 1.5, 3 and 4.5 million splenocytes preincubated in control medium (H2K$^b$→H2K$^{b/d}$) manifest severe GvHD (see, e.g., Figure 14B) and significant 10-15% weight loss (see, e.g., Figure 14C). The clinical score of GvHD was corroborated by histological analysis of ear skin and liver specimens (see, e.g., Figure 14D). Preincubation of the infused naive splenocytes with FasL for 24 hours decreased the clinical GvHD score (p<0.005) blunted weight loss (p<0.005) and reduced lymphocytic infiltrates in the affected target organs (p<0.005). Residual low-grade GvHD activity did however persist after infusion of 3-4.5x10$^6$ FasL-pretreated haploidentical splenocytes. It is noteworthy that this dose of T cells corresponds to doses of 4-6x10$^7$ T cells/Kg, numbers of T-cells often administered in unmanipulated allogeneic whole bone marrow transplants from matched unrelated donors.

### Immuno-hematopoietic reconstitution following haploidentical BMT and selective depletion of apoptosis-sensitive cells

GvHD depresses graft function and profoundly abrogates recipient immune responses. To determine how depletion of Fas-sensitive donor splenocytes affects immuno-hematopoietic reconstitution following transplantation, hosts were assessed for survival of the grafted donor lymphocytes, chimerism and immune responsiveness to unrelated antigens. Recipients of the highest dose (4.5x10$^6$) of unmanipulated donor splenocytes preincubated in control medium displayed decreased levels of chimerism at 3 weeks and 6 weeks post-transplantation (see, e.g., Figure 15A), as compared to recipients of FasLpretreated splenocytes, demonstrating that pre-treatment of donor lymphocytes abrogated the graft suppressive effects of the untreated cells.

Because the mature lymphocytes that were infused at the time of transplantation compete with lymphocytes generated by the bone marrow graft for peripheral homeostatic expansion and effect the net lymphocyte numbers, the impact of FasL treated lymphocyte infusions on lymphoid reconstitution was examined. Minor CD45 antigen disparity (CD45.1 vs. CD45.2) were used to differentiate between infused splenocytes and BMC graft-derived T cells in the recipients during the weeks following transplant. Despite the fact that F1 recipients do not reject parental immune cells, few of the infused splenocytes (H2K$^b$, CD45.2$^+$GFP$^+$) were detected in peripheral lymphoid organs of the recipients at 3 weeks post-transplantation, whether or not they had been incubated with FasL or in control medium (see, e.g., Figure 15B). Qualitatively, recipients of control and FasL-pretreated splenocytes displayed unresponsiveness to both donor and host antigens as expected for chimeric mice, and responded equally well to third party (H2K$^k$) antigens (see, e.g., Figure 15C). Therefore, ex vivo treatment of lymphocytes with FasL seems to effect neither quantitative nor functional immune reconstitution after transplantation, yet prevention of GvHD improves donor cell engraftment.

We questioned whether prevention of GvHD by preincubation of unstimulated lymphocytes with FasL is caused by impaired navigation capacity of the cells. This possibility was assessed by infusion of viable splenocytes into sublethally irradiated F1 recipients (H2K$^b$-GFP→H2K$^{b/d}$) and demonstration that lymphocytes homed to recipient spleen and mesenteric lymph nodes 24 hours after infusion, whether the cells had been preexposed to FasL or to control medium (see, e.g., Figure 15D). These data demonstrate that preexposure of lymphocytes depletes potential GvHD effectors without impairing their navigation ability.

### Exposure of hematopoietic cells to death ligands improves engraftment

Depletion of all T cells from the hematopoietic grafts removes engraftment-facilitating cells, requiring a compensatory infusion of large doses of stem cells. Preincubation of the graft with FasL for 24 hours (see, e.g., Figure 16A) did not affect the levels of donor chimerism induced by transplantation of 2x10$^6$ allogeneic (H2K$^d$→.H2K$^b$) whole BMC into irradiated hosts (see, e.g., Figure 16B). Therefore, engraftment is not lost when heterogeneous whole BMC populations are exposed to death ligands before transplantation.

T cells support hematopoietic progenitor engraftment through two mechanisms: a) donor T cells may counteract graft rejection by residual donor immunity, and b) T cells co-reside with progenitors at sites of seeding in the bone marrow and support engraftment through unidentified non-immunogenic mechanisms. To determine whether depletion of apoptosis-sensitive T cells to prevent rejection also eliminates engraftment-facilitating cells, irradiated H2K$^b$ mice were grafted with 10$^6$ viable unmanipulated H2K$^d$ lin$^-$ progenitors. In this model splenocytes from F1 donors (H2K$^{b/d}$), which are devoid of GvHD activity were used. Infusion of 10$^6$ viable F1 splenocytes after incubation in control medium or with FasL increased the levels of donor chimerism (see, e.g., Figure 16C), demonstrating that depletion of Fas-sensitive splenocytes does not eliminate cells that facilitate HSPC engraftment.

### Preexposure of donor lymphocytes to death ligands preserves the engraftment-supporting activity of delayed donor lymphocyte infusion

Post-transplant infusion of donor lymphocytes (DLI) is an effective approach to improve donor chimerism, enhance responsiveness to infections and foster graft versus tumor reactions. Although delayed DLI is better tolerated and causes less GvHD than infusion of lymphocytes in conditioned recipients at the time of hematopoietic transplantation, the lymphocytes have the capacity to increase the severity of GvHD. Delayed donor lymphocyte infusion in a model of mixed chimerism (see, e.g., Figure 16E) was effective in increasing the levels of donor chimerism irrespective of pretransplant exposure of the lymphocytes to FasL (see, e.g., Figure 16F). Therefore, depletion of Fas-sensitive unstimulated lymphocytes does not impair the efficacy of post-transplant DLI.

FasL alleviates GVHD without impairing GvT

The overall success of stem cell transplantation performed for the treatment of malignant disease may depend on graft versus tumor (GvT) effects. Bulk depletion of T cells (either *ex vivo* or by administration of potent *in vivo* immune suppression) can blunt the GvT effect and increase post-transplant relapse rates in a variety of clinical situations. It was examined whether FasL incubations abrogated GvT effects in parallel to its salutary effect on GvHD in two experimental models. In a first model the preservation of GvT reactivity of host-matched lymphocytes after FasL-mediated depletion against an allogeneic tumor was assessed. CT26 colon carcinoma cells (H2K$^d$) were implanted in NOD.SCID mice (H2K$^{g7}$) adoptively transferred with $1.5 \times 10^7$ splenocytes from NOD (immunocompetent) donors (H2K$^{g7}$, see, e.g., Figure 17A). Splenocytes preincubated with FasL for 24 hours suppressed the growth of tumor implants to a similar degree as compared with splenocytes incubated in control medium (see., e.g., Figure 17B), confirming that incubation with FasL preserves lymphocyte-mediated GvT reactivity. In the second model H2K$^a$ mice bearing MHC-matched neuroblastoma (Neuro-2a, H2K$^a$) tumors were sublethally irradiated and grafted with $2 \times 10^6$ lin$^-$ progenitors from allogeneic donors (H2K$^b$, see, e.g., Figure 17C). Infusion of lymphocytes from Fl donor (H2K$^{b/d}$), which recognize the tumor as self, had no significant impact on tumor growth (see, e.g., Figure 17D). In variance, infusion of allogeneic splenocytes (H2K$^b$) reduced tumor growth rates, however 80% of the mice died within 3 weeks because of severe GvHD. *Ex vivo* Fas-mediated depletion of unstimulated donor splenocytes (H2K$^b$) showed equal tumor-suppression effect while alleviating lethal GvHD, and all mice survived to the experimental end point. These data confirm that GVHD is not a prerequisite for GvT effects, therefore prevention of GvHD does not impair antitumor activity of selectively-depleted lymphocytes.

FasL-mediated purging of malignant cells

Despite the significant advantages and safety of autologous transplants after aggressive radiochemotherapy, contamination of the graft with residual malignant cells is a risk factor of disease relapse. To evaluate this possibility, mice were infused with a mixture of bone marrow cells and A20 B cell lymphoma, at a dose that is lethal in Syngeneic BALB/c mice (see., e.g., Figure 17E). Whereas infusion of cell mixtures incubated in medium resulted in death of 80% of the recipients within 4 weeks, all mice infused with grafts exposed to FasL survived this period (see., e.g., Figure 17F). These data document the efficacy of short incubation with death ligands for depletion of apoptosis-sensitive malignant cells from the hematopoietic graft.

Depletion of Fas-sensitive cells prevents adoptive transfer of autoimmunity in autologous transplants

Type 1 diabetes is an autoimmune reaction that destroys the insulin-producing β- cells in the pancreatic islets of Langerhans. This disorder is generally modeled in non- obese diabetic mice (NOD), with a disease incidence above 80% in females aged 30 weeks. Autoimmune diabetes is adoptively transferred by T cells, but not so efficient by transplantation of whole bone marrow cells. Preliminary experiments determined that $5 \times 10^7$ bone marrow cells transfer the disease into ~50% of sublethally-irradiated NOD.SCID mice (see., e.g., Figure 17G), both strains bearing the same haplotype (H2K$^{g7}$). In addition, NOD.SCID mice do not reject the donor cells since they lack competent T and B lymphocytes. Whereas adoptive transfer of BMC incubated in medium for 48 hours transferred the disease effectively, none of the recipients of BMC exposed to FasL displayed hyperglycemia (see, e.g., Figure 17H). Therefore, exposure of hematopoietic cells to death ligands *ex vivo* eliminates cells with autoimmune reactivity.

While a number of embodiments of the present invention have been described, it is understood that these embodiments are illustrative only, and not restrictive, and that many modifications may become apparent to those of ordinary skill in the art. Further still, the various steps may be carried out in any desired order (and any desired steps may be added and/or any desired steps may be eliminated).

The methods are disclosed herein.

**Claims**

1. A selective surface, wherein said selective surface comprising:

   a biocompatible polymer comprising maleic anhydride molecules; and
   an apoptosis inducing ligand bound to the maleic anhydride molecules;
   wherein said biocompatible polymer is polyethylene or a fluorocarbon polymer;
   and wherein the apoptosis-inducing ligand is FasL.

2. The selective surface of claim 1,wherein said selective surface is capable of selecting apoptosis-signaling resistant cells.

3. The selective surface of any one of the preceding claims wherein said selective surface is in the form of an inner surface of a container, a film, a disc or beads.

4. The selective surface of any one of the preceding claims wherein said fluorocarbon polymer is Ethylene tetrafluoroethylene (ETFE).

5. The selective surface of claim 3 wherein said beads are selected from the group consisting of magnetic beads, paramagnetic beads, and dextran beads.

6. The selective surface of claim 3 wherein said beads are agarose beads, polystyrene beads, sepharose beads or silica beads bound to N-hydroxysuccinimide (NHS), NHS-activated agarose beads or beads comprising aldehyde-activated agarose, azlactone-activated support, carbonyl diimidzaole agarose/Trisacryl, carboxyl groups, cyanogen bromide, polyethylene glycol (PEG), and any combination thereof.

7. The selective surface of any one of the preceding claims, wherein the FasL is biologically active.

8. The selective surface of any one of the preceding claims wherein from about 0.01 to about 3ng/cm$^2$ of FasL is bound to the selective surface.

9. A device comprising the selective surface of any one of the preceding claims.

10. The device of claim 9 wherein said device is a container.

11. The device of any one of claims 9 to 10 wherein the selective surface is the selective surface of a plurality of beads contained within the container.

12. The selective surface of any one of claims 1 to 8 or the device of any one of claims 9 to 11 for use in selecting an apoptosis-signaling resistant cell.

13. Use of the selective surface of any one of claims 1 to 8 or the device of any one of claims 9 to 11 in a method of selecting an apoptosis-signaling resistant cell.

14. A method of preparing a device having a selective surface, said method comprising:

    (i) Obtaining a selective surface comprising a biocompatible polymer coated with or comprising maleic anhydride molecules (maleic anhydride-copolymer); wherein said biocompatible polymer is polyethylene or a fluorocarbon polymer;
    (ii) incubating said selective surface with an immobilization buffer or coating buffer comprising dissolved FasL;
    (iii) removing the unbound FasL from the selective surface;
    thereby obtaining a device having a selective surface comprising a biocompatible polymer coated with or comprising maleic anhydride molecules (maleic anhydride-copolymer), and wherein said surface further having FasL immobilized thereon;
    wherein said biocompatible polymer is polyethylene or a fluorocarbon polymer.

15. A cell selection kit comprising:

    a. the selective surface of any one of claims 1 to 9 or the device of any one of claims 9 to 11; and
    b. Instructions for use in the selection of stem and progenitor cells which are resistant to receptor-mediated apoptosis.

**Patentansprüche**

1. Selektive Oberfläche, wobei die selektive Oberfläche umfasst:

    ein biokompatibles Polymer, das Maleinsäureanhydrid-Moleküle umfasst; und

einen Apoptose-induzierenden Liganden, der an die Maleinsäureanhydrid-Moleküle gebunden ist; wobei das biokompatible Polymer Polyethylen oder ein Fluorkohlenstoff-Polymer ist; und wobei der Apoptose-induzierende Ligand FasL ist.

2. Selektive Oberfläche nach Anspruch 1, wobei die selektive Oberfläche fähig ist, gegen Apoptose-Signalgebung resistente Zellen zu selektieren.

3. Selektive Oberfläche nach einem der vorangehenden Ansprüche, wobei die selektive Oberfläche in Form einer inneren Oberfläche eines Behälters, eines Films, einer Scheibe (disc) oder von Beads ist.

4. Selektive Oberfläche nach einem der vorangehenden Ansprüche, wobei das Fluorkohlenstoff-Polymer Ethylen-Tetrafluorethylen (ETFE) ist.

5. Selektive Oberfläche nach Anspruch 3, wobei die Beads ausgewählt sind aus der Gruppe bestehend aus magnetischen Beads, paramagnetischen Beads und Dextranbeads.

6. Selektive Oberfläche nach Anspruch 3, wobei die Beads Agarosebeads, Polystyrolbeads, Sepharosebeads oder Silicabeads, die an N-Hydroxysuccinimid (NHS) gebunden sind, NHS-aktivierte Agarosebeads oder Beads, die Aldehyd-aktivierte Agarose umfassen, Azlacton-aktivierter Träger, Carbonyldiimidazol-Agarose/Trisacryl, Carboxylreste, Cyanogenbromid, Polyethylenglycol (PEG), und eine beliebige Kombination davon sind.

7. Selektive Oberfläche nach einem der vorangehenden Ansprüche, wobei der FasL biologisch aktiv ist.

8. Selektive Oberfläche nach einem der vorangehenden Ansprüche, wobei von etwa 0,01 bis etwa 3ng/cm$^2$ FasL an die selektive Oberfläche gebunden ist.

9. Vorrichtung, die die selektive Oberfläche nach einem der vorangehenden Ansprüche umfasst.

10. Vorrichtung nach Anspruch 9, wobei die Vorrichtung ein Behälter ist.

11. Vorrichtung nach einem der Ansprüche 9 bis 10, wobei die selektive Oberfläche die selektive Oberfläche einer Vielzahl an Beads ist, die in dem Behälter enthalten sind.

12. Selektive Oberfläche nach einem der Ansprüche 1 bis 8 oder Vorrichtung nach einem der Ansprüche 9 bis 11 zur Verwendung beim Selektieren einer gegen Apoptose-Signalgebung resistenten Zelle.

13. Verwendung einer selektiven Oberfläche nach einem der Ansprüche 1 bis 8 oder Vorrichtung nach einem der Ansprüche 9 bis 11 in einem Verfahren des Selektierens einer gegen Apoptose-Signalgebung resistenten Zelle.

14. Verfahren zur Herstellung einer Vorrichtung, die eine selektive Oberfläche hat, wobei das Verfahren umfasst:

(i) Erhalten einer selektiven Oberfläche, die ein biokompatibles Polymer umfasst, das mit Maleinsäureanhydrid-Molekülen (Maleinsäureanhydrid-Copolymer) beschichtet ist oder diese umfasst; wobei das biokompatible Polymer Polyethylen oder ein Fluorkohlenstoff-Polymer ist;
(ii) Inkubieren der selektiven Oberfläche mit einem Immobilisationspuffer oder Beschichtungspuffer, der gelösten FasL umfasst;
(iii) Entfernen des ungebundenen FasL von der selektiven Oberfläche;
wodurch eine Vorrichtung mit einer selektiven Oberfläche erhalten wird, die ein biokompatibles Polymer umfasst, das mit Maleinsäureanhydrid-Molekülen (Maleinsäureanhydrid-Copolymer) beschichtet ist oder diese umfasst, und wobei die Oberfläche des Weiteren einen FasL aufweist, der auf ihr immobilisiert ist; wobei das biokompatible Polymer Polyethylen oder ein Fluorkohlenstoff-Polymer ist.

15. Zellselektionskit, der umfasst:

a. die selektive Oberfläche nach einem der Ansprüche 1 bis 9 oder die Vorrichtung nach einem der Ansprüche 9 bis 11; und
b. Anleitungen zur Verwendung bei der Selektion der Stamm- und Vorläuferzellen, die gegenüber Rezeptor-vermittelter Apoptose resistent sind.

**Revendications**

1. Surface sélective, laquelle surface sélective comprend :

   un polymère biocompatible comprenant des molécules d'anhydride maléique ; et
   un ligand inducteur d'apoptose lié aux molécules d'anhydride maléique ;
   dans laquelle ledit polymère biocompatible est un polyéthylène ou un polymère fluorocarboné ;
   et dans lequel le ligand inducteur d'apoptose est FasL.

2. Surface sélective selon la revendication 1, laquelle surface sélective est capable de sélectionner des cellules résistantes à la signalisation de l'apoptose.

3. Surface sélective selon l'une quelconque des revendications précédentes, laquelle surface sélective est sous la forme d'une surface intérieure d'un récipient, d'un film, d'un disque ou de billes.

4. Surface sélective selon l'une quelconque des revendications précédentes, dans laquelle ledit polymère fluorocarboné est l'éthylène-tétrafluoroéthylène (ETFE).

5. Surface sélective selon la revendication 3, dans laquelle lesdites billes sont choisies dans le groupe constitué par les billes magnétiques, les billes paramagnétiques, et les billes de dextrane.

6. Surface sélective selon la revendication 3, dans laquelle lesdites billes sont des billes d'agarose, des billes de polystyrène, des billes de Sepharose ou des billes de silice liées à du N-hydroxysuccinimide (NHS), des billes d'agarose activées par du NHS ou des billes comprenant de l'agarose activé par un aldéhyde, un support activé par une azlactone, du carbonyldiimidazole-agarose/Trisacryl, des groupes carboxyle, du bromure de cyanogène, du polyéthylèneglycol (PEG), et l'une quelconque de leurs combinaisons.

7. Surface sélective selon l'une quelconque des revendications précédentes, dans laquelle le FasL est biologiquement actif.

8. Surface sélective selon l'une quelconque des revendications précédentes, dans laquelle d'environ 0,01 à environ 3 ng/cm$^2$ de FasL sont liés à la surface sélective.

9. Dispositif comprenant la surface sélective de l'une quelconque des revendications précédentes.

10. Dispositif selon la revendication 9, lequel dispositif est un récipient.

11. Dispositif selon l'une quelconque des revendications 9 et 10, dans lequel la surface sélective est la surface sélective d'une pluralité de billes contenues dans le récipient.

12. Surface sélective selon l'une quelconque des revendications 1 à 8 ou dispositif selon l'une quelconque des revendications 9 à 11, pour une utilisation dans la sélection d'une cellule résistante à la signalisation de l'apoptose.

13. Utilisation de la surface sélective de l'une quelconque des revendications 1 à 8 ou du dispositif de l'une quelconque des revendications 9 à 11 dans une méthode de sélection d'une cellule résistante à la signalisation de l'apoptose.

14. Méthode de préparation d'un dispositif ayant une surface sélective, ladite méthode comprenant :

   (i) l'obtention d'une surface sélective comprenant un polymère biocompatible revêtu par ou comprenant des molécules d'anhydride maléique (copolymère d'anhydride maléique) ; dans laquelle ledit polymère biocompatible est le polyéthylène ou un polymère fluorocarboné ;
   (ii) incubation de ladite surface sélective avec un tampon d'immobilisation ou un tampon de revêtement comprenant du FasL dissous ;
   (iii) élimination du FasL non lié de la surface sélective ;

   ce qui donne ainsi un dispositif ayant une surface sélective comprenant un polymère biocompatible revêtu par ou comprenant des molécules d'anhydride maléique (copolymère d'anhydride maléique), et dans laquelle ladite surface a en outre du FasL immobilisé sur celle-ci ;

dans laquelle ledit polymère biocompatible est un polyéthylène ou un polymère fluorocarboné.

**15.** Kit de sélection cellulaire comprenant :

a. la surface sélective de l'une quelconque des revendications 1 à 9 ou le dispositif de l'une quelconque des revendications 9 à 11 ; et
b. des instructions pour une utilisation dans la sélection de cellules souches et progénitrices qui sont résistantes à l'apoptose à médiation par un récepteur.

Fig. 1A

Fig. 1B

Fig. 2A

Fig. 2B

Fig. 2C

Fig. 2D

% of stained cells

- Early apoptosis
- Late apoptosis
- Necrosis

Fig. 2E

Fig. 2F

**Fig. 3A**

**Fig. 3B**

**Fig. 3C**

**Fig. 3D**

**Fig. 3E**

**Fig. 3F**

Fig. 4A

Fig. 4B

Fig. 4C

Fig. 4D

Fig. 4E

Fig. 4F

Fig. 4G

EP 3 303 563 B1

fresh    medium    FasL    TNFα

24 hours

- CD34
- CD3
- CD19
- CD33

48 hours

**Fig. 5A**

**Fig. 5B**

CFU/10³ cells

**Fig. 5C**

EP 3 303 563 B1

Fig. 6A

Fig. 6B

Fig. 6C

Fig. 6D

Fig. 7A

Fig. 7B

Fig. 7C

*Equal numbers of initial cells from the same UCB unit*

Fig. 7D

**Fig. 8A**

**Fig. 8B**

**Fig. 8C**

**Fig. 8D**

EP 3 303 563 B1

Fig. 8F

Fig. 8E

Fig. 9A

Fig. 9B

Fig. 9C

Fig. 9D

Fig. 9E

Fig. 10A

Fig. 10B

Fig. 10C

Fig. 10E

Fig. 10D

Fig. 11A

Fig. 11B

Fig. 11C

Fig. 11D

**Fig. 11E**

**Fig. 11F**

**Fig. 11G**

**Fig. 11H**

EP 3 303 563 B1

Fig. 12A

Fig. 12B

Fig. 12C

Fig. 12D

**Fig. 12E**

Fig. 13A

Fig. 13B

Fig. 13C

Fig. 13D

Fig. 13E

Fig. 13F

EP 3 303 563 B1

Fig. 14A

Fig. 14B

Fig. 14C

**Fig. 14D**

**Fig. 14E**

EP 3 303 563 B1

Fig. 15A
Fig. 15B
Fig. 15C
Fig. 15D

Fig. 16A

Fig. 16B

Fig. 16C

Fig. 16D

Fig. 16E

Fig. 16F

EP 3 303 563 B1

Fig. 17A

Fig. 17B

Fig. 17C

Fig. 17D

Fig. 17E

Fig. 17F

Fig. 17G

Fig. 17H

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013132477 A
- US 20140072603 A
- US 62171403 B
- US 62171412 B

**Non-patent literature cited in the description**

- **NIPITHAKUL T. et al.** *Chinese J. of Polymer Science,* 2012, vol. 30 (2
- **CHAUSSE et al.** *Clin. Chem,* 1990, vol. 36 (3), 525-528